# EUROPÄISCHE PATENTSCHRIFT

(11) **EP 0 658 198 B1**
(45) Veröffentlichungstag und Bekanntmachung des Hinweises auf die Patenterteilung: **27.01.1999**
(21) Anmeldenummer: 93919176.3
(22) Anmeldetag: 26.08.1993
(51) Int. Cl.: C12N 15/67, C12N 15/63, C12N 15/85, C12N 5/10, C07K 14/49

(54) **MULTICISTRONISCHE EXPRESSIONSEINHEITEN UND DEREN VERWENDUNG**
MULTICISTRONIC EXPRESSION UNITS AND THEIR USE
UNITES MULTICISTRONIQUES D'EXPRESSION ET LEUR UTILISATION

(30) Priorität: 27.08.1992 DE 4228458
(43) Veröffentlichungstag der Anmeldung: 21.06.1995
(73) Patentinhaber: Beiersdorf Aktiengesellschaft, 20245 Hamburg (DE); Gesellschaft für Biotechnologische Forschung mbH (GBF), D-38124 Braunschweig (DE)
(72) Erfinder: DIRKS, Wilhelm, D-38106 Braunschweig (DE); WIRTH, Manfred, D-38300 Wolfenbüttel (DE); HAUSER, Hansjörg, D-38104 Braunschweig (DE); EICHNER, Wolfram, D-22301 Hamburg (DE); ACHTERBERG, Volker, D-20257 Hamburg (DE); DÖRSCHNER, Albrecht, D-20357 Hamburg (DE); MEYER-INGOLD, Wolfgang, D-22457 Hamburg (DE); MIELKE, Heiko, D-21629 Neu Wulmstorf (DE)
(74) Vertreter: UEXKÜLL & STOLBERG
(86) Internationale Anmeldenummer: EP9302294
(87) Internationale Veröffentlichungsnummer: WO9405785

(56) Entgegenhaltungen:
- EP-A- 0 259 632
- WO-A-90/01550
- WO-A-90/08163
- WO-A-93/03143
- J. BIOL. CHEM. Bd. 263, Nr. 31 , 5. November 1988 , AM. SOC. MOL. BIOL., INC.,BALTIMORE, US; Seiten 16202 - 16208 A. OSTMAN ET AL. 'Synthesis and assembly of a functionally active recombinant platelet-derived growth factor AB heterodimer' in der Anmeldung erwähnt
- BIOCHEMISTRY Bd. 29, Nr. 1 , 9. Januar 1990 , AM. CHEM. SOC.,WASHINGTON,DC,US; Seiten 166 - 172 C.E. HART ET AL. 'Purification of PDGF-AB and PDGF-BB from human platelet extracts and identification of all three PDGF dimers in human platelets' in der Anmeldung erwähnt
- MOL. CELL. BIOL. Bd. 11, Nr. 5 , Mai 1991 , AM. SOC. MICROBIOL., WASHINGTON, D.C. US; Seiten 2656 - 2664 D. FALCONE AND D.W. ANDREWS 'Both the 5' untranslated region and the sequences surrounding the start site contribute to efficient initiation od translation in vitro' in der Anmeldung erwähnt
- J. BIOL. CHEM. Bd. 263, Nr. 31 , 5. November 1988 , AM. SOC. MOL. BIOL., INC.,BALTIMORE, US; Seiten 16493 - 16498 A. HAMMACHER ET AL. 'A major part of platelet-derived growth factor purified from human platelets is a heterodimer of one A and one B chain' in der Anmeldung erwähnt
- TRENDS IN BIOCHEMICAL SCIENCE Bd. 15, Nr. 12 , Dezember 1990 , ELSEVIER SCIENCE, AMSTERDAM, NL; Seiten 477 - 483 R.J. JACKSON ET AL. 'The novel mechanism of initiation of picornavirus RNA translation' in der Anmeldung erwähnt
- NUCL. ACID RES. Bd. 19, Nr. 16 , 25. August 1991 , IRL PRESS, OXFORD, ENGLAND; Seiten 4485 - 4490 R.J. KAUFMAN ET AL. 'Improved vectors for stable expression of foreign genes in mammalian cells by use of the untranslated leader sequence from EMC virus' in der Anmeldung erwähnt

## Beschreibung

Die Erfindung betrifft multicistronische Expressionseinheiten und deren Verwendung zur äquimolaren Expression von Polypeptiden oder Untereinheiten derselben in Säugerzellen als Wirtszellen.

Es ist bereits seit langem möglich, einzelne Proteine, deren Gene durch Klonierung isoliert wurden, nach Manipulation und Gentransfer in verschiedenen prokaryontischen und eukaryontischen Zellen herzustellen. Für das Erreichen der vollen biologischen Aktivität vieler Proteine sind korrekte Faltungen, richtiges Prozessieren und gegebenenfalls auch posttranslationale Modifikationen erforderlich, welche in prokaryontischen und niederen eukaryontischen Expressionssystemen oftmals nicht korrekt ausgeführt werden. Aus diesem Grund bedient man sich häufig Säugerzellen als Wirte. Säugerzellen sind darüber hinaus in der Lage, große Mengen von Proteinen zu sekretieren.

Aus den verschiedensten Gründen bedarf es oftmals der simultanen Herstellung zweier oder mehrerer Proteinketten. Zum Beispiel sind viele natürliche Proteine in ihrer funktionellen Form aus mehreren Untereinheiten zusammengesetzt (z.B. Antikörper). Natürlicherweise erfolgt die Assoziation verschiedener Untereinheiten von komplexen Proteinen nach der Proteinsynthese. An dieser Assoziation sind häufig andere Komponenten des zellulären Apparates als Katalysatoren oder Kontrollelemente beteiligt, wobei es gelegentlich auch zu Umfaltungen der ursprünglichen Strukturen kommt. Störungen der Assoziation, z.B. durch ungleiche Synthese der einzelnen Komponenten, können sowohl für die zu bildenden Proteine als auch für die Wirtszelle negative Konsequenzen haben. Natürlicherweise unterliegt dieses System einer ausgefeilten, meist zellspezifischen Regulation. Da diese Regulation in genetisch manipulierten Zellen im allgemeinen nicht nachstellbar ist, wurden die nachfolgend erläuterten Alternativen zur simultanen Herstellung mehrerer Fremdproteine entwickelt und angewandt:
1) Die Gene werden getrennt in Expressionsvektoren integriert und dann in einem geeigneten Verhältnis in die Zellen cotransferiert. Dies setzt voraus, daß mehrere Plasmidkopien gleichzeitig stabil aufgenommen und bei der Teilung weitergetragen werden. Das Verhältnis der Expression der verschiedenen Gene zueinander hängt sowohl von der Kopienzahl als auch von der Integrationsstelle im Genom der Wirtszelle ab. Durch aufwendige Screeningverfahren ist es möglich, Zellklone zu isolieren, welche die einzelnen Genprodukte im gewünschten Verhältnis zueinander exprimieren.
2) Um die Kopienzahl zu nivellieren, werden die verschiedenen Gene in unabhängigen Transkriptionseinheiten auf einem Vektor plaziert. Dies sichert weitgehend die stöchiometrische Repräsentanz der Gene, aber auch dieses Verfahren ist mit Problemen behaftet. Selbst wenn nämlich Expressionseinheiten mit Promotoren gleicher Stärke verwendet werden, ist keineswegs sichergestellt, daß die mRNAs, welche für die verschiedenen Proteine kodieren, die gleiche Stabilität und Translationseffizienz aufweisen. Auch die Transkriptionseffizienz beider Gene muß nicht zwangsläufig identisch sein. In diesem Fall wird mit Hilfe von gentechnischen Tricks (Positionierung der Transkriptionseinheiten zueinander, Modulation der Stärke der einzelnen Promotoren durch Wegnehmen oder Hinzufügen einzelner Elemente) die Stöchiometrie der Expression schrittweise hergestellt.
3) Zur Vermeidung der Probleme im Zusammenhang mit der Stabilität der mRNA verschiedener Transkripte wurden bi- oder multicistronische Vektoren entwickelt. Hierzu liegen die einzelnen Leseraster der die Proteinketten kodierenden Genabschnitte - Cistrons - auf einer Transkriptionseinheit (Expressionseinheit). Die Expression des multicistronischen Gens erfolgt durch einen einzigen Promotor. Bei derartigen Vektoren wird normalerweise das erste Cistron sehr effizient translatiert, die nachfolgenden aber in Abhängigkeit von den intercistronischen Sequenzen. Verwendet man für diese intercistronischen Sequenzen normale 5' nicht translatierte Sequenzen (5'UTR) aus monocistronischen Genen, so ist die Expression des nachfolgenden Cistrons meist sehr niedrig (in der Regel um 0,5 bis 2% der Translation des ersten Cistrons, Kaufman et al., 1987; Boel et al., 1987). Diese Effizienz konnte zunächst durch Einfügen von Leader-sequenzen (High Efficiency Leadern, HEL) auf etwa 20% gesteigert werden. Mit der Entdeckung und Verwendung von bestimmten zellulären und viralen Sequenzen, welche eine interne Translationsinitiation ermöglichen (IRES; Jackson et al., 1990) war es möglich, ein Translationsverhältnis zwischen dem ersten und dem nachfolgenden Cistron von 3:1 zu erzielen.

Die Schlüsselrolle bei der Verwendung bi- oder multicistronischer Vektoren spielt die Translation. Normalerweise erfolgt die Initiation der Translation in Eukaryonten nach dem "cap"-abhängigen Mechanismus, im Verlaufe dessen ein Prä-Initiationskomplex aus Proteinen und RNA am 5'Ende einer mRNA mit "cap" (methyliertes Nukleotid) aufgebaut wird. Von dort aus wird ein geeignetes Translationsinitiationskodon ausgesucht, von dem ausgehend die Translation gestartet wird. Man glaubt, daß dies über einen "Scanning"-Prozeß abläuft, wobei sich der Prä-Initiationskomplex entlang der mRNA in 3'Richtung bewegt. Auf diese Weise wird, von einigen Ausnahmen abgesehen, immer das am 5'Ende liegende Cistron effizient translatiert (Kozak, 1989). Alle nachfolgenden Cistrons werden gar nicht oder sehr ineffizient translatiert. Die Translations-Effizienz der nachfolgenden Cistrons konnte durch Optimierung des Abstandes zwischen den Genen (intercistronische Regionen; Kozak, 1987; Wirth et al., 1991) oder durch Verwendung von sogenannten "high efficiency leader"-Sequenzen (HEL, s.o.) verbessert werden (z.B. Falcone und Andrews, 1991 und Ref. darin). HEL's sind solche 5'nicht translatierten Bereiche von Genen oder auch anderen Sequenzen, welche die Initiation der "cap"-abhängigen Translation stimulieren. Auch bei derartigen Konstrukten sind jedoch die erreichbaren Expressionswerte für das zweite und die nachfolgenden Cistrons immer deutlich geringer als die des "cap"-abhängig regulierten ersten Cistrons.

Ein in den letzten Jahren auf gedeckter Mechanismus zur internen Translationsinitiation, d.h. der Start der Translation an einer mRNA ohne "cap"-Struktur, benutzt spezifische Nukleinsäuresequenzen. Zu diesen Sequenzen zählen die nicht translatierten Bereiche einzelner Picorna-Viren, z.B. Polio-Virus, Encephalomyocarditis-Virus, (Pelletier und Sonenberg, 1988; Jang et al., 1988; Jang et al., 1989) sowie einiger zellulärer Proteine, z.B. BiP (Macejak und Sarnow, 1991). Bei den Picorna-Viren sorgt ein kurzer Abschnitt des 5'nicht translatierten Bereichs, das sogenannte IRES (internal ribosomal entry site), für die interne Bindung eines Prä-Initiationskomplexes. Ein Bereich von 628 nt ist im Fall des Poliovirus Typ 1 für die effiziente Initiation dieser Translation notwendig. Untersuchungen zeigten, daß für eine effiziente Translation nicht nur die 400 Basenpaare des 3'-Bereiches von IRES, sondern auch der extreme 5'Teil des Poliovirus nicht-translatierten Region notwendig ist (Simoes und Sarnow, 1991). Auf der anderen Seite führt das "capping", die Voraussetzung für den normalen Initiationsmechanismus der Translation, zu einer Reduktion der Effizienz der internen Initiation von Polio-Virus IRES, wenn er am 5'Ende einer entsprechenden mRNA lokalisiert ist (Hambidge und Sarnow, 1991). Der negative Effekt wird aufgehoben, wenn die IRES für die Initiation des zweiten Cistrons verantwortlich ist, also zwischen "cap" und IRES ein Cistron liegt.

IRES-Elemente können also als Initiatoren für die effiziente Translation von Leserastern fungieren. Dabei beeinflussen sie die "cap"-abhängige Translation des ersten Cistrons nicht. Auch umgekehrt scheint eine Beeinflussung der IRES-abhängigen Initiation unabhängig von der "cap"-abhängigen Translationsinitiation zu sein. Die Mechanismen beider Vorgänge unterscheiden sich auch deutlich in der Verwendung verschiedener zellulärer Faktoren (Meerovitch et al., 1989; Jang und Wimmer, 1990). In der vergangenen Zeit wurden mehrere Untersuchungen publiziert, bei denen bicistronische Expressionsplasmide verwendet wurden (Adam et al., 1991; Ghattas et al., 1991; Kaufman et al.,1991; Wood et al., 1991; Wirth et al., 1991). Da aber offensichtlich die "cap"-abhängige Translation stärker ist als die IRES-abhängige Translation, konnte eine stöchiometrische Expression zweier Proteinketten nicht erreicht werden. Die bisherigen Verwendungen konzentierten sich deshalb auf die Verwendung von Selektionsmarkern im zweiten Cistron. Die enge Expressionskoppelung des Selektionsmarkers mit dem zu exprimierenden Gen, welches das erste Cistron darstellt, ist besonders vorteilhaft bei der Selektion auf Hochexpression, insbesondere, wenn eine vorausgehende Genamplifikation erforderlich ist. Die Synthese äquimolarer Proteinmengen von bi- oder multicistronischen Expressionsvektoren ist jedoch bisher nicht erreicht worden.

Ein typisches Beispiel für die Bedeutung, welche der äquimolaren Expression zweier verschiedener Proteinketten in rekombinanten Herstellungsverfahren zukommen kann, ist die gentechnologische Gewinnung des Wachstumsfaktors aus Blutplättchen, "Platelet-Derived-Growth-Factor" (PDGF), einem der Hauptmitogene im menschlichen Blutserum. Aus menschlichen Thrombozyten aufgereinigtes PDGF besteht aus zwei unterschiedlichen, aber nahe verwandten Polypeptidketten, die durch Disulfidbrücken miteinander verknüpft sind. Unter reduzierenden Bedingungen zerfällt das dimere PDGF in seine monomeren Untereinheiten, wovon die größere (Mᵣ 15-17.000 D) als PDGF-A-Kette und die kleinere (Mᵣ 14.000 D) als PDGF-B-Kette bezeichnet wird (Johnsson et al., 1984).

Die Proteinketten PDGF-A und -B werden von verschiedenen Genen kodiert. Die vollständige Struktur beider Genprodukte konnte durch cDNA-Klonierung aufgeklärt werden (Ratner et al., 1985, Betsholtz et al., 1986). Dabei zeigte es sich, daß beide PDGF-Moleküle zunächst als ungewöhnlich lange Vorläufermoleküle, sog. Precursoren, synthetisiert und anschließend intrazellulär zu den reifen PDGF-Ketten prozessiert werden. Durch alternatives Splicen lassen sich zwei verschiedene PDGF-A-Transkripte erklären, die sich durch An- oder Abwesenheit eines 69-bp Segmentes im 3'-Bereich unterscheiden (Betsholtz et al., 1986; Wise et al., 1989). Durch dieses Insert kommt es zu einer Änderung im codierenden Abschnitt mit der Folge, daß eine kurze (PDGF-A_{K}, 110 Aminosäuren) und eine lange (PDGF-A_{L}, 125 Aminosäuren) Form der PDGF-A-Kette gebildet wird. Beide Varianten sind als normale zelluläre Proteine nebeneinander nachweisbar, wobei die kürzere Form die häufigere Spezies ist (Matoskova et al., 1989; Young et al., 1990).

Beide Gene sind auf unterschiedlichen Chromosomen lokalisiert und zeigen einen hohen Homologiegrad. Eine Vielzahl von Arbeiten zeigt, daß beide Gene unterschiedlichen Regulationsmechanismen unterliegen. Eine Folge davon ist, daß beide PDGF-Ketten natürlicherweise in verschiedenen Zelltypen in unterschiedlichem Verhältnis zueinander produziert werden.

Alle drei möglichen Isoformen des PDGF (AA, AB und BB) kommen natürlicherweise vor und sind in Thrombozyten in sogenannten A-Granula gespeichert. Aus überlagerten menschlichen Blutplättchen kann neben dem die Hauptmenge bildenden PDGF-AB Heterodimer auch PDGF-BB zu etwa 30 % isoliert werden (Hammacher et al., 1988). Frisch präparierte Blutplättchen enthalten auch einen hohen Anteil (27%) an PDGF-AA (Hart et al., 1990). Es kann daher angenommen werden, daß in den Vorläuferzellen der Thrombozyten, den Megakaryozyten, der Anteil beider Homodimere zusammen etwa dem AB-Heterodimer-Anteil entspricht. Da die Konzentration jeder PDGF-Spezies im Thrombozyten direkt korrelieren sollte mit ihrer individuellen Bedeutung im Wundheilungsgeschehen, kommt insbesondere der häufigsten Isoform, dem PDGF-AB, eine herausragende Bedeutung auf der Suche nach einem "Wundheilungshormon" zu.

Jede der verschiedenen Isoformen besitzt biologische Aktivität *in vitro.* Erst die Verfügbarkeit der hochreinen, rekombinanten PDGF-Isoformen (Hoppe et al., 1989; Hoppe et al., 1990) machte vergleichende Studien zur Differenzierung der unterschiedlichen Wirkungsspektren der verschiedenen PDGF-Spezies möglich. Inzwischen belegen eine Reihe von Untersuchungen die unterschiedliche Potenz von PDGF-AA, AB und BB im Chemotaxis- und DNA-Proliferationstest (Hosang et al., 1989; Nister et al., 1988; Reilly & Broski, 1989; Siegbahn et al, 1990), sowie deren unterschiedlichen Einfluß auf die Freisetzung von Inositol-1,4,5-trisphosphat, Produktion von Diacylglycerol und [Ca²⁺]ᵢ-Mobilisierung (Block et al., 1989; Sachinidis et al., 1990 A, 1990 B). Zwei unterschiedliche PDGF-Rezeptorpopulationen, von denen der PDGF-α-Rezeptor alle PDGF-Isoformen und der β-Rezeptor nur PDGF-BB bindet (Hart et al., 1988; Heldin et al., 1988) liefern eine plausible Erklärung dafür, wie sich Wirkungsunterschiede der PDGF-Isoformen über deren unterschiedliche Potenz zur Rezeptor-aktivierung entfalten können. Die meßbaren unterschiedlichen *in* *vitro*-Effekte der PDGF-Isoformen, zusammen mit dem Nachweis zweier verschiedenener Rezeptorpopulationen, lassen Rückschlüsse auf unterschiedliche *in vivo* Wirkungsspektren von PDGF-AA, AB und BB zu. Daher ist die Produktion von reinem PDGF-AB, ohne PDGF-BB oder PDGF-AA als Begleitproteine, wünschenswert. Um ein homogenes, gut charakterisiertes Heterodimer zu erhalten, müßten die Homodimere sonst durch Reinigung vollständig eliminiert werden, was durch die sehr ähnlichen chromatographischen Eigenschaften aller PDGF Spezies zusätzlich erschwert wird.

Eine Reihe verschiedener Wege zur Herstellung von rekombinanten PDGF-Homodimeren, insbesondere PDGF-BB, sind zum Teil schon seit längerer Zeit bekannt (Kelly et al., 1985; Heldin et al., 1986; Hoppe et al., 1989; Beckmann et al., 1988; Bywater et al., 1988; Stroobant & Waterfield 1984). Ein Herstellungsverfahren für hochreines PDGF-AB wurde von Hoppe et al. (1990, s.a. PCT/EP 90/00 063) beschrieben. Hier werden die getrennt in unterschiedlichen *E. coli*-Zellen hergestellten, inaktiven Monomere durch *in vitro*-Renaturierung in biologisch aktives PDGF-AB überführt.

Die bislang synthetisierten Genprodukte der drei PDGF-Isoformen weisen, trotz variierender Länge der A- bzw. B-Einzelstränge, weitgehend übereinstimmende biologische Aktivität auf.

Für die heterologe Expression von PDGF-AB Heterodimeren in eukaryontischen Systemen gelten die eingangs erwähnten Kriterien der simultanen Expression zweier (oder mehrerer) Proteine. Die bisher publizierten Strategien zur Herstellung von PDGF-AB in rekombinanten CHO-Zellen (Östman et al., 1988) und mit Hilfe von Hefe-Expressionssystemen [EP 0 259 632] entsprechen dem oben unter 2) erläuterten Fallbeispiel, wo sich beide PDGF-Gene in unabhängigen Transkriptionseinheiten auf einem Vektor befinden. Die Quantifizierung der auf diese Weise in CHO-Zellen exprimierten unterschiedlichen PDGF-Dimere ergab 19% für PDGF-AA, 69% für PDGF-AB und 12% für PDGF-BB (Östman et al., 1988).

Eine Grundvoraussetzung für die bevorzugte Synthese von PDGF-AB Heterodimeren mit Hilfe eukaryontischer Expressionssysteme ist nicht nur in der stöchiometrischen Repräsentanz beider Gene, sondern in erster Linie auch in deren koordinierter Expression zu sehen. Daher bieten sich bicistronische Expressionseinheiten als mögliche Hilfsmittel für die Expression heterodimerer Proteine und damit des PDGF-AB an. In WO 90/01550 wird ein derartiges System auch für die Expression von PDGF beschrieben. Wie unter Punkt 3) oben näher erläutert, liefern diese Konstrukte jedoch nur sehr limitierte Expressionsraten für das zweite (und nachfolgende) Cistron(s). Abhängig von der im ersten Cistron lokalisierten PDGF-Kette werden vorwiegend Homodimere dieses Typs gebildet. Bisher in der Literatur beschriebene Versuche, beide PDGF-Gene mit Hilfe anderer Expressionssysteme in einer eukaryontischen Zelle zu exprimieren, führten zu Homodimer-Nebenproduktanteilen im Bereich von 30% oder mehr. Um dennoch mit diesen Zellsystemen hochreines PDGF-AB zu erhalten, müssen aufwendige und extrem verlustreiche Reinigungstechniken angewendet werden.

Es ist demgemäß Aufgabe der Erfindung, Mittel zu schaffen, mit deren Hilfe die rekombinante Herstellung von 2 oder mehreren Polypeptiden oder deren Untereinheiten in jeweils äquimolaren Mengen möglich ist und die weiterhin die bevorzugte Bildung von Hetero(di)meren gewährleisten. Ausbeute und Wirtschaftlichkeit der nachgeschalteten Proteinreinigungsverfahren wird dadurch beträchtlich verbessert.

Zur Lösung der Aufgabe wird erfindungsgemäß eine multicistronische Expressionseinheit zur äquimolaren Expression von Polypeptiden oder Untereinheiten derselben in Säugerzellen als Wirtszellen vorgeschlagen, welche gekennzeichnet ist durch die allgemeine Formel

p - 5'UTR - C₁ - (IRES - Y - C₂)ₙ - 3'UTR - polyA,

in der
"p" ein transkriptionaler Promotor ist,
"5'UTR" eine nicht translatierte Nukleotidsequenz ist,
n 1, 2 oder 3 ist,
"C₁" und "C₂" Cistrons sind, welche jeweils ein für ein Polypeptid oder dessen Untereinheit kodierendes Gen enthalten, wobei dann, wenn n 2 oder 3 ist, die Sequenzen C₂ der aufeinanderfolgenden Gruppen (IRES-Y-C₂) untereinander gleich oder verschieden sein können und ferner C₁ und C₂ gleich oder verschieden sein können,
"IRES" eine Nukleotidsequenz viralen, zellulären oder synthetischen Ursprungs ist, die in der Stufe der Translation für die interne Initiation verantwortlich ist,
"Y" eine Nukleotidsequenz ist, welche im Zusammenwirken mit IRES für eine Expression des (der) in C₂ enthaltenen Gens(e) auf solche Weise sorgt, daß die Genprodukte von C₁ und C₂ in äquimolaren Mengen exprimiert werden,
"3'UTR" eine nicht translatierte Nukleotidsequenz ist
   und
"polyA" ein Polyadenylierungssignal ist.

In den patentgemäßen Konstrukten wurde durch Einführung intercistronischer Elemente eine Äquivalenz der Translationseffizienz erreicht und überraschenderweise eine 1:1 Stöchiometrie der Genprodukte gefunden. Damit ist die wesentliche Grundlage für die Expression von Hetero(di)meren in Animalzellen geschaffen. Dadurch, daß die Expressionskapazität der Zelle auf der Ebene der Transkription und Translation voll ausgeschöpft ist und zudem als Folge einer nahezu vollständig erfolgten Heterodimerisierung aufwendige Reinigungsschritte zur Entfernung von Homo(di)meren weitestgehend entfallen können, wird eine hohe Wirtschaftlichkeit der Produktion des jeweiligen Proteins in Säugerzellen gewährleistet.

In den erfindungsgemäßen Expressionseinheiten kommen als Promotoren alle diejenigen Promotoren in Betracht, die in eukaryontischen Zellen wirksam sind, d. h., die die Genexpression in eukaryontischen Zellen initiieren können. Insbesondere können alle konstitutiven und induzierbaren Promotoren viralen (beispielsweise die retroviralen "Long terminal repeats (LTR's) oder der frühe Promotor des Cytomegalie-Virus (CMV), zellulären (beispielsweise die humanen Actin- oder Ubiquitin-Promotoren) oder synthetischen Ursprungs verwendet werden. Erfindungsgemäß ist der SV40-Promotor bevorzugt.

Die 5'UTR und die 3'UTR sind beliebige, in der Regel nichttranslatierte Nukleotidsequenzen, die regulierende Elemente enthalten können und die der operativen Verknüpfung von "C₁" bzw. "C₂" mit den Transkriptionskontrollelementen dienen. Erfindungsgemäß geeignet ist beispielsweise die SV-40-Sequenz aus pBEH nach Artelt et al. (1988).

Als IRES können alle diejenigen Sequenzen viralen, zellulären oder synthetischen Ursprungs verwendet werden, welche eine interne Bindung der Ribosomen vermitteln. Beispiele für derartige Sequenzen sind die IRES aus *Poliovirus* Typ 1, 2 oder 3 sowie ferner die 5'UTR des *Encephalomyocarditis Virus* (EMCV), des *"Theilers murine encephalomyelitis virus"* (TMEV), des *"foot and mouth disease virus"* (FMDV), des *"bovine enterovirus"* (BEV), des *"coxsackie B virus"* (CBV), des *"human rhinovirus"* (HRV) und die "human immunoglobulin heavy chain binding protein" (BIP) 5'UTR, die *Drosophila Antennapediae* 5'UTR, die *Drosophila Ultrabithorax* 5'UTR oder genetische Hybride oder Fragmente aus den oben angeführten Sequenzen. Erfindungsgemäß bevorzugt ist die IRES aus Poliovirus Typ 1 gemäß SEQ ID Nr. 5 welche die ersten 628 Nukleotide der 5' nicht-translatierten Region des Poliovirus Typ 1 einschließt.

Als "Y" können alle diejenigen Nukleotidsequenzen eingesetzt werden, die im Zusammenwirken mit IRES wie in der allgemeinen Formel angegeben für eine Expression des (der) in C₂ enthaltenen Gens(e) auf solche Weise sorgt, daß die Genprodukte von C₁ und C₂ in äquimolaren Mengen exprimiert werden. Insbesondere kommen die *Xenopus laevis* β-Globin 5' UTR (Falcone and Andrews, 1991; Patient et al., 1983), die *Alfalfa mosaic virus* RNA4 5' UTR (Jobling and Gehrke, 1987), die Ferritin 5' UTR (animal, Klausner and Harford, 1989), die *Tobacco mosaic virus* 5' UTR ("Omega") plus Leadermutanten (Gallie et al., 1987A, 1987B; Gallie et al.,(1988), die *Turnip yellow mosaic virus* (TYMV) 5' UTR, die *Brome mosaic virus* (BMV) RNA3 5' UTR und die *Rous sarcoma virus* (RSV) 5' UTR (vgl. jeweils Gallie et al., 1987B), die Adenovirus tripartite leader (L1-3) und Varianten (Berkner, Zymogenetics) WO 90/01550; Berkner and Sharp (1985); Kaufman (1985), die *Xenopus borealis* 5' UTR β-Globin und die *Xenopus tropicalis* 5' UTR β-Globin (vgl. jeweils Knoechel et al., 1986) in Betracht, wobei die β-Globinsequenz aus *Xenopus laevis* gemäß SEQ ID NO: 4 erfindungsgemäß besonders bevorzugt ist.

Gemäß einer besonders bevorzugten Ausführungsform der Erfindung ist IRES die Poliovirus Typ 1 UTR gemäß SEQ ID NO: 3 und "Y" die β-Globinsequenz aus *Xenopus laevis* gemäß SEQ ID NO: 4.

Weitere geeignete IRES und "Y" Sequenzen können zudem mit dem unten näher beschriebenen Verfahren, welches ebenfalls Teil der Erfindung ist, ermittelt werden.

Die Cistrons C₁ und C₂ können unabhängig voneinander und in beliebiger Reihenfolge jeweils ein Gen enthalten, welches für eine Polypeptid-Komponente eines aus 2 oder mehreren derartiger Komponenten bestehenden singulären oder heteromeren Proteins kodiert, wobei die Gene erfindungsgemäß äquimolar exprimiert werden und die Komponenten demgemäß innerhalb einer Wirtszelle jeweils im Verhältnis 1:1 zur Verfügung stehen. Es können also die Cistrons C₁ und C₂ untereinander gleich oder verschieden sein, und die Cistrons C₂ der aufeinanderfolgenden Gruppen (IRES-Y-C₂) können untereinander gleich oder verschieden sein. Insbesondere können C₁ und C₂ jeweils Gene enthalten, welche für die verschiedenen Untereinheiten von Faktor VIII, Kreatin-Kinase, Hämoglobin, Immunglobulinen, Histokompatibilitäts-Antigenen, T-Zell Rezeptoren, Scatter-Faktor (HGF-SF), Mitglieder aus der Famlie des Transforming Growth Factor Typ β, Bone Morphogenic Protein (BMP), Mitglieder der Integrin-Familie, Mitglieder der Inhibin-Familie, PDGF oder deren natürliche oder synthetische Varianten und Derivate kodieren.

Gemäß einer besonders bevorzugten Ausführungsform betrifft die Erfindung die Expression von PDGF-AB; demgemäß ist in der besonders bevorzugten Expressionseinheit "n" gleich 1, und C₁ und C₂ enthalten alternativ ein für die A- oder die B-Kette von PDGF, ein biologisch aktives Analogon oder ein Fragment derselben kodierendes Gen, wobei beide Gene gleichzeitig in der Expressionseinheit vertreten sind.

Prinzipiell mußten jedoch für die Produktion von PDGF-AB zusätzlich Veränderungen am PDGF-B-precursor vorgenommen werden, da sich PDGF-A- und B-Vorläufermoleküle in ihren biophysikalischen Eigenschaften unterscheiden. Es ist bekannt, daß die Expression von PDGF-B nicht zwangsläufig mit der Sekretion biologisch aktiven Materials korreliert. Ein Großteil des exprimierten PDGF-BB bleibt in enger Assoziation mit der Cytoplasmamembran (Robbins et al., 1985). In CHO-Zellen ist die Expression von PDGF-B mit Hilfe monocistronischer Expressionsvektoren deutlich geringer als die von PDGF-A. Die Ursache hierfür liegt darin, daß PDGF-BB extrazellulär über eine elektrostatische Wechselwirkung an der Plasmamembran der Produzentenzelle zurückgehalten und nur zu einem geringen Teil in das Medium abgegeben wird (La Rochelle et al., 1990; La Rochelle et al., 1991; Östman et al., 1991). Für die Vermittlung der Retention ist ein kurzer Abschnitt des C-terminalen Bereich des PDGF-B-precursors verantworlich (Östman et al., 1991). In den patentgemäßen Konstrukten wurde dieser Abschnitt der PDGF-B-Vorläufersequenz durch die Einführung eines Stop-codons an das 3'-Ende der reifen PDGF-B-Kette entfernt. Die entsprechend verkürzte DNA-Sequenz für den PDGF-B-precursor wird als B190 gekennzeichnet. Sekretiertes PDGF-BB aus Kulturüberständen von Zellen, die mit diesem Konstrukt transformiert wurden, wird als B* bezeichnet.

Zur Herstellung von PDGF-AB nach der Erfindung können C₁ oder C₂ die PDGF-A_{K}- (SEQ ID Nr. 1) oder die PDGF-A_{L} Vorläufer-Sequenz, die vollständige PDGF-B Vorläufersequenz (SEQ ID Nr. 3), das *v-sis*-Gen aus Simian Sarcoma Virus oder die Basenpaare 283 bis 609 gemäß SEQ ID Nr. 3 oder ein Genfragment enthalten, das für ein PDGF-B-Vorläufermolekül kodiert, welches durch Ersetzen des für Arginin kodierenden Codons in der Aminosäureposition 191 des PDGF-B-precursors durch ein Translations-Stop-Codon verkürzt ist. Die vorgenannten Gene können in beliebiger Kombination vorliegen, soweit jeweils ein für die A- und die B-Kette kodierendes Gen vorhanden ist.

Erfindungsgemäß besonders bevorzugt ist eine Expressionseinheit, in der C₁ und C₂ alternativ die PDGF-A_{K}-Sequenz (SEQ ID Nr. 1) oder die verkürzte PDGF-B Vorläufersequenz (SEQ ID Nr. 24) enthalten und beide Gene gleichzeitig in der Expressionseinheit vertreten sind.

Zur Ermittlung weiterer geeigneter IRES und "Y" Sequenzen wie unten im Einzelnen beschrieben dienen Expressionseinheiten, in denen "n" 1 ist und C₁ und C₂ voneinander verschiedene Reportergene enthalten. Gemäß einer besonders bevorzugten Ausführungform der Erfindung enthält eine derartige Expressionseinheit als Reportergene die für Luciferase (SEQ ID Nr. 22) und für sekretorische alkalische Phosphatase (SEQ ID Nr. 20) kodierenden Gene.

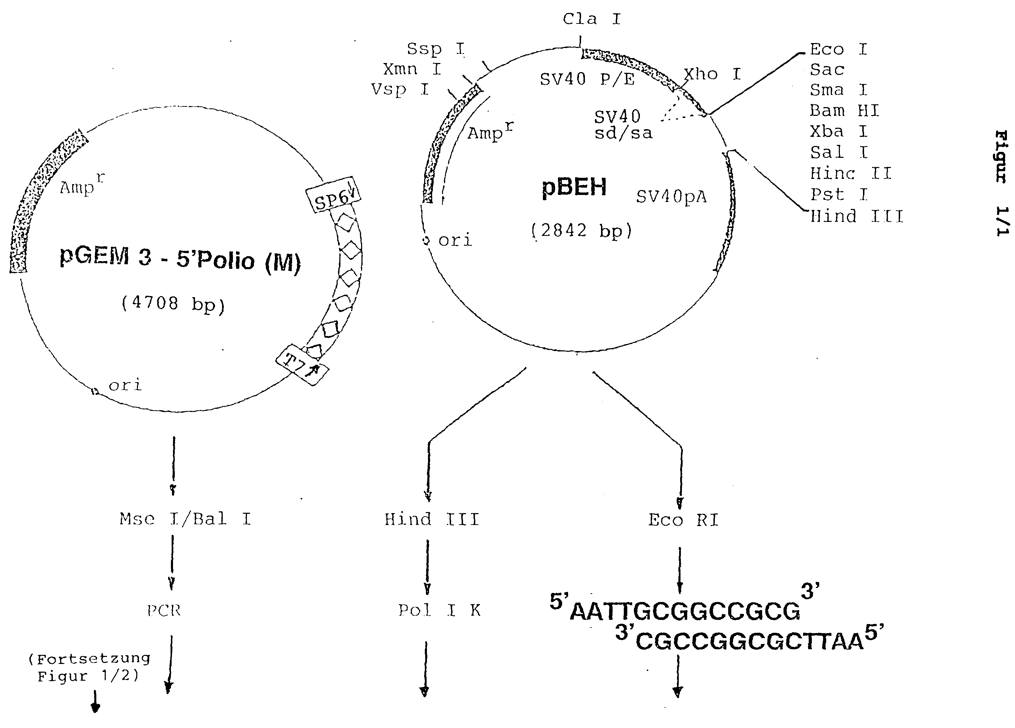

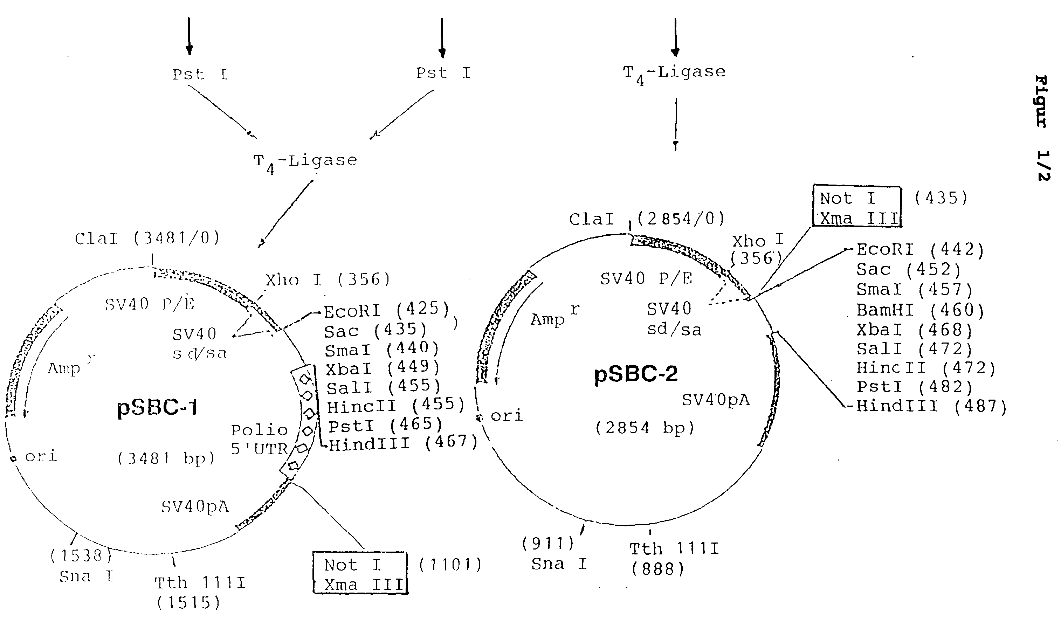

Gegenstand der Erfindung sind ferner rekombinante DNA Vektoren, welche die erfindungsgemäßen Expressionseinheiten operativ insertiert enthalten. Erfindungsgemäß bevorzugte Vektoren und deren Herstellung sind in den Figuren 1 bis 6C dargestellt.

Die Erfindung schließt ferner Wirtszellen ein, welche Säugerzellen sind und die mit einem Vektor transformiert sind, der die erfindungsgemäße Expressionseinheit operativ insertiert trägt. Vorzugsweise handelt es sich um CHO- oder BHK-Zellen.

Eine besonders bevorzugte Ausführungsform der Erfindung betrifft Wirtszellen, insbesondere BHK-Zellen, die mit Vektoren transformiert sind, die eine der für PDGF-AB kodierenden Expressionseinheiten tragen, wie sie oben im einzelnen beschrieben sind. Vorzugsweise handelt es sich um Vektoren, in denen C₁ und C₂ alternativ die PDGF-A_{K}-Sequenz (SEQ ID Nr. 1) oder die vollständige (SEQ ID Nr. 3) bzw. die verkürzte (SEQ ID Nr. 24) PDGF-B Vorläufersequenz enthalten.

Erfindungsgemäß transformierte PDGF-AB produzierende BHK-Zellen wurden unter der Bezeichnung 92-22-6 (pSBC-PDGF-A/-G-B190, s. Tab. 2) entsprechend DSM ACC 2048 oder 92-22-7 (pSBC-PDGF-B190/ G-A, s. Tab. 2) entsprechend DSM ACC 2049 am 11. 8. 1992 bei der Deutschen Sammlung von Mikroorganismen und Zellkulturen GmbH (DSM) hinterlegt.

Zur Ermittlung weiterer geeigneter IRES oder "Y" Sequenzen werden Wirtszellen kultiviert, die mit Vektoren transformiert sind, die Reportergene enthaltende Expressionseinheiten tragen, wie sie oben im einzelnen beschrieben sind. Vorzugsweise handelt es sich um erfindungsgemäße Vektoren, welche die für Luciferase und für sekretorische alkalische Phosphatase kodierenden Gene enthalten.

Erfindungsgemäß mit den Genen für Luciferase (SEQ ID Nr. 22) und sekretorische alkalische Phosphatase (SEQ ID Nr. 20) transformierte Wirtszellen wurden unter der Bezeichnung 91-46-9 (pSBC-SEAP/-G-LUC, s. Tab. 1) entsprechend DSM ACC 2046 und 91-46-10 (pSBC-G-SEAP/LUC, s. Tab. 1) entsprechend DSM ACC 2047 am 11. 8. 1992 bei der Deutschen Sammlung von Mikroorganismen und Zellkulturen GmbH (DSM) hinterlegt.

Die Erfindung schließt ferner Verfahren zur Herstellung von solchen Proteinen ein, die aus äquimolaren Anteilen unterschiedlicher Polypeptiduntereinheiten bestehen, indem man Wirtszellen, die mit den oben im einzelnen beschriebenen erfindungsgemäßen Expressionseinheiten transformiert sind, in einem geeigneten Medium kultiviert und das so erzeugte Protein von den Zellen und dem Medium abtrennt.

Beispielsweise können auf diese Weise Proteine wie Faktor VIII, Kreatin-Kinase, Hämoglobin, Immunglobuline, Histokompatibilitäts-Antigene, T-Zell Rezeptoren, Scatter-Faktor (HGF-SF), Mitglieder aus der Famlie des Transforming Growth Factor Typ β, Bone Morphogenic Protein (BMP), Mitglieder der Integrin-Familie, Mitglieder der Inhibin-Familie, PDGF oder natürliche oder synthetische Varianten oder Derivate derselben hergestellt werden.

Mit den erfindungsgemäßen Vektoren ist es auch erstmals möglich, Dimere des PDGF oder anderer Proteine, von denen verschiedene Spliceformen existieren, wie beispielsweise VEGF (Vascular Endothelial Growth Factor) zu erzeugen, die bisher nicht ohne weiteres herstellbar waren, wie dimeres PDGF-A des Typs lange/kurze Kette, PDGF-A_{L}/PDGF-A_{K} oder Moleküle des Typs PDGF-B/v-*sis*. Eine andere Möglichkeit stellen Di- oder Multimere dar, in denen nur eine Kette Signal sequenzen für eine posttranslationale Modifikation, beispielsweise ein Glykosylierungssignal, enthält. Damit sind also "einseitig" glykosylierte oder anderweitig modifizierte Proteine herstellbar.

Gemäß einer besonders bevorzugten Ausführungsform der Erfindung dient das Verfahren zur Herstellung von heterodimerem rPDGF-AB indem man Wirts zellen, die mit Vektoren transformiert sind, welche erfindungsgemäße Expressionseinheiten tragen, die für die PDGF-A- und B-Ketten kodierende Gene tragen, in einem geeigneten Medium kultiviert, wie oben im einzelnen beschrieben. Das so erzeugte rPDGF-AB wird anschließend von den Zellen und dem Medium abgetrennt.

In Beispiel 2 konnte gezeigt werden, daß es mit Hilfe der erfindungsgemäßen bicistronischen Vektorsysteme möglich ist, ausschließlich oder nahezu ausschließlich PDGF-AB Heterodimere zu erzeugen und der Synthese von PDGF-Homodimeren entgegenzuwirken. Unerwarteterweise wird in diesem Konstrukt die Expressionshöhe des zweiten Cistrons derart stimuliert, daß diese der Expressionshöhe des ersten Cistrons entspricht.

Vorzugsweise werden in diesem Zusammenhang erfindungsgemäß BHK Zellen kultivert, die mit Vektoren transformiert sind, in denen C₁ und C₂ alternativ jeweils die PDGF-A_{K}-Sequenz (SEQ ID Nr. 1) oder die vollständige (SEQ ID Nr. 3) bzw. die verkürzte (SEQ ID Nr. 24) PDGF-B Vorläufersequenz enthalten.

Als Medium kommen alle bekannten Medien zum Kultivieren von Säugerzellen in Betracht, einschließlich synthetischer, proteinfreier oder proteinarmer Produktionsmedien. Erfindungsgemäß wurde DMEM (Dulbecco's Modified Eagle Medium), angereichert mit 4,5 g/l Glukose und 5 bis 10 % FCS, bevorzugt.

Das rPDGF-AB kann nach herkömmlichen Verfahren (vgl. beispielsweise Östmann et al. 1988), von den Zellen und dem Medium abgetrennt werden. Vorzugsweise bietet sich ein für PDGF-AA aus BHK-Zellen entwickeltes und hoch effizientes Verfahren (Eichner et al., 1989) an.

Durch Kultivieren der oben beschriebenen, erfindungsgemäßen Wirtszellen ist schließlich heterodimeres rPDGF-AB erhältlich, welches im wesentlichen frei ist von homodimeren Begleitprodukten. Überraschenderweise hat es sich gezeigt, daß die mit dem erfindungsgemäßen Konstrukt transformierten Wirtszellen das heterodimere PDGF-AB mit einer Reinheit von 90% und mehr, bezogen auf die Gesamtmenge des gebildeten PDGF, sezernieren. Erfindungsgemäß bevorzugt ist heterodimeres PDGF-AB durch Kultivieren von BHK-Zellen zugänglich, die transformiert mit dem erfindungsgemäßen Konstrukt transformiert sind.

Das erfindungsgemäß erhältliche rPDGF-AB unterscheidet sich von den bisher bekannten rekombinanten PDGF-AB Produkten in erster Linie durch seinen hohen Reinheitsgrad.

Den im Stand der Technik bekannten Produkten haften, abhängig von deren Herstellung, in mehrfacher Hinsicht Nachteile an. So ist es bekannt, daß die heterologe Genexpression in Hefezellen, wie in EP 259 632 oder 288 307 beschrieben, zu Proteinprodukten mit gegenüber dem humanen Produkt veränderten Glykosylierungsmustern führt. Zudem ist in Hefezellen exprimiertes PDGF-B zumindest teilweise unvollständig prozessiert und/oder proteolytisch abgebaut (vgl. WO 92/01716). Derartige Produkte weisen somit ein verändertes Kohlenhydratmuster auf, und sie sind mit proteolytischen Abbauprodukten verunreinigt. Zur Vermeidung der vorgenannten Nachteile beschreibt die WO 92/01716 Verfahren zur Herstellung modifizierter PDGF-Ketten, bei denen die Konsensus-Sequenzen für die Glykosylierung bzw. die Protease sensitiven Domänen entfernt sind. Derartige Modifikationen beeinflussen jedoch die biologische Aktivität des Produktes (vgl. WO 92/01716).

Gemäß einer besonders bevorzugten Ausführungsform der Erfindung wird durch Kultivieren von erfindungsgemäß transformierten BHK-Zellen, beispielsweise durch Kultivieren derjenigen Zellen, welche unter der Bezeichnung 92-22-6 entsprechend DSM ACC 2048 oder 92-22-7 entsprechend DSM ACC 2049 am 11. 8. 1992 bei der Deutschen Sammlung von Mikroorganismen und Zellkulturen GmbH (DSM) hinterlegt wurden, heterodimeres rPDGF-AB gewonnen.

Zwar ist aus der WO 90/08163 die rekombinante Herstellung von PDGF-AB in Bakterienzellen, insbesondere in *E. coli* bekannt, welche zwangläufig zu einem nicht glykosylierten Produkt führt. Eine nach diesem Verfahren in *E. coli* Zellen exprimierte PDGF-B Kette ist jedoch aminoterminal um 12 Aminosäuren verkürzt. Darüberhinaus muß das Produkt aus Bakterien *in vitro* renaturiert werden, ein Vorgang, bei dem die korrekte inter- und intramolekularen Bildungen der Disulfidbrücken und die korrekte Faltung des Proteins nicht gewährleistet ist, mit der Folge, daß die immunologischen Eigenschaften des Produkts verändert und die biologische Aktivität beeinflußt werden können.

Das heterodimere rPDGF-AB gemäß der Erfindung wird vorzugsweise zusammen mit pharmazeutisch verträglichen Hilfs- und Trägerstoffen als pharmazeutisches Präparat, insbesondere für die Wundheilung formuliert. In diesem Zusammenhang kann es als Wirkstoff in Pflastern, Wundverbänden und dergleichen enthalten sein. Es ist besonders für die topische Verabreichung geeignet, es kommen jedoch auch Applikationen in Betracht, in deren Verlauf der Wirkstoff in die Wunde eingebracht oder subkutan verabreicht wird. Beispielsweise kann das PDGF-AB in einer geeigneten Matrix mit Depotfunktion im Wundrandbereich subkutan appliziert werden oder direkt subkutan injiziert werden.

Ferner eignet sich das erfindungsgemäße heterodimere rPDGF-AB zur Herstellung von kosmetischen Präparaten, zum Beispiel zur Hautregeneration, zur Hautglättung, zur Verhinderung der Narbenbildung oder der Hautalterung sowie zur Anwendung bei Sonnenbrand.

Geeignete Hilfs- und Trägerstoffe schließen Cellulose-Gele auf wässriger Basis, biologisch abbaubare Polymere sowie jegliche Salben- und Cremebasis und Sprays ein. Ferner können zusätzliche Wirkstoffe, welche die Wundheilung beeinflussen, wie beispielsweise Kollagen, Fibronektin, Faktor XIII, Fibroblasten Wachstumsfaktor (aFGF, bFGF), Transformierender Wachstumsfaktor Typ α oder β, Epidermis Wachstumsfaktor, Insulin oder Insulin-Like Growth Factor (IGF I, II) oder weitere Wachstumsfaktoren in den erfindungsgemäßen Präparaten enthalten sein. Die erfindungsgemäßen Produkte können beispielsweise auch in Wundverbänden in wässriger Lösung vorliegen.

Wie oben dargelegt liegt der Erfindung die Erkenntnis zugrunde, daß durch den Einbau einer bestimmten Sequenz "Y" die IRES-abhängige Translation von C₂ so gesteigert werden kann, daß sie die "cap"-abhängige Translationseffizienz erreicht. Die Sequenz "Y" ist demgemäß in der Lage, mit der IRES so zu kooperieren, daß es zu einer Steigerung der IRES-abhängigen Translationsinitiation kommt, welche mindestens der Effizienz der cap-abhängigen Translationseffizienz entspricht. Sequenzen "Y", welche diese Funktion erfüllen, sind oben ausführlich beschrieben. Patentgemäß wird die β-Globin 5'UTR aus *Xenopus laevis* bevorzugt.

Weitere Sequenzen, welche die erfindungsgemäßen Voraussetzungen erfüllen, lassen sich nach einem Verfahren ermitteln, bei dem zum Auffinden von translations-beeinflussenden Sequenzen "Y", welche im Zusammenwirken mit IRES in Expressionseinheiten nach der Erfindung die äquimolare Expression der Genprodukte von C₁ und C₂ bewirken,
(a) die zu untersuchenden Sequenzen als Y in bi- oder multicistronische Expressionseinheiten eingebracht werden, in denen C₁ und C₂ jeweils ein Reportergen enthalten, wobei diese Gene für verschiedene und leicht voneinander differenzierbare Genprodukte kodieren,
(b) Vektoren konstruiert werden, welche die jeweilige Expressionseinheit operativ insertiert enthalten,
(c) Säugerzellen als Wirtszellen mit den Vektoren aus Stufe (b) transformiert und in einem geeigneten Medium kultiviert werden, und
(d) die Expressionsprodukte von C₁ und C₂ in dem Medium oder nach Abtrennen von den Zellen und/oder dem Medium quantifiziert werden.

Vorzugsweise werden in Stufe (c) CHO- oder BHK-Zellen als Wirtszellen verwendet werden, wobei BHR-21-Zellen besonders bevorzugt sind.

Alternativ können weitere IRES Sequenzen, welche die erfindungsgemäßen Voraussetzungen erfüllen, nach einem Verfahren ermittelt werden, bei dem man zum Auffinden von translations-initiierenden Sequenzen IRES, welche im Zusammenwirken mit "Y" in Expressionseinheiten nach den Ansprüchen 1 bis 15 die äquimolare Expression der Genprodukte von C₁ und C₂ bewirken,
(a) die zu untersuchenden Sequenzen als IRES in Expressionseinheiten einbringt, in denen C₁ und C₂ jeweils ein Reportergen enthalten, wobei diese Gene für verschiedene und leicht voneinander differenzierbare Genprodukte kodieren,
(b) Vektoren konstruiert, welche die jeweilige Expressionseinheit operativ insertiert enthalten,
(c) Säugerzellen als Wirtszellen mit den Vektoren aus Stufe (b) transformiert und in einem geeigneten Medium kultiviert, und
(d) die Expressionsprodukte von C₁ und C₂ in dem Medium oder nach Abtrennen von den Zellen und/oder dem Medium quantifiziert.

Vorzugsweise werden in diesem Verfahren CHO- oder BHK-Zellen als Wirtszellen verwendet und in besonders bevorzugter Weise BHK-Zellen, die als Reportergene die für Luciferase und für sekretorische alkalische Phosphatase kodierenden Gene, (LUC) bzw. (SEAP), enthalten.

Die Erfindung wird nachfolgend anhand von Figuren und Beispielen erläutert.

### I. Beschreibung der Figuren:

### Fig. 1) Herstellung der Basisvektoren pSBC-1 und pSBC-2

Für die Konstruktion des Vektors pSBC-1 wurde ein 627 bp MseI/ BalI-Fragment aus dem Plasmid pGEM3-5'Polio (M) (Sarnow, 1989) als Matritze für eine PCR mit folgenden Primern verwendet (Fig. 1):

Das nach der Amplifikation erhaltene 652 bp Fragment wurde mit Pol I K behandelt, anschließend mit PstI gespalten und in den entsprechend präparierten Vektor pBEH (Artelt et al., 1988) insertiert. Für die Konstruktion des Vektors pSBC-2 wurde das Plasmid pBEH mit EcoRI linearisiert und die folgenden Oligonukleotid-sequenzen hybridisiert und insertiert:

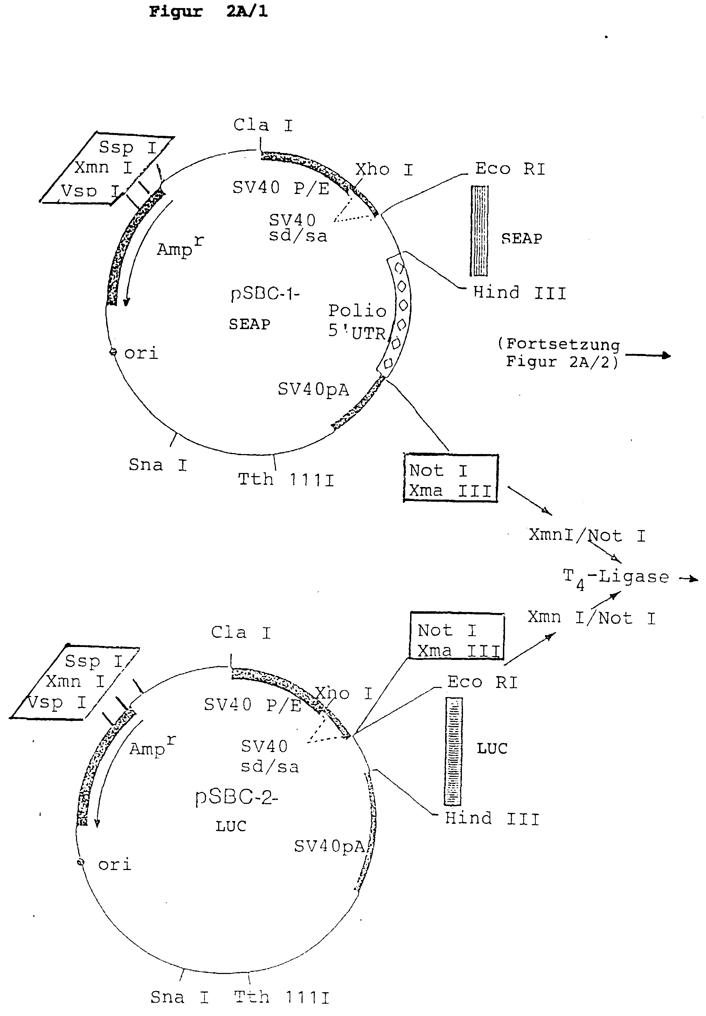

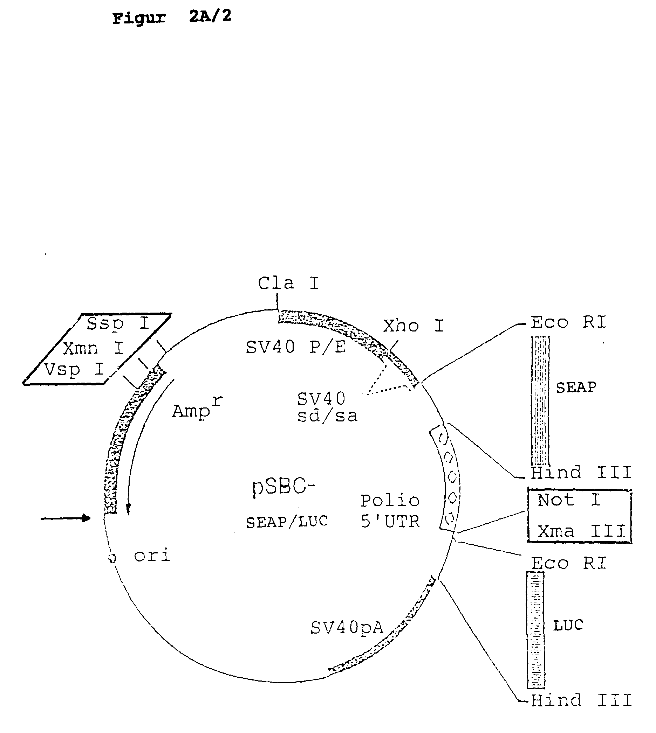

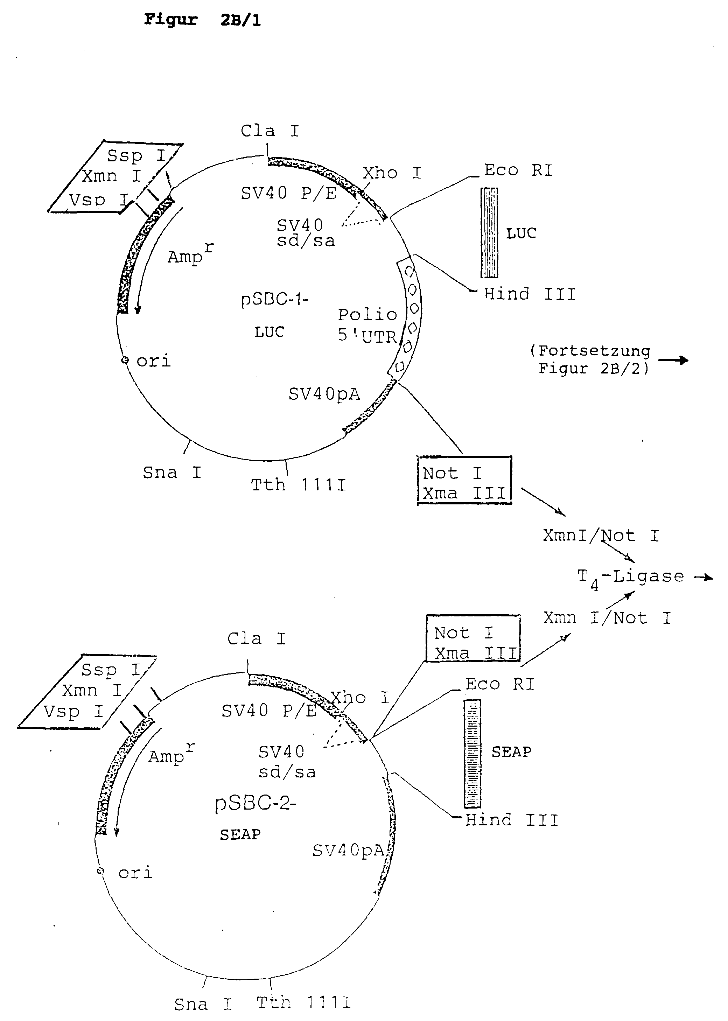

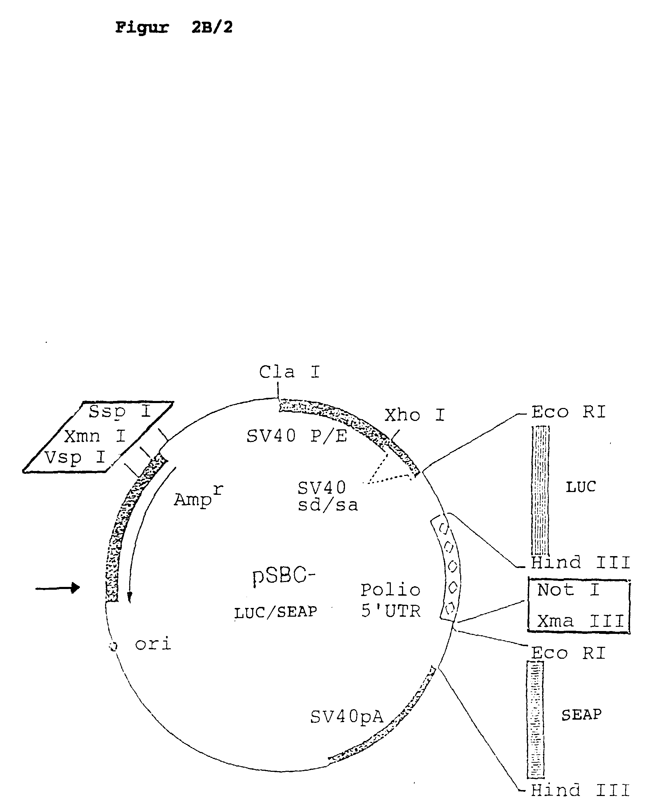

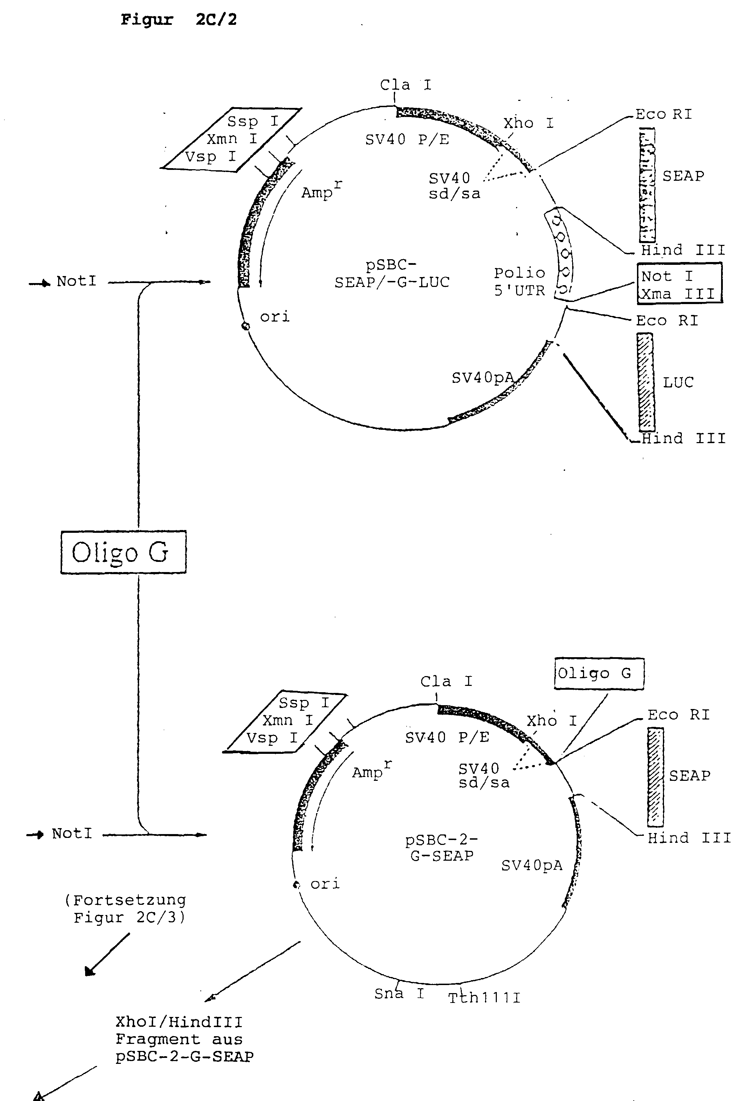

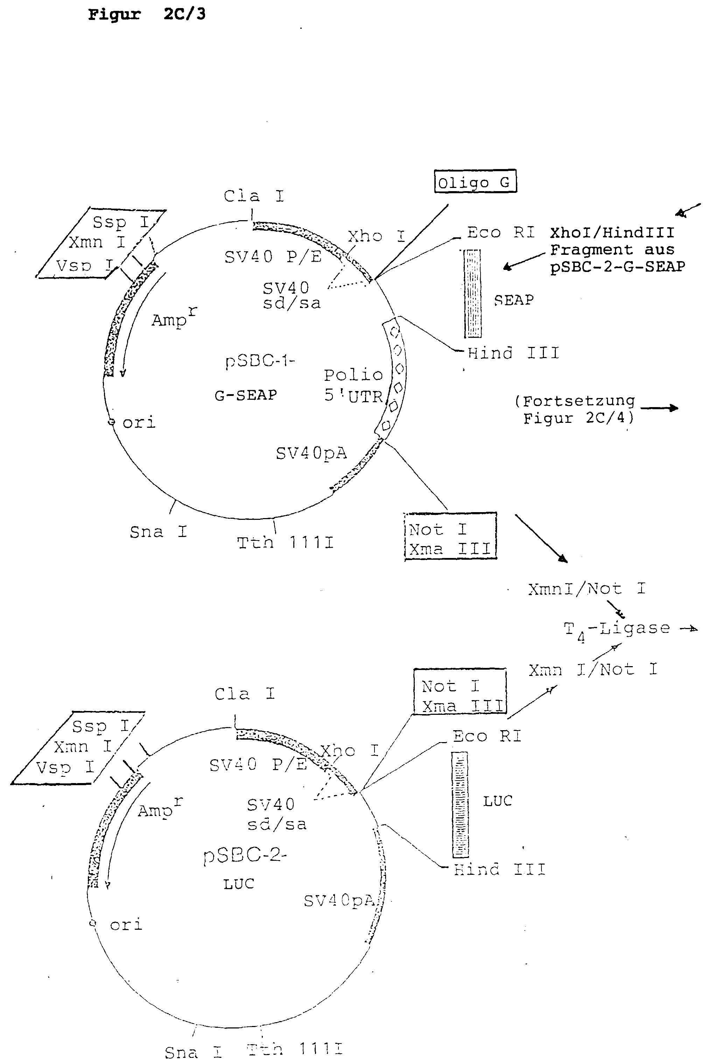

### Fig. 2A und B) Konstruktion der Expressionsvektoren pSBC-LUC/ SEAP und pSBC-SEAP/LUC

Die kodierenden cDNA-Sequenzen der Gene für Luciferase und sekretierte alkalische Phosphatase wurden unter Verwendung von EcoRI/HindIII Restriktionen in die monocistronischen Vektoren pSBC-1 und -2 insertiert (Fig. 2A und 2B). Die Fusion beider Vektoren zu einer bicistronischen Expressionseinheit wurde mit Hilfe der Restriktionsenzyme XmnI/NotI durchgeführt.

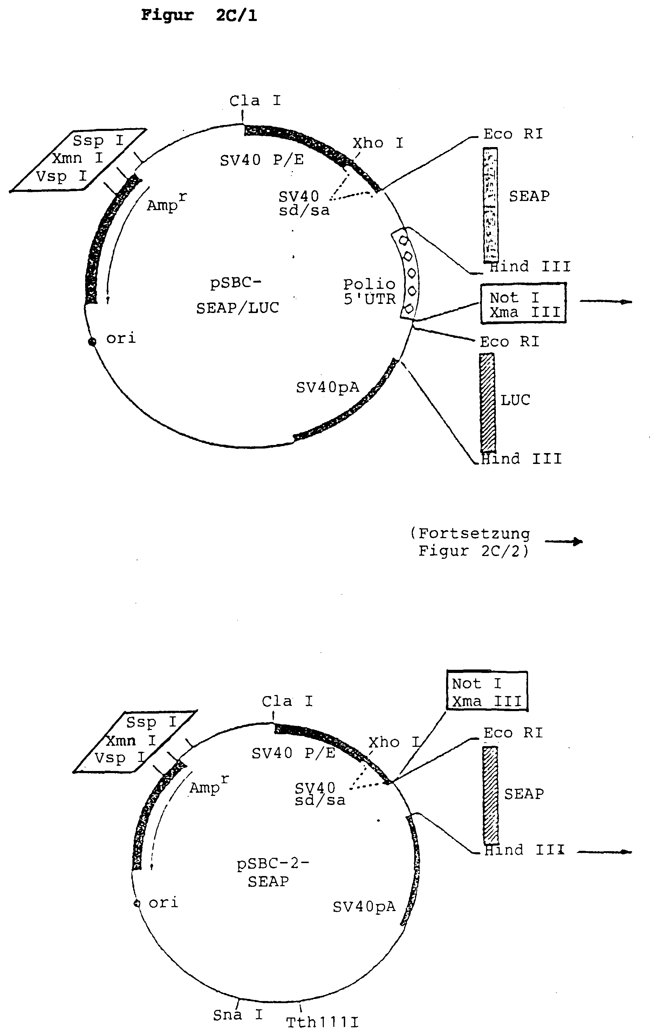

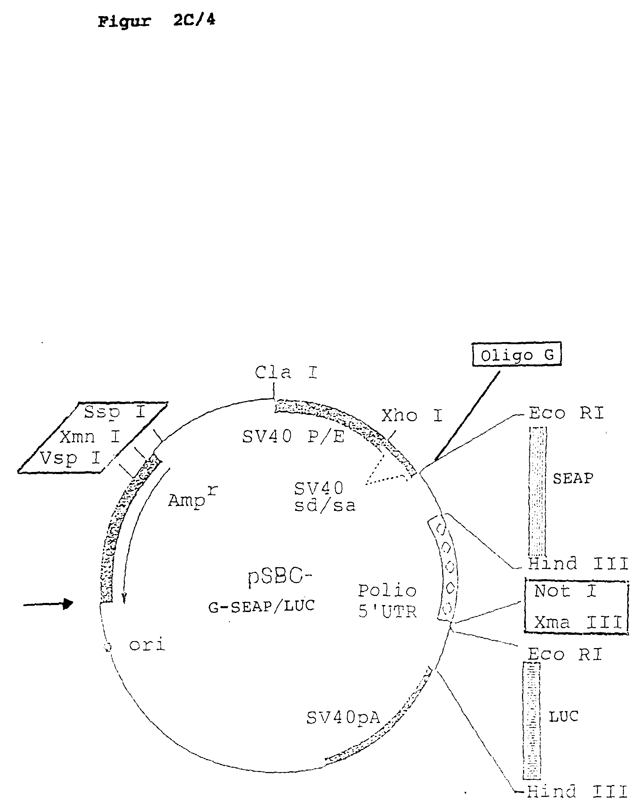

### Fig. 2C) Konstruktion der Plasmide pSBC-SEAP/-G-LUC und pSBC-LUC/-G-SEAP

Die Expressionskonstrukte pSBC-SEAP/-G-LUC und pSBC-LUC/-G-SEAP leiten sich aus den in Fig. 2A und 2B dargestellten Plasmiden ab. Sie enthalten zusätzlich das Oligomer G (SEQ ID Nr. 6), das in die singuläre NotI-site ligiert wurde. Das Oligomer G ist so konstruiert, daß durch Ligation in die NotI-site die 5'-NotI-site verloren geht (eine SalI-site ist hier enthalten), aber die 3'-NotI-site erhalten bleibt.

### Fig. 3) Schematische Darstellung des Vektors M13BCL1

In der Vektorkarte ist der *c-sis* (PDGF-B) homologe Bereich aus pMVW-2 angegeben. Der Bereich des reifen PDGF-B und der NcoI/ SalI Adapter sind durch schwarze Balken hervorgehoben.

### Fig. 4) Rekonstitution der vollständigen PDGF-B-Vorläufersequenz

Das Plasmid pMVW-2 enthält die cDNA des humanen PDGF-B Gens, welches im 5'-translatierten Bereich der Vorläufersequenz unvollständig ist (Weich et al., 1986). Für die Rekonstitution des authentischen PDGF-B precursors wurde in den 5'-terminalen Bereich des Vorläufers durch einen C-T-Austausch in Position 30 des codogenen Abschnittes des Klons pMVW-2 eine BclI-Schnittstelle eingeführt. Durch diesen Schritt geht letzlich nur ein kurzer Abschnitt des kodierenden Bereiches verloren und die lokal codierte Aminosäure (Asparaginsäure) bleibt dabei erhalten. Da die BclI-Schnittstelle in den meisten *E.coli*-Stämmen durch Methylierung resistent gegenüber enzymatischer Spaltung ist, muß das diese Schnittstelle enthaltene Fragment entweder in einen dam⁻-Stamm umkloniert, oder über einen PCR-Schritt amplifiziert werden. Der fehlende Bereich des Vorläufers wird dann als synthetisches SalI/BclI-Fragment [Oligomere PPDGFB1 und PPDGFB2] eingesetzt.

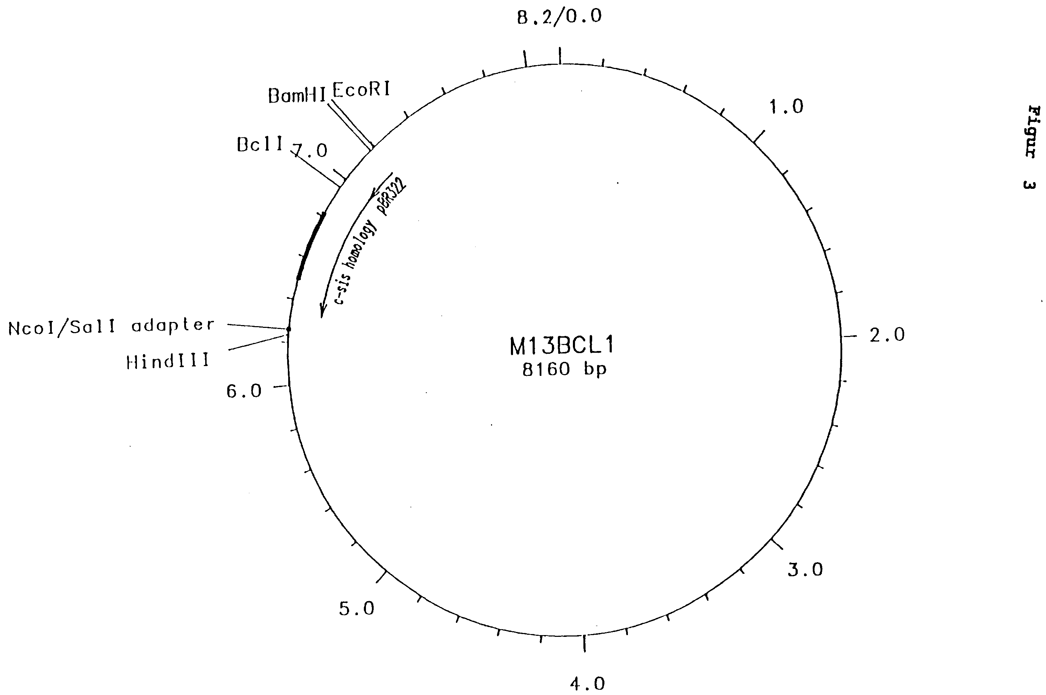

Hierfür wurde zunächst das 914 bp BamHI/NcoI-Fragment aus pMVW-2 über einen synthetischen Adapter [Oligomere NCCLSA1, SEQ ID Nr. 9 und NCCLSA2, SEQ ID Nr. 10] in den BamHI/SalI-gespaltenen Bakteriophagen M13mp19 (Pharmacia) insertiert. Dieses Konstrukt lieferte die notwendige Einzelstrang-DNA für den nachgeschalteten *in vitro* Mutageneseschritt, der mit Hilfe des Oligomer-directed *in vitro* mutagenesis system (Version 2) der Fa. Amersham, basierend auf der Methode von Eckstein et al. [Taylor et al., (1985), Nakamaye K. und Eckstein F. (1986), Sayers et al. (1988)] durchgeführt wurde. Durch den synthetischen Primer PDGBBCL (SEQ ID NR. 11) wird nach der Mutagenese ein Basenaustausch (C zu T) in Position 144 der unter SEQ ID Nr. 3 dargestellten Sequenz erreicht und dadurch im 5'-Bereich des PDGF-B precursors eine BclI-Schnittstelle eingeführt. Dieses Mutagenesederivat wurde als M13BCL1 bezeichnet (Fig. 3).

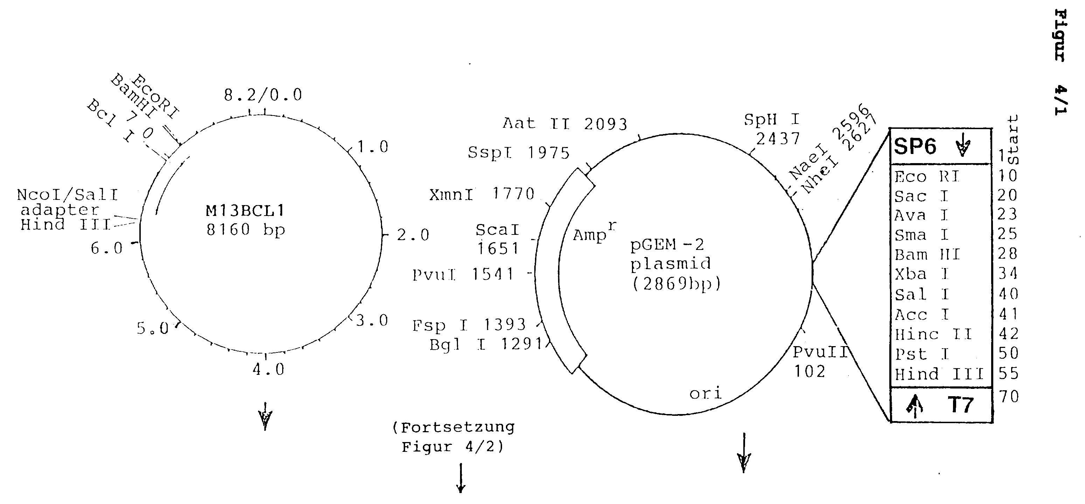

Ein 1100 bp Fragment aus M13BCL1 wurde über einen PCR-Schritt mit Hilfe der Primer M1317MER (SEQ ID Nr. 7) und M1324MER (SEQ ID Nr. 8) amplifiziert, anschließend einer BclI/HindIII-Restriktion unterworfen und das resultierende 770 bp Fragment isoliert. Die synthetischen Oligomere PPDGFB1 (SEQ ID Nr. 12) und PPDGFB2 (SEQ ID Nr. 13) bilden den fehlenden 5'-Bereich des PDGF-B-Vorläufers bis zur BclI-Schnittstelle. Nach dem Annealing wurde dieses doppelsträngige Oligomer anschließend, zusammen mit dem 770 bp PDGF-B Fragment, in den mit einer SalI/HindIII Restriktion vorbereiteten Vektor pGEM-2 (Promega) ligiert (Fig. 4). Die authentische Sequenz von PDGF-B wurde durch vollständige Sequenzierung verifiziert.

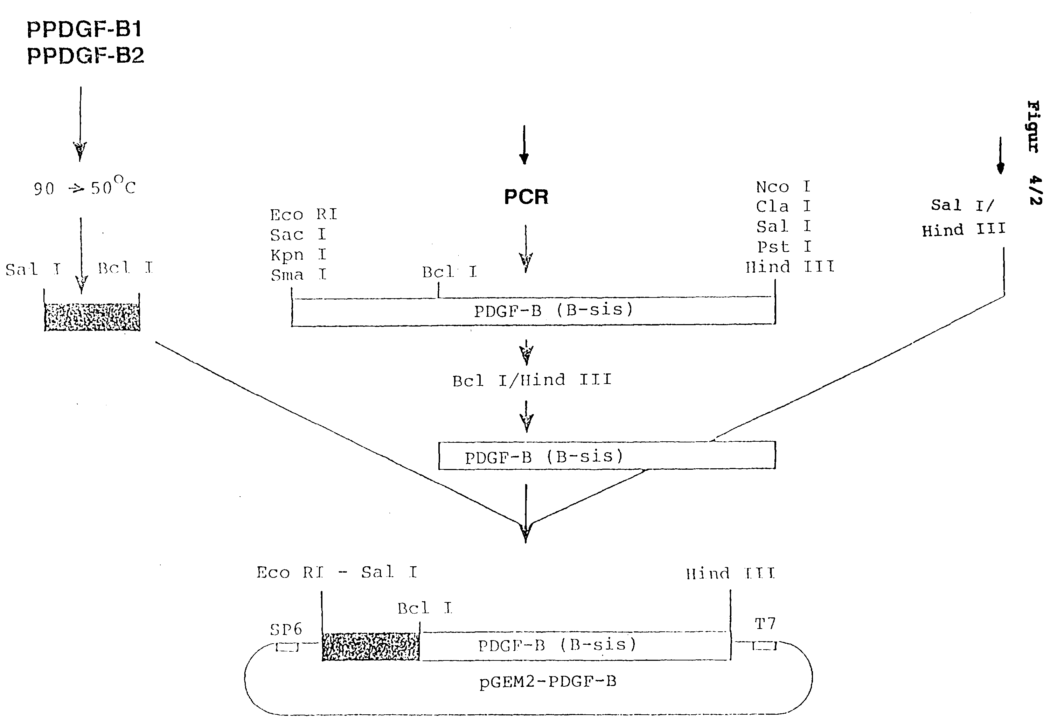

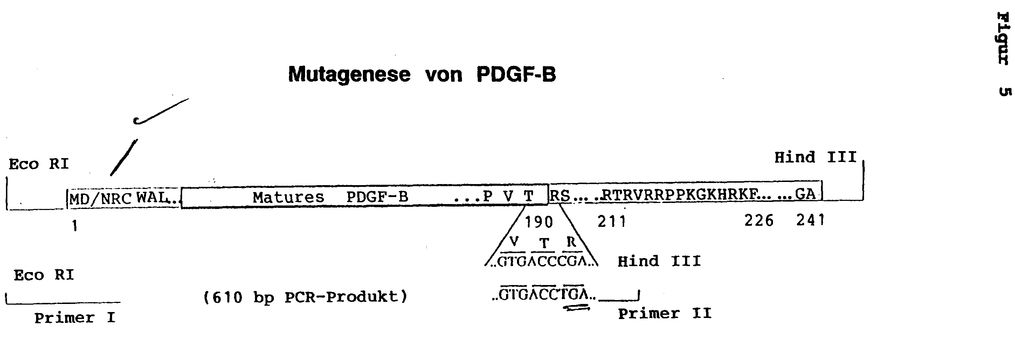

### Fig. 5) Herstellung einer sekretorischen PDGF-B-Kette

Die Expression des PDGF-B Gens über monocistronische Expressionsvektoren ist in BHK-Zellen geringer als die von PDGF-A. Die Ursache hierfür ist, daß PDGF-BB extrazellulär an der Plasmamembran der Produzentenzelle zurückgehalten und nur zu einem geringen Teil in das Medium abgegeben wird (La Rochelle et al., 1991; Östmann et al., 1991). Die Retention von PDGF-B wird durch den carboxyterminalen Bereich, der bei der Freisetzung von PDGF-B natürlicherweise abgespalten wird, vermittelt (La Rochelle et al., 1991). Für die Herstellung einer besser sekretierbaren PDGF-B Variante wurde eine PCR-vermittelte Mutagenese durchgeführt, bei der ein Stopcodon an der Aminosäure in Position 191 des PDGF-B-precursors (Arg) eingefügt wurde. In der so hergestellten Mutante (PDGF-B190, SEQ ID Nr. 24) wird der für die Retention verantwortliche Bereich nicht exprimiert. Das 610 bp lange PCR-Produkt wurde unter Verwendung folgender Primer erhalten (Fig. 5):

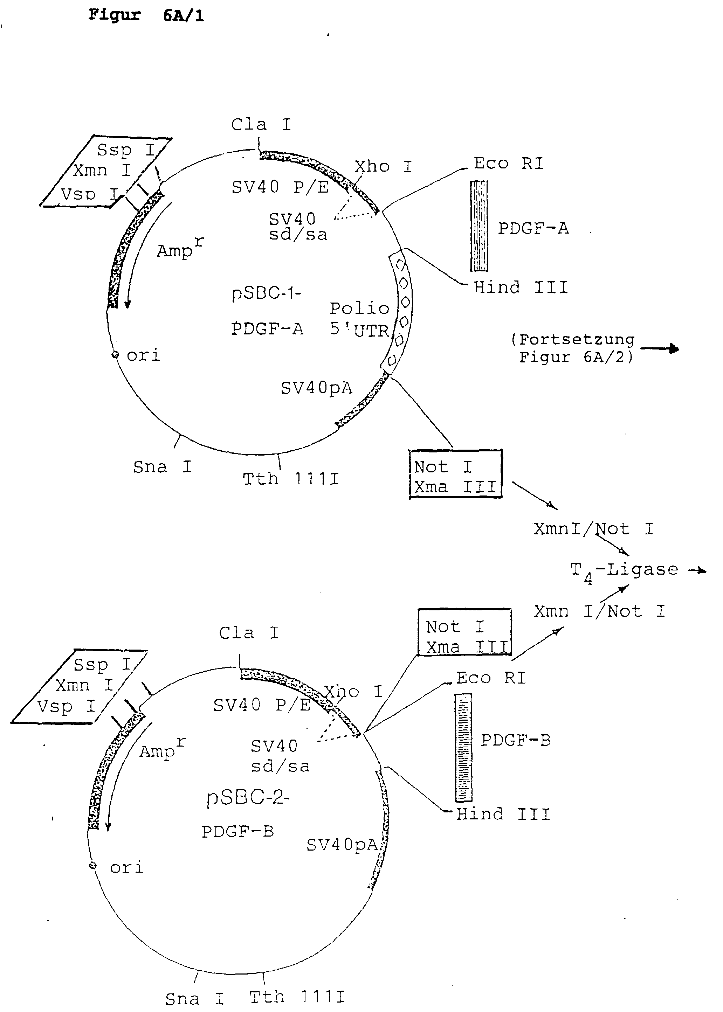

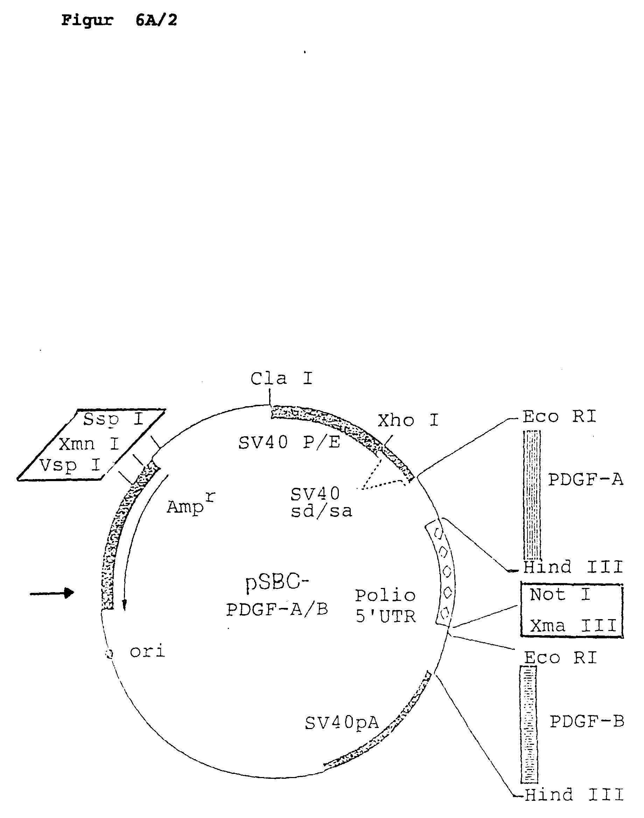

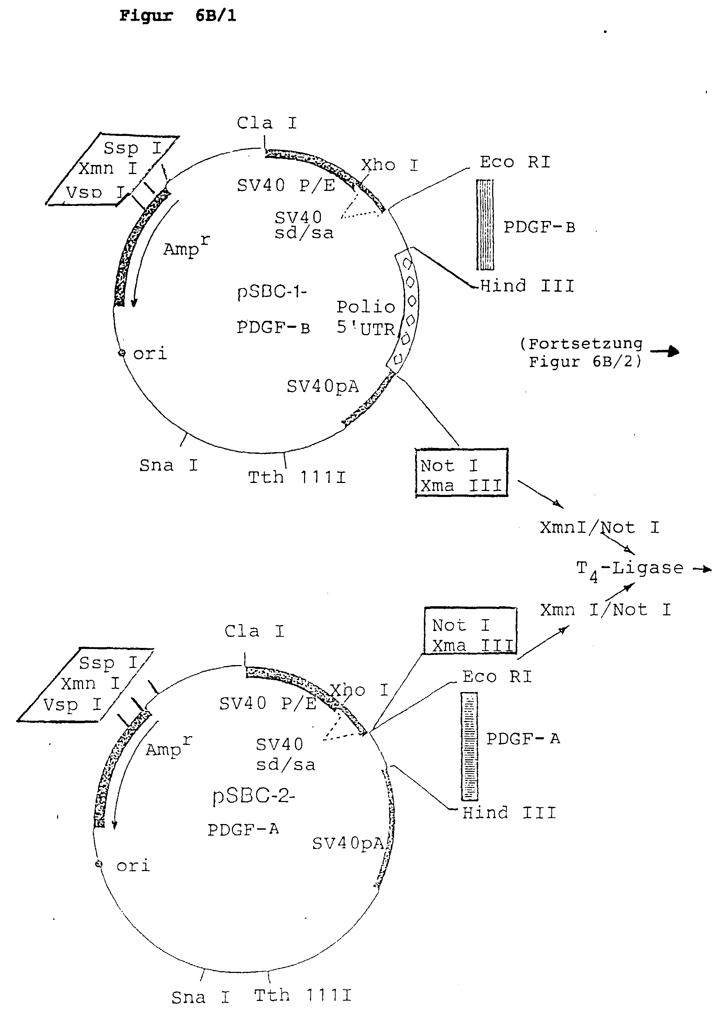

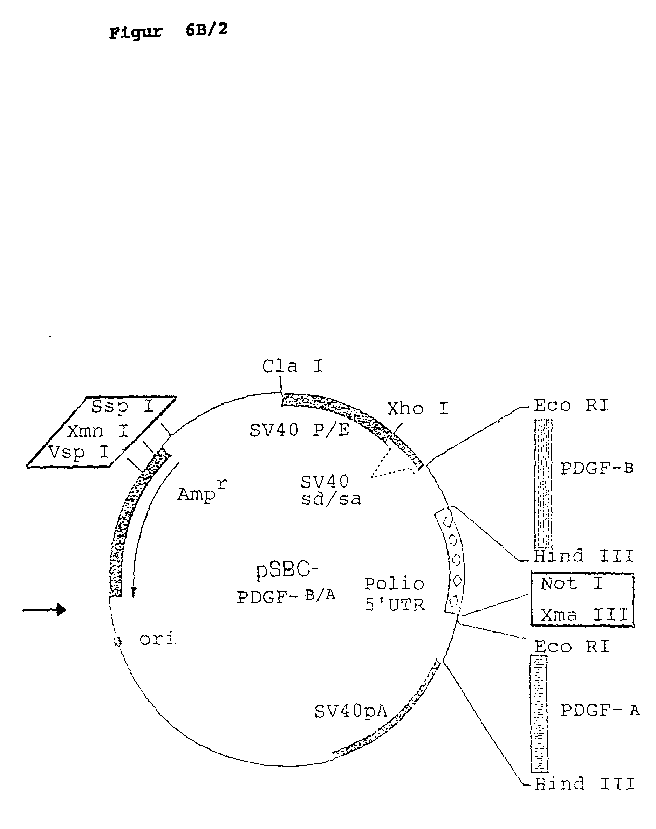

### Fig. 6A und B) Konstruktion der Expressionsvektoren pSBC-PDGF-A/B und pSBC-PDGF-B/A

Die vollständige codierende cDNA für den PDGF-B precursor (Ratner et al., 1985) liegt in dem Vektor pGEM2-PDGF-B vor (Fig.4). Die vollständige cDNA-Sequenz der kurzen Variante der PDGF-A-Kette (Betsholtz et al., 1986) ist im Expressionsvektor pODA (Eichner et al., 1989) enthalten. Dieser Vektor wurde erhalten durch Klonierung des RsaI-Fragments aus pPGF-1 (Hoppe et al., 1987) in den SV-40 Expressionsvektor pBEH (Artelt et al., 1988). Die kodierenden cDNA-Sequenzen der PDGF-A- und -B-Kette wurden unter Verwendung von EcoRI/HindIII Restriktionen in die monocistronischen Vektoren pSBC-1 und -2 (Fig. 1) insertiert. Die Fusion beider Vektoren zu einer bicistronischen Expressionseinheit wurde mit Hilfe der Restriktionsenzyme XmnI/NotI durchgeführt (Fig. 6A, 6B).

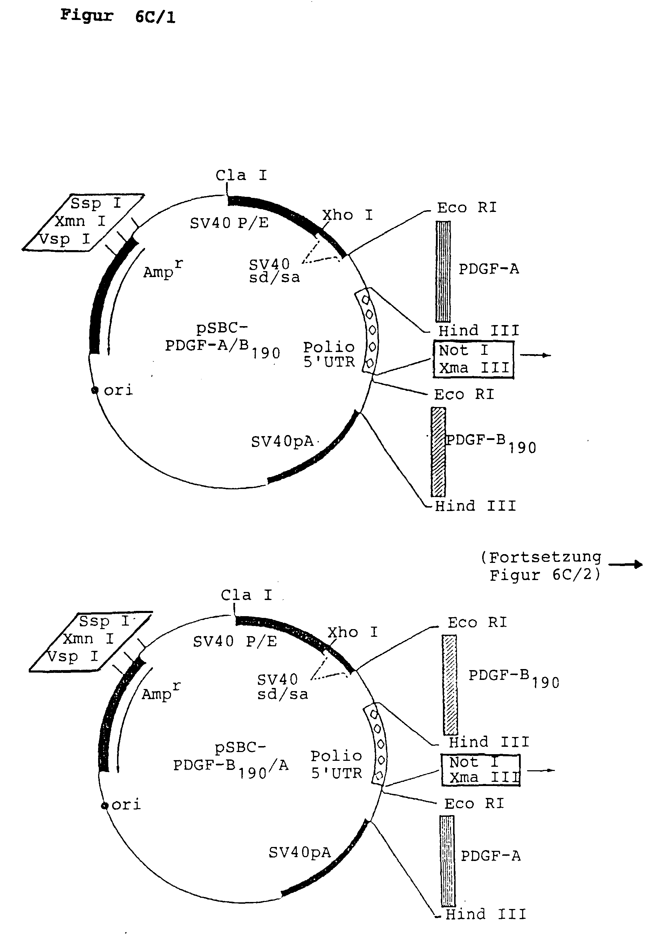

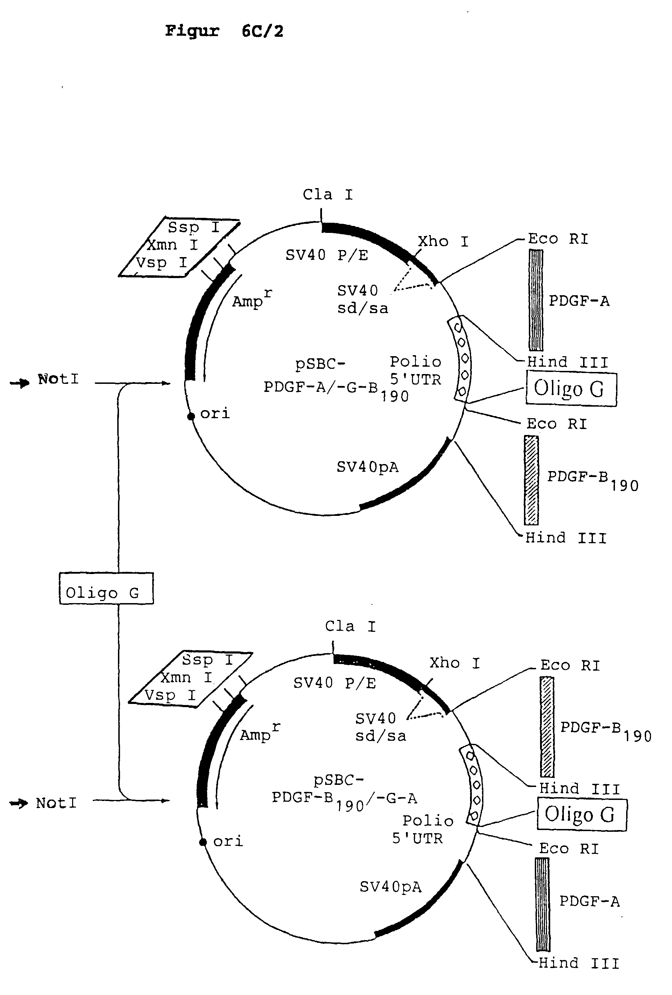

### Fig. 6C) Schematische Darstellung der Plasmide pSBC-PDGF-A/-G-B190 und pSBC-PDGF-B190/-G-A

Die Expressionskonstrukte pSBC-PDGF-A/-G-B190 und pSBC-PDGF-B190/-G-A leiten sich aus den in Fig. 6A und 6B dargestellten Plasmiden ab. Sie enthalten zusätzlich das Oligomer G (SEQ ID Nr. 6), das in die singuläre NotI-site ligiert wurde. Das Oligomer G ist so konstruiert, daß durch Ligation in die NotI-site die 5'-NotI-site verloren geht (eine SalI-site ist hier enthalten), aber die 3'-NotI-site erhalten bleibt.

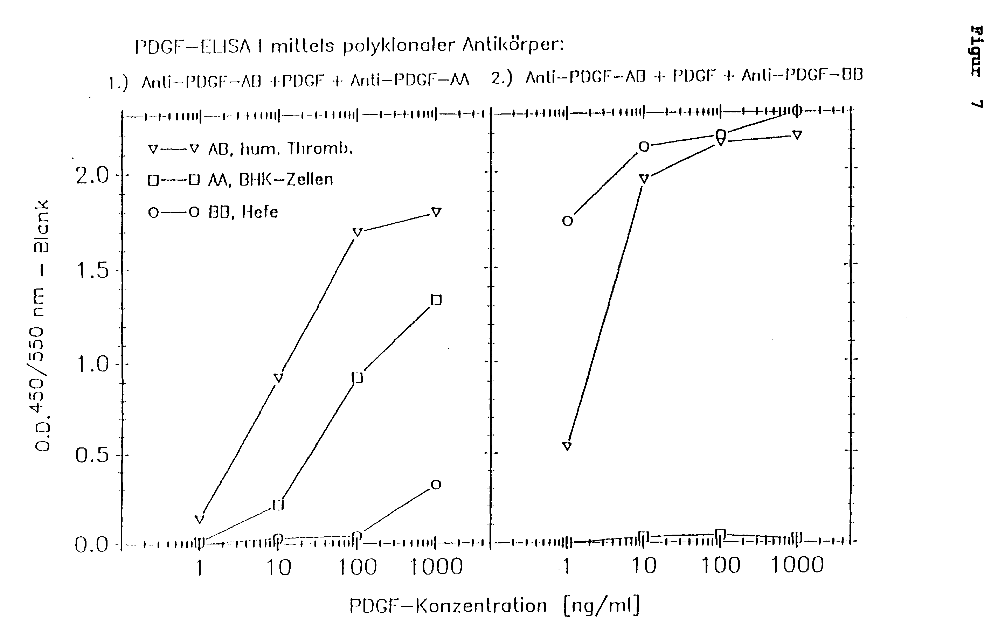

### Fig. 7) Sandwich-ELISA zum Nachweis von PDGF-A- bzw. PDGF-B-Kette mit Hilfe von zwei polyklonalen Anti-PDGF-Antikörpern: Eichkurven von PDGF-Standards.

Polystyrolplatten wurden mit Ziege-Anti-PDGF-AB-IgG (polyklonal, von Collaborative Research) beschichtet; nach Inkubation mit verschiedenen PDGF-Standards (s. unten) wurde gebundenes PDGF mit Hilfe von polyklonalem Kaninchen-Anti-PDGF-AA bzw. -Anti-PDGF-BB, gefolgt von Peroxidase-markiertem Anti-Kaninchen-IgG detektiert.
Bei der Anwendung von Anti-PDGF-AA (ELISA I.1) erhält man O.D.-Signale in der Reihenfolge: PDGF-AB > PDGF-AA >> PDGF-BB (7.1). Mit Anti-PDGF-BB (ELISA I.2) ergeben sich maximale O.D.-Werte für PDGF-AB und -BB ab 10 ng/ml, PDGF-AA liefert bis 1000 ng/ml kein Signal (7.2).
[Quelle der Standards: AB: aus humanen Thrombozyten, von Promega Corp. No. G 6191; BB: rekomb. aus Hefe, von Promega Corp. No. G 5191; AA: rekomb. aus BHK-Zellen, ca. 70 %ig, (Eichner et al., 1989)].

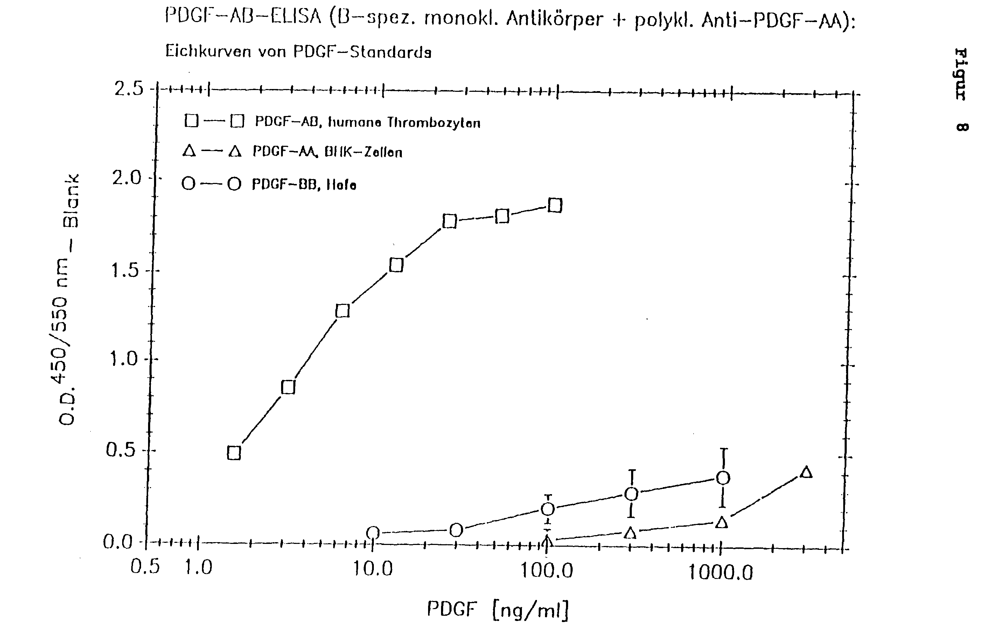

### Fig. 8) Sandwich-ELISA zum Nachweis von PDGF-AB mit Hilfe eines monoklonalen und eines polyklonalen Anti-PDGF-Antikörpers: Eichkurven von PDGF-Standards.

Polystyrolplatten wurden mit Schaf-Anti-Maus-IgG beschichtet und anschließend mit einem Maus-Hybridoma-Überstand (von Klon 1B3, enthält monoklonale Antikörper gegen die B-Kette in PDGF-AB und -BB) inkubiert; nach Inkubation mit verschiedenen PDGF-Standards (s. Legende zu Fig. 7) wurde PDGF-AB mit Hilfe eines polyklonalen Kaninchen-Anti-PDGF-AA, gefolgt von Peroxidase-markiertem Anti-Kaninchen-IgG nachgewiesen.
Für PDGF's aus eukaryontischen Quellen ergibt sich ein spezifisches Signal mit PDGF-AB (aus humanen Thrombozyten) mit einer geringen Kreuzreaktion mit PDGF-BB.

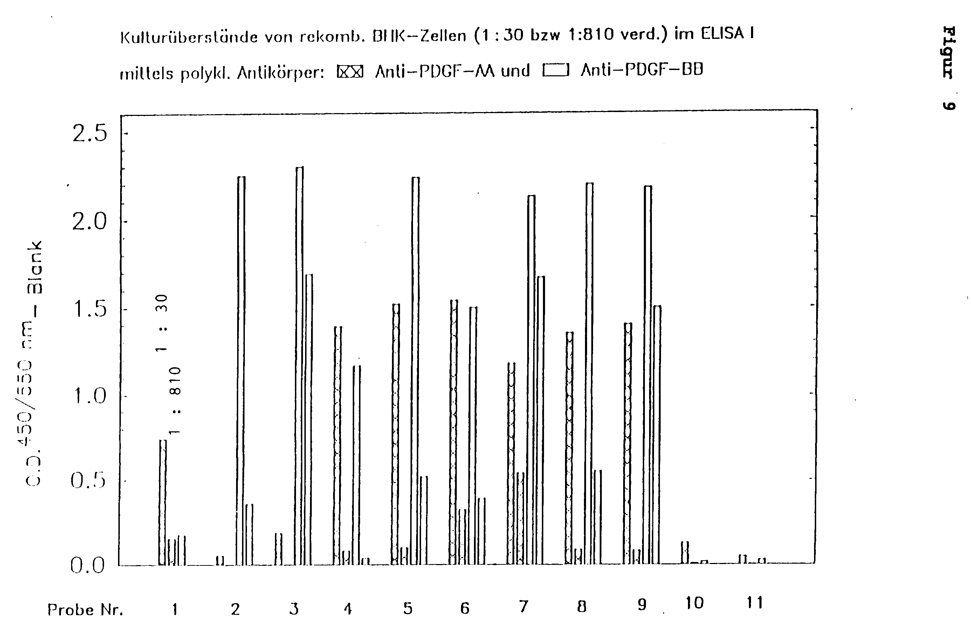

### Fig. 9) Nachweis von PDGF-A-Kette bzw. B-Kette in Kulturüberständen von rekombinanten BHK-Zellen mittels ELISA I:

Eichkurven von Standards s. Fig. 7.1 und 2; die Proben stammten von BHK-Zellen, die mit folgenden Genen transfiziert worden waren:
Probe 1: pSBC-2-PDGF-A; Probe 2: pSBC-2-PDGF-B ; Probe 3: pSBC-2-G-PDGF-B190; Probe 4: pSBC-PDGF-A/B; Probe 5: pSBC-PDGF-B/A; Probe 6: pSBC-PDGF-A/-G-B190 Probe 7: pSBC-PDGF-B190/-G-A; Probe 8: pSBC-2-PDGF-A + pSBS-2-PDGF-B; Probe 9: pSBC-2-PDGF-A + pSBC-2-G-PDGF-B190; Probe 10: pSBC-Luc/-G-Seap; Probe 11: pSBC-Seap/ G-Luc.

**Tabelle 1)**

| Steigerung der Expression von Reportergenen durch eine insertierte zelluläre Sequenz (Globin) in mono- und bicistronischen Vektoren | | |
|---|---|---|
| links: schematische Darstellung der DNA-Konstrukte | | |
| | DNA | erwartete Größe der mRNA |
| 1) | pSBC-2-LUC | 1870 nt |
| 2) | pSBC-1-LUC | 2497 |
| 3) | pSBC-2-G-LUC | 1904 |
| 4) | pSBC-1-G-LUC | 2531 |
| 5) | pSBC-SEAP/LUC | 4407 |
| 6) | pSBC-G-SEAP/LUC | 4441 |
| 7) | pSBC-SEAP/G-LUC | 4441 |
| 8) | pSBC-SEAP/LUC (Delta) Polio | 3780 |
| 9) | pSBC-LUC/SEAP | 4407 |

- L =: Strukturgen für Luciferase
- S =: Strukturgen für sekretierte alkalische Phosphatase
- IRES =: "internal ribosomal entry site"
- G =: Sequenz aus *Xenopus laevis* Globin mRNA
- pA =: poly Adenylierungssite aus SV40

### Mitte: Northern Blot Analyse

Die mRNA aus dem Gesamtpool der BHK-Zellen, die stabil mit den monocistronischen und bicistronischen Expressionskonstrukten für LUC und SEAP transfiziert worden waren, wurde untersucht. Die RNA wurde nach Purchio et al. (1979) isoliert, über ein 1%iges Agarose-Formaldehydgel fraktioniert (Lehrach et al., 1977), auf eine Nylonmembran geblottet und mit [³²P]-markierten Actin-, LUC- und SEAP-spezifischen Sonden hybridisiert. Erwartungsgemäß zeigen die monocistronischen mRNAs eine Größe von etwa 1900 - 2500 nt, während bei den bicistronischen mRNAs die Größe der codierenden Sequenzen beider Reportergene (etwa 3800 - 4400 Nucleotide) vorhanden ist. Hiermit ist gezeigt, daß die entsprechenden Genprodukte von einer einzigen bicistronischen mRNA abgelesen werden.

### rechts: Ergebnisse für Luciferase- und SEAP-Expression

Die Ergebnisse wurden ermittelt, wie unter 1.1 und 1.2 beschrieben.

### Tabelle 2) Produktivität der mono- und bicistronischen Expressionsvektoren für die PDGF-Ketten A und B in BHK-zellen

Die PDGF-Konzentration in den Kulturüberständen wurde mit Hilfe des Mitogentests ermittelt. Der spezifische Nachweis von PDGF-AB erfolgte durch ELISA II (s. 2.3, Eichkurven von Standards s. Fig. 8).

### II. Beispiele:

Die in den Beispielen aufgeführten Anwendungen zur Expression basieren auf monocistronischen und bicistronischen Transkriptionseinheiten. Die zu exprimierenden Gene werden jeweils in die Vektoren pSBC-1 bzw. pSBC-2 integriert. Die Vektorkonstruktion vereinfacht das Rekombinieren von pSBC-1 und pSBC-2 zum bicistronischen Vektor, wie es in den Fig. 2A - 2C für die Expression der Gene LUC und SEAP und in den Fig. 6A - 6C am Beispiel der Gene von PDGF-A und PDGF-B gezeigt ist. Nach Transfer des Plasmids pSBC-PDGF-A/-G-B190 (G = β-Globin-Sequenz aus *Xenopous laevis* gemäß SEQ ID Nr.4) in Animalzellen wird PDGF-A cap-abhängig und PDGF-B in Abhängigkeit vom Polio-IRES translatiert. In entsprechender Weise werden pSBC-PDGF-B190/-G-A sowie die Reportergene LUC und SEAP von mono- bzw. bicistronischen mRNA-Molekülen translatiert.

### Beispiel 1: Expression der Reportergene LUC und SEAP mit Hilfe des bicistronischen Vektorsystems

### 1.1 Nachweisverfahren für Luciferase

Die Luciferase ist in Zellextrakten enthalten. Ihre Aktivität kann durch Zugabe von Luciferin (Substrat), ATP und Mg²⁺ quantitativ bestimmt werden und als Maß der Aktivität des Luciferase-gens gelten. Dabei spielt sich folgende Reaktion ab (de Wet et al., 1987):
Luciferase + Luciferin + ATP + Mg²⁺ ←→ Luciferase • Luciferyl-AHP + PPᵢ
Luciferase • Luciferyl-AMP + O₂ → Luciferase + Oxyluciferin + AMP + CO₂ + *hv*

### 1.2 Nachweisverfahren für sekretierte alkalische Phosphatase

Alkalische Phophatase ist ein Enzym, das die Hydrolyse von gebundenem Phosphat katalysiert. Das membranständige, in Eukaryonten vorkommende Enzym verfügt über einen Glykophospholipidanker, mit dem es C-terminal mit der Membran verbunden ist. Da sezernierte Proteine oft bequemer nachweisbar sind als zellinterne oder membranständige, wurde in die Sequenz der alkalischen Phosphatase aus humaner Placenta (513 Aminosäuren) an Position 489 ein künstliches Terminations-Translationssignal eingeführt (Berger et al., 1988). Die nach Transfektion des entsprechenden Expressionsplasmids hergestellte Proteinmutante wird effizient ins Medium sekretiert und eignet sich hervorragend als Reportermolekül (SEAP = sekretierte alkalische Phosphatase). Der Nachweis kann kolorimetrisch oder luminometrisch erfolgen (Berger et al., 1988).

### 1.3 Herstellung transformierter BHK-Zellen

Die Transfektion der mono- und bicistronischen Expressionsvektoren, die die codierenden Sequenzen der Reportergene LUC und SEAP bzw. der PDGF-A- und B-Kette tragen (vergl. Fig. 2A-C, 6A-C) wurde mit der Calciumphosphat-Präzipitationstechnik in BHK-Zellen durchgeführt (Wigler et al., 1979; Graham & van der Eb, 1973). Einen Tag vor der Transfektion wurden 2-3 x 10⁵ BHK-Zellen/24 cm² in neue Kulturflaschen umgesetzt. Vier Stunden vor der Transfektion wurde ein Medienwechsel mit DME-Medium durchgeführt. 5 µg der o. g. Plasmid-DNA wurden zusammen mit 0,5 µg der Selektionsplasmide pAG60 und pSV2pac (Colbére-Garapin, 1981; Vara et al., 1986), welche für ein Neomycinresistenzgen bzw. für eine Puromycin-Resistenz kodieren, wurden in 250 µl 250 mM CaCl₂ suspendiert. Die Lösung wurde langsam unter ständiger Verwirbelung durch steril eingeblasene Luft zu 250 µl 2 x HEPES-Puffer (280 mM NaCl; 50 mM HEPES; 1,5 mM NaH₂PO₄ pH 7,1) gegeben und und das erhaltene Präzipitat dem Nährmedium zugesetzt. Zwei Tage nach der Transfektion wurde durch Medienwechsel von DME- auf Doppelselektionsmedium (5 µg/ml Puromycin; 500 µg/ml G418) (Wirth et al., 1988) die Selektion auf stabil transfizierte Zellen begonnen. Repräsentative Klone der PDGF produzierenden bzw. LUC/SEAP produzierenden BHK-Zellen wurden am 11. 8. 1992 bei der DSM wie folgt hinterlegt:
- pSBC-PDGF-A/-G-B190 = DSM ACC2048
- pSBC-PDGF-B190/-G-A = DSM ACC2049
- pSBC-SEAP/-G-LUC = DSM ACC2046
- pSBC-G-SEAP/LUC = DSM ACC2047

### 1.4 Expression äquimolarer Mengen der Genprodukte LUC und SEAP durch Einführung einer translationssteigernden Sequenz vor das IRES-regulierte Cistron

Die Ergebnisse aus den Untersuchungen mit den Reportergenkonstrukten pSBC-LUC/SEAP und pSBC-SEAP/LUC in Tab. 1 zeigen, daß die Expression der IRES-abhängigen Translation im bicistronischen Konstrukt immer deutlich geringer ist als die im cap-abhängig translatierten Cistron. Dies entspricht den aus der Literatur bekannten Werten. Die β-Globin Sequenz aus *Xenopous laevis* (SEQ ID Nr. 6) wurde in den mono- und bicistronischen Reportergenkonstrukten in die singuläre NotI-Schnittstelle insertiert (Fig. 2C). In den bicistronischen Expressionsvektoren befindet sie sich unmittelbar zwischen Promotor und 5'UTR des ersten Cistrons bzw. zwischen dem IRES-Element und dem 5'-UTR des zweiten Cistrons.
Die Steigerung der Translationseffizienz der einzelnen Cistrons wurde anhand der Reportergenkonstrukte, wie sie in Fig. 2A - 2C dargestellt sind, gemessen. In Tabelle 1 ist gezeigt, daß die β-Globin-Sequenz die cap-abhängige Translation von Luciferase in der monocistronischen Expressionseinheit um den Faktor 5, die IRES-abhängige Translation in bicistronischen Expressionseinheiten um den Faktor 3 stimuliert. Letzteres führt zur äquimolaren Expression der Cistrons 1 und 2 in bicistronischen Vektoren. Der in der Tabelle 1 dargestellte Northern Blot zeigt, daß die entsprechenden Genprodukte von einer mono- bzw. bicistronischen mRNA abgelesen werden. Dadurch, daß die spezifischen mRNA-Konzentrationen in der gleichen Größenordnung in den Zellen vorhanden sind, ist bewiesen, daß die expressionssteigernde Wirkung der Globinsequenz sich auf der Ebene der Translation vollzieht.

Die äquimolare Expression der Genprodukte des ersten und zweiten Cistrons wurde durch das Einführen des 5'UTR des Globingens aus *Xenopous laevis* (SEQ ID Nr. 6) erreicht.

### Beispiel 2: Expression von PDGF-AB Heterodimer mit Hilfe des bicistronischen Vektorsystems

### 2.1 Gewinnung konditionierter Zellkulturüberstände

Die Transformation der BHK-Zellen erfolgte analog 1.3. Nach dem Auszählen der Kolonien werden die Zellen abtrypsinisiert, in frischem Selektionsmedium aufgenommen und auf eine Zellzahl von 10⁵ Zellen/ml eingestellt. Je 10 ml dieser Zellsuspension werden in eine Flasche mit 65 cm² Bodenfläche überführt und für weitere 24 h kultiviert. Danach wird das Medium entfernt, der Zellrasen 2x mit PBS gewaschen und das Medium durch 10 ml Produktionsmedium (DMEM, ohne Serum und Selektions-Antibiotoka) ersetzt. Nach 24 h wird das Medium abgenommen. Die geernteten Überstände werden bis zur Analyse bei -20°C gelagert. Die Zellen werden gezählt und in flüssigem Stickstoff gelagert. Zum Zeitpunkt der Ernte beträgt die Zellzahl/Flasche 0.8-1.2x10⁷.

### 2.2 Nachweis von PDGF in den Kulturüberständen mit Hilfe des Mitogentests

Die Messung der Stimulierung der DNA-Syntheserate von dichtearretierten Fibroblasten erlaubt eine Bestimmung der mitogenen Aktivität des PDGF. Eine Unterscheidung zwischen den Isoformen ist dabei nicht möglich, da alle PDGF-Spezies in diesem Test biologisch aktiv sind.

Der Assay wurde gemäß Shipley et al. (1984) mit AKR-2B-Mausfibroblasten in 24-Well-Platten durchgeführt. Reines PDGF zeigt in dem Test eine halbmaximale Stimulierung bei einer Konzentration von etwa 5 ng/ml. Dieser Wert wurde benutzt, um Produktivitäten zu bestimmen. Die Ergebnisse aus dem Mitogentest sind in Tab. 2 den Werten aus dem PDGF-AB-ELISA gegenübergestellt.

### 2.3 Nachweis von PDGF-AB Heterodimer in den Kulturüberständen mit Hilfe von PDGF-ELISA's

Es wurden zwei 'two-antibody sandwich assays' aufgebaut, die I.) eine grobe Quantifizierung der PDGF-A- bzw. der -B-Kette in PDGF-Dimeren und II.) eine spezifische Quantifizierung von PDGF-AB neben PDGF-AA und -BB erlauben.
**I. Sandwich-Assay mit Hilfe von zwei polyklonalen Anti-PDGF-Antikörpern**
   Polystyrolplatten mit 96 Kavitäten (Fa. Dynatech, U-Platte, No. M124B) werden in folgender Reihenfolge beschichtet (zwischen jedem Schritt jeweils 4 x Waschen mit PBS mit 0,05 % Tween 20):
   I.1 Polyklonales Ziege-Anti-PDGF-AB-IgG (Fa. Collaborative Research, No. 40017); bindet PDGF-AB, -BB und in geringem Maße -AA), 2 µg/ml in 0,05 M Carbonat/Bicarbonat-Puffer, 50 µl über Nacht bei 4 °C
   I.2 % BSA (Fa. E. Merck, Nr. 12018) in PBS, pH 7,5, 100 µl für 1 h bei R.T.
   I.3 PDGF-haltige Lösungen, verdünnt in PBS mit 0,1 % BSA und 0,05 % Tween 20 (PBS+), 50 µl für 1 h bei R.T.
   I.4.1 Polyklonales Kaninchen-Anti-PDGF-AA-IgG (Fa. Genzyme, No. ZP-214, bindet an der A-Kette von dimerem PDGF), 2 µg/ml in PBS+, 50 µl für 1 h bei R.T. (ELISA I.1) bzw.
   I.4.2 Polyklonales Kaninchen-Anti-PDGF-BB-IgG (Fa. Genzyme, No. ZP-215, bindet an der B-Kette von dimerem PDGF), wie I.4.1 (ELISA I.2)
   I.5 POD-markiertes Ziege-Anti-Kaninchen IgG (Fa. Pierce, No. 31460), 0,1 µg/ml in PBS+, 50 µl für 1 h bei R.T., Detektion mit dem Substrat Tetramethylbenzidin gemäß E.S. BOS et al. (J. Immunoassay 2 (1981), 187-204).
**II. Sandwich-Assay mit Hilfe eines monoklonalen und eines polyklonalen Anti-PDGF-Antikörpers**
   Dieselben Platten wie im ELISA I werden in folgender Reihenfolge beschichtet (Mengen, Puffer und Inkubationszeiten wie oben):
   II.1 Schaf-Anti-Maus-IgG (Fa. Boehringer Mannheim, Nr. 1097 105), 3 µg/ml.
   II.2 1 % BSA in PBS
   II.3 Maus-Hybridoma-Überstand von Klon 1B3 [erhalten durch Fusion von SP2/O-Myelomzellen mit Milzzellen von Mäusen, die mit rekomb. PDGF-AB (aus *E.coli* gemäß J. Hoppe et al., 1990) immunisiert worden waren], 2 µg/ml IgG2a/ml. Der monoklonale Antikörper bindet spezifisch an der B-Kette von PDGF-Dimeren.
   II.4 PDGF-haltige Lösungen
   II.5 Polyklonales Kaninchen-Anti-PDGF-AA-IgG (s. I.4.1), 2 µg/ml
   II.6 wie I.5

### 2.4 Expression äquimolarer Mengen der PDGF-Ketten A und B durch Einführung einer translationssteigernden Sequenz vor das IRES-regulierte Cistron

Bicistronische Konstrukte mit dem wie in Fig. 5 beschriebenen mutierten PDGF-B sollten zur Expression der PDGF-Ketten A und B im Verhältnis 3:1, entsprechend der Konstellation im bicistronischen Vektor führen. Die äquimolare Expression beider Gene wurde durch das Einführen von translationssteigernden Sequenzen in den 3'- Bereich der internen ribosomalen Eintrittsstelle des Polio-Elements erreicht. Ein solches Element ist z.B. die β-Globin-Sequenz aus *Xenopous laevis* (SEQ ID Nr. 6). Diese β-Globin-Sequenz (Oligomer G) wurde in den bicistronischen Vektoren in die singuläre NotI-Schnittstelle insertiert (Fig. 6C). In den daraus resultierenden Plasmiden befindet sie sich unmittelbar zwischen dem IRES-Element und dem 5'-UTR des zweiten Cistrons.

### 2.5 Ergebnisse:

In Figur 9 und Tabelle 2 sind die Resultate von drei unterschiedlichlichen Analysen für PDGF aus Kulturüberständen rekombinanter BHK-Zellen dargestellt.

Durch den ELISA I ist es möglich, eine grobe Aussage über die Mengenanteile beider PDGF-Ketten zu treffen. Somit können Rückschlüsse auf die Effizienz der intercistronischen Elemente gemacht und bicistronische Konstukte charakterisiert werden, in denen annähernd gleiche Mengen von PDGF-A und -B translatiert werden. Hierbei ist allerdings zu berücksichtigen, daß in ELISA I.1 PDGF-AB ein stärkeres Signal als PDGF-AA ergibt.
Der Mitogentest liefert einen brauchbaren Wert für die Gesamtmenge des in den Kulturüberständen vorhandenen rPDGF, ohne zwischen den verschiedenen Isoformen (PDGF-AA, AB oder BB) differenzieren zu können.
Der spezifische Anteil an heterodimerem PDGF-AB kann durch den PDGF-AB-spezifischen ELISA II bestimmt werden. Aus der Differenz dieser Analyse zum Ergebnis des Mitogentests kann der prozentuale Anteil von PDGF-Homodimeren mit hoher Genauigkeit ermittelt werden.

### Abkürzungen:

- B190 -: C-terminal verkürzter PDGF-B-precursor (DNA)
- B* -: PDGF-B-Kette (Protein), hervorgegangen aus verkürztem PDGF-B-precursor
- BHK -: Hamsterzellinie
- bp -: Basenpaar(e)
- BSA -: Rinderserumalbumin
- CHO -: Hamsterzellinie
- DMEM -: Dulbecco's Modified Eagle Medium
- ELISA -: enzyme-linked immunosorbent assay
- G -: β-Globin-Sequenz aus *Xenopous laevis*
- IgG -: Immunglobulin der Klasse G
- IRES -: internal ribosomal entry site
- LUC -: Luciferase
- nt -: Nukleotid(e)
- PBS -: phosphatgepufferte Kochsalzlösung
- PCR -: Polymerase Kettenreaktion
- PDGF -: Wachstumsfaktor aus Thrombozyten
- SEAP -: sekretierte alkalische Phosphatase
- UTR -: nicht translatierte Region

### LITERATUR

**Adam M. A., Ramesh N., Miller A. D., and Osborne W. R. A.** (1991) J. Virol. **65**, 4985-4990.

**Artelt P., Morelle C., Ausmeier M., Fitzek M., and Hauser H.** (1988) Gene **68**, 213-219.

**Beckmann M. P., Betsholtz C., Heldin C.-H., Westermark B., Di Marco E., Di. Fiore P. P., Robbins K. C., and Aaronson S. A.** (1988) Science **241**, 1344-1349.

**Berger J., Hauber J., Hauber R., Geiger R., Cullen B. R.** (1988) Gene **66**, 1-10.

**Berkner K. L. and Sharp P. A.** (1985) Nucl. Acids Res. **13**, 841-857.

**Betsholtz C., Johnsson A., Heldin C.-H., Westermark B., Lind P., Urdea M. S., Eddy R., Shows T. B., Philpott K., Mellor A. L., Knott T. J., and Scott J.** (1986) Nature **320,** 695-699.

**Block L. H., Emmons L. R., Vogt E., Sachinidis A., Vetter W., and Hoppe J.** (1989) Proc. Natl. Acad. Sci. USA **86**, 2388-2392.

**Boel E., Berkner K. L., Nexoe B. A., and Schwartz T. W.** (1987) FEBS Lett. **219**, 181-188.

**Bywater M., Rorsman F., Bongcam-Rudloff E., Mark G., Hammacher A., Heldin C.-H., Westermark B., and Betsholtz C.** (1988) Mol. Cell. Biol. **8**, 2753-2762.

**Colbére-Garapin F., Horodniceanu F., Kourilsky P., and Garapin A. C.** (1981) J. Mol. Biol. **150**, 1-14.

**de Wet, J. R., Wood K. V., DeLuca M., Helinski D. R. and Subramani S.** (1987) Mol. Cell. Biol. **7**, 725-737.

**Eichner W., Jäger V., Herbst D., Hauser H. and Hoppe J.** (1989) Eur. J. Biochem. **185**, 135-140.

**Falcone D., and Andrews D.W.** (1991) Mol. Cell. Biol. **11** (5), 2656-2664.

**Gallie D. R., Sleat D. E., Watts J. W., Turner P. C. and Wilson T. M. A.** (1987A) Nucl. Acids Res. **15**, 3257-3272.

**Gallie D. R., Sleat D. E., Watts J. W., Turner P. C. and Wilson T. M. A.** (1987B) Nucl. Acids Res. **15**, 8692-8711.

**Gallie D. R., Sleat D. E., Watts J. W., Turner P. C. and Wilson T. M. A.** (1988) Nucl. Acids Res. **16**, 883-893.

**Ghattas I. R., Sanes J. R., and Majors J. E.** (1991) Mol. Cell. Biol. **22**, 5848-5859.

**Graham F., and van der Eb L.** (1973) Virology **52**, 456-487.

**Hambidge S. J., and Sarnow P.** (1991) J. Virol. **65**, 6312-6315.

**Hammacher A., Hellmam U., Johnsson A., Östman A., Gunnarsson K., Westermark B., Wasteson Å., and Heldin C.-H.** (1988) J. Biol. Chem. **263**, 16493-16499.

**Hart C. E., Forstrom J. W., Kelly J. D., Seifert R. A., Smith R. A., Ross R., Murray M. J., and Bowen-Pope D. F.** (1988) Science **240**, 1529-1531.

**Hart C. E., Bailey M., Curtis D. A., Osborn S., Raines E., Ross R., and Forstrom J. W.** (1990) Biochemistry **29**, 166-172.

**Heldin C.-H., Johnsson A., Wennergren S., Wernstedt C., Betsholtz C., and Westermark B.** (1986) Nature **319**, 511-514.

**Heldin C.-H., Bäckström G., Östman A., Hammacher A., Rönnstrand L., Rubin K., Nister M., and Westermark B.** (1988) EMBO J. **7**, 1387-1393.

**Hoppe J., Schumacher L., Eichner W. and Weich H.A.** (1987), FEBS Lett. **223**, 243-246.

**Hoppe J., Weich H. A., and Eichner W.** (1989) Biochemistry **28**, 2956-2960.

**Hoppe J., Weich H. A., and Eichner W., and Tatje D.** (1990) Eur. J. Biochem. **187**, 207-214.

**Hosang M., Rouge M., Wipf B., Eggiman B., Kaufmann F., and Hunziker W.** (1989) J. Cell. Physiol. **149**, 558-564.

**Jackson R. J., Howell M. T., and Kaminski A.** (1990) Trends Biochem. Sci. **15**, 477-483.

**Jang S. K., Kräusslich H., Nicklin M. J. H., Duke G. M., Palmenberg A. C., and Wimmer E.** (1988) J. Virol. **62**, 2636.

**Jang S. K., Davies M. V., Kaufmann R. J., and Wimmer E.** (1989) J. Virol. **63** (4), 1651-1660.

**Jang S. K., and Wimmer E.** (1990) Genes Dev. **4**, 1560-1572.

**Jobling S. A. and Gehrke L.** (1987) Nature **325**, 622-625.

**Johnsson A., Heldin C.-H., Wasteson A., Westermark B., Deuel T. F., Huang J. S., Seeburg P. H., Gray A., Ullrich A., Scrace G., Stroobant P., Waterfield M. D.** (1984) EMBO J. **136**, 921-928.

**Kaufman R. J.** (1985) Proc. Natl. Acad. Sci. (USA) **82**, 689-693.

**Kaufman R. J., Murtha P., and Davies M. V.** (1987) EMBO J. **6**, 187-193.

**Kaufman R. J., Davies M. V. Wasley L. C., and Michnick D.** (1991) Nucleic Acids Res. **19**, 4485-4490.

**Kelly J. D., Raines E. W., Ross R., and Murray M. J.** (1985) EMBO J. **4**, 3399-3405.

**Klausner R. D. and Harford J. B.** (1989) Science **246**, 870-872.

**Knoechel W.. Korge E., Basner A., and Meyerhof W.** (1986) J. Mol. Evol. **23**, 211-223.

**Kozak M.** (1987) Mol. Cell. Biol. **7** (10), 3438-3445.

**Kozak M.** (1989) Mol. Cell. Biol. **9**, 5134-5142.

**La Rochelle W. J., Giese N., May-Siroff M., Robbins K. C., and Aaronson S. A.** (1990) Science **248**, 1541-1544.

**La Rochelle W. J., May-Siroff M., Robbins K. C., and Aaronson S. A.** (1991) Genes & Development **5**, 1191-1199.

**Lehrach H., Diamond D., Wozney J. M., and Boedtker H.** (1977) Biochemistry **16**, 4743-4751.

**Macejak D. G., and Sarnow P.** (1991) Nature (London) **353**, 90-94.

**Matoskova B., Rorsman F., Svensson V. and Betsholtz C.** (1989), Mol. Cell. Biol. **9**, 3148-3150.

**Meerovitch K., Pelletier J., and Sonenberg N.** (1989) Genes Dev. **3**, 1026-1034.

**Millan, J.L.** (1986) J. Biol. Chem. **261**, 3112-3115

**Nakamaye K. and Eckstein F.** (1986) Nucl. Acids Res. **14**, 9679-9698.

**Nister M., Hammacher A., Mellström K., Siegbahn A., Rönnstrang L., Westermark B., and Heldin C.-H.** (1988); Cell **52**, 791-799.

**Östman A., Rall L., Hammacher A., Wormstead M. A., Coit D., Valenzuela P., Betsholtz C., Westermark B., and Heldin C.-H.** (1988) J. Biol. Chem. **263**, 16202-16208.

**Östman A., Andersson M., Betsholtz C., Westermark B., and Heldin C.-H.** (1991) Cell Regulation **2**, 503-512.

**Patient R. K., Harris R., Walmsley M. E. and Williams J. G.** (1983) J. Biol. Chem. **258**, 8521-8523.

**Pelletier J., and Sonenberg N.** (1988) Nature **334**, 320.

**Purchio A. F. and Fareed G. C.** (1979) J. Virol. **29**, 763-769.

**Ratner L., Josephs S. F., Jarrett R., Reitz M. S. and Wong-Staal F.** (1985), Nucl. Acids Res. **13**, 5007-5018.

**Reilly C. F. and Broski J. E.** (1989) Biochem. Biophys. Res. Commun. **160**, 1047-1054.

**Robbins K. C., Leal F., Pierce J. H., and Aaronson S. A.** (1985) EMBO J. **4**, 1783-1792.

**Sachinidis A., Locher R., Vetter W., Tatje D., and Hoppe J.** (1990) J. Biol. Chem. **265**, 10238-10243.

**Sachinidis A., Locher R., Hoppe J., and Vetter W.** (1990) FEBS Lett. **275**, 95-98.

**Sarnow P.** (1989) J. Virol. **63**, 467-470.

**Sayers J. R., Schmidt W. and Eckstein F.** (1988) Nucl. Acids Res. **16**, 791-802.

**Shipley G. D., Childes C. B., Volkenant M. E. and loses H. L.** (1984) Cancer Res. **44**, 710-716.

**Siegbahn A., Hammacher A., Westermark B., and Heldin C.-H.** (1990) J. Clin. Invest. **85**, 916-920.

**Simoes E. A. F., and Sarnow P.** (1991) J. Virol. **65**, 913-921.

**Stroobant P., and Waterfield M. D.** (1984) EMBO J. **3,** 2963-2967.

**Taylor J. W., Ott J. and Eckstein F.** (1985) Nucl. Acids Res. **13**, 8764-8785.

**Vara J., Portela A., Oritin J. and Jimenez A.** (1986) Nucl. Acids Res. **14**, 4617-4624.

**Weich H. A., Sebald W., Schairer H. U., and Hoppe J.** (1986), FEBS Lett. **198**, 344-348.

**Wigler M., Sweet R., Sim G. K., Wold B., Pellicer A., Lacy E., Maniatis T., Silverstein S., and Axel R.** (1979) Cell **16**, 777-785.

**Wirth M., Bode J., Zettlmeißl G., and Hauser H.** (1988) Gene **73,** 419-426.

**Wirth M., Schumacher L., and Hauser H.** (1991) In Modern Approaches to Animal Cell Technology, Griffiths B., Spier R., and Meigner R., eds. Butterworths), pp. 338-343.

**Wise R. J., Orkin S. H. and Collins T.** (1989) Nucl. Acids Res. **17**, 6591-6601.

**Wood C. R., Morris G. E., Alderman E. M., Fouser L., and Kaufman R. J.** (1991) Proc. Natl. Acad. Sci. USA **88**, 8006-8010.

**Young R. M., Mendoza A. E., Collins T. and Orkin S. H.** (1990) Mol. Cell. Biol. **10**, 6051-6054.

### SEQUENZPROTOKOLL

### (1) ALLGEMEINE INFORMATION:

(i) ANMELDER:
   (A) NAME: Beiersdorf AG
   (B) STRASSE: Unnastr. 48
   (C) ORT: Hamburg
   (E) LAND: Bundesrepublik Deutschland
   (F) POSTLEITZAHL: 20245

   (A) NAME: GBF - Gesellschaft fuer Biotechnologische Forschung mbH
   (B) STRASSE: Mascheroder Weg 1
   (C) ORT: Braunschweig
   (E) LAND: Bundesrepublik Deutschland
   (F) POSTLEITZAHL: 38124
(ii) ANMELDETITEL: Multicistronische Expressionseinheiten und deren Verwendung
(iii) ANZAHL DER SEQUENZEN: 25
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 748 Basenpaare
   (B) ART: Nukleinsäure
   (C) STRANGFORM: Einzel
   (D) TOPOLOGIE: linear
(ii) ART DES MOLEKÜLS: cDNS
(vi) URSPRÜNGLICHE HERKUNFT:
   (A) ORGANISMUS: Homo sapiens
(vii) UNMITTELBARE HERKUNFT:
   (B) CLON: pODA (Eichner et al., 1989)
(ix) MERKMALE:
   (A) NAME/SCHLÜSSEL: CDS
   (B) LAGE: 95..682
   (D) SONSTIGE ANGABEN: /product= "PDGF-A Vorlaeufersequenz (kurze Spliceform)"
      /note= "humanes PDGF-A Gen (kurze Spliceform, [2]) aus pODA. flankiert von 5'-EcoRI und 3'-HindIII Restriktionsschnittstellen"
      /citation= ([2])
(ix) MERKMALE:
   (A) NAME/SCHLÜSSEL: mat_peptide
   (B) LAGE: 353..682
   (D) SONSTIGE ANGABEN: /product= "mature PDGF-A Kette"
(x) VERÖFFENTLICHUNGSINFORMATION:
   (A) AUTOREN:
      Eichner, W.
      Jaeger, V.
      Herbst, D.
      Hauser, H.
      Hoppe, J.
   (C) ZEITSCHRIFT: Eur. J. Biochem.
   (D) BAND: 185
   (F) SEITEN: 135-140
   (G) DATUM: 1989
(x) VERÖFFENTLICHUNGSINFORMATION:
   (A) AUTOREN:
      Hoppe, J.
      Schumacher, L.
      Eichner, W.
      Weich, H. A.
   (C) ZEITSCHRIFT: FEBS Lett.
   (D) BAND: 223
   (F) SEITEN: 243-246
   (G) DATUM: 1987
(xi) SEQUENZBESCHREIBUNG: SEQ ID NO: 1:
   GAATTCCCAC TGAATTTCGC CGCCACAGGA GACCGGCTGG AGCGCCCGCC CCGCGCCTCG        60
   CCTCTCCTCC GAGCAGCCAG CGCCTCGGGA CGCG ATG AGG ACC TTG GCT TGC           112
                                         Met Arg Thr Leu Ala Cys
                                         -86 -85
   CTG CTG CTC CTC GGC TGC GGA TAC CTC GCC CAT GTT CTG GCC GAG GAA         160
   Leu Leu Leu Leu Gly Cys Gly Tyr Leu Ala His Val Leu Ala Glu Glu
   -80                 -75                 -70                 -65
   GCC GAG ATC CCC CGC GAG GTG ATC GAG AGG CTG GCC CGC AGT CAG ATC         208
   Ala Glu Ile Pro Arg Glu Val Ile Glu Arg Leu Ala Arg Ser Gln Ile
                   -60                 -55                 -50
   CAC AGC ATC CGG GAC CTC CAG CGA CTC CTG GAG ATA GAC TCC GTA GGG         256
   His Ser Ile Arg Asp Leu Gln Arg Leu Leu Glu Ile Asp Ser Val Gly
               -45                 -40                 -35
   AGT GAG GAT TCT TTG GAC ACC AGC CTG AGA GCT CAC GGG GTC CAC GCC         304
   Ser Glu Asp Ser Leu Asp Thr Ser Leu Arg Ala His Gly Val His Ala
           -30                 -25                 -20
   ACT AAG CAT GTG CCC GAG AAG CGG CCC CTG CCC ATT CGG AGG AAG AGA         352
   Thr Lys His Val Pro Glu Lys Arg Pro Leu Pro Ile Arg Arg Lys Arg
       -15                 -10                  -5
   AGC ATC GAG GAA GCT GTC CCC GCT GTC TGC AAG ACC AGG ACG GTC ATT         400
   Ser Ile Glu Glu Ala Val Pro Ala Val Cys Lys Thr Arg Thr Val Ile
     1               5                  10                  15
   TAC GAG ATT CCT CGG AGT CAG GTC GAC CCC ACG TCC GCC AAC TTC CTG         448
   Tyr Glu Ile Pro Arg Ser Gln Val Asp Pro Thr Ser Ala Asn Phe Leu
                20                  25                  30
   ATC TGG CCC CCG TGC GTG GAG GTG AAA CGC TGC ACC GGC TGC TGC AAC         496
   Ile Trp Pro Pro Cys Val Glu Val Lys Arg Cys Thr Gly Cys Cys Asn
            35                  40                  45
   ACG AGC AGT GTC AAG TGC CAG CCC TCC CGC GTC CAC CAC CGC AGC GTC         544
   Thr Ser Ser Val Lys Cys Gln Pro Ser Arg Val His His Arg Ser Val
        50                  55                  60
   AAG GTG GCC AAG GTG GAA TAC GTC AGG AAG AAG CCA AAA TTA AAA GAA         592
   Lys Val Ala Lys Val Glu Tyr Val Arg Lys Lys Pro Lys Leu Lys Glu
    65                  70                  75                  80
   GTC CAG GTG AGG TTA GAG GAG CAT TTG GAG TGC GCC TGC GCG ACC ACA         640
   Val Gln Val Arg Leu Glu Glu His Leu Glu Cys Ala Cys Ala Thr Thr
                    85                  90                  95
   AGC CTG AAT CCG GAT TAT CGG GAA GAG GAC ACG GAT GTG AGG                 682
   Ser Leu Asn Pro Asp Tyr Arg Glu Glu Asp Thr Asp Val Arg
               100                 105                 110
   TGAGGATGAG CCGCAGCCCT TTCCTGGGAC ATGGATGTGG GGATCCGTCG ACCTGCAGCC       742
   AAGCTT                                                                  748

### (2) INFORMATION ZU SEQ ID NO: 2:

(i) SEQUENZ CHARAKTERISTIKA:
   (A) LÄNGE: 196 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 2:
   Met Arg Thr Leu Ala Cys Leu Leu Leu Leu Gly Cys Gly Tyr Leu Ala
   -86 -85                 -80                 -75
   His Val Leu Ala Glu Glu Ala Glu Ile Pro Arg Glu Val Ile Glu Arg
   -70                 -65                 -60                 -55
   Leu Ala Arg Ser Gln Ile His Ser Ile Arg Asp Leu Gln Arg Leu Leu
                   -50                 -45                 -40
   Glu Ile Asp Ser Val Gly Ser Glu Asp Ser Leu Asp Thr Ser Leu Arg
               -35                 -30                 -25
   Ala His Gly Val His Ala Thr Lys His Val Pro Glu Lys Arg Pro Leu
           -20                 -15                 -10
   Pro Ile Arg Arg Lys Arg Ser Ile Glu Glu Ala Val Pro Ala Val Cys
        -5                   1               5                  10
   Lys Thr Arg Thr Val Ile Tyr Glu Ile Pro Arg Ser Gln Val Asp Pro
                    15                  20                  25
   Thr Ser Ala Asn Phe Leu Ile Trp Pro Pro Cys Val Glu Val Lys Arg
                30                  35                  40
   Cys Thr Gly Cys Cys Asn Thr Ser Ser Val Lys Cys Gln Pro Ser Arg
            45                  50                  55
   Val His His Arg Ser Val Lys Val Ala Lys Val Glu Tyr Val Arg Lys
        60                  65                  70
   Lys Pro Lys Leu Lys Glu Val Gln Val Arg Leu Glu Glu His Leu Glu
    75                  80                  85                  90
   Cys Ala Cys Ala Thr Thr Ser Leu Asn Pro Asp Tyr Arg Glu Glu Asp
                    95                 100                 105
   Thr Asp Val Arg
               110

### (2) INFORMATION ZU SEQ ID NO: 3:

(i) SEQUENZ CHARAKTERISTIKA:
   (A) LÄNGE: 868 Basenpaare
   (B) ART: Nukleinsäure
   (C) STRANGFORM: Einzel
   (D) TOPOLOGIE: linear
(ii) ART DES MOLEKÜLS: cDNS
(vi) URSPRÜNGLICHE HERKUNFT:
   (A) ORGANISMUS: Homo sapiens
(vii) UNMITTELBARE HERKUNFT:
   (B) CLON: pMVW-2 (Weich et al., 1986)
(ix) MERKMALE:
   (A) NAME/SCHLÜSSEL: CDS
   (B) LAGE: 40..762
   (D) SONSTIGE ANGABEN: /product= "PDGF-B Vorlaeufersequenz"
      /note= "humanes PDGF-B Gen aus pGEM2-PDGF-B, flankiert von 5'-EcoRI und 3'-HindIII Restriktionsschnittstellen"
(ix) MERKMALE:
   (A) NAME/SCHLÜSSEL: mat_peptide
   (B) LAGE: 283..609
   (D) SONSTIGE ANGABEN: /product= "mature PDGF-B Kette"
(x) VERÖFFENTLICHUNGSINFORMATION:
   (A) AUTOREN:
      Weich, H. A.
      Sebald, W.
      Schairer, H. U.
      Hoppe, U.
   (C) ZEITSCHRIFT: FEBS Lett.
   (D) BAND: 198
   (F) SEITEN: 344-348
   (G) DATUM: 1986
(xi) SEQUENZBESCHREIBUNG: SEQ ID NO: 3:
GAATTCGAGC TCGCCCGGGG ATCCTCTAGA GTCGACACC ATG AAT CGC TGC TGG        54
   GCG CTC TTC CTG TCT CTC TGC TGC TAC CTG CGT CTG GTC AGC GCC GAG      102
   GGG GAC CCC ATT CCC GAG GAG CTT TAT GAG ATG CTG AGT GAT CAC TCG      150
   ATC CGC TCC TTT GAT GAT CTC CAA CGC CTG CTG CAC GGA GAC CCC GGA       198
   GAG GAA GAT GGG GCC GAG TTG GAC CTG AAC ATG ACC CGC TCC CAC TCT       246
   GGA GGC GAG CTG GAG AGC TTG GCT CGT GGA AGA AGG AGC CTG GGT TCC       294
   CTG ACC ATT GCT GAG CCG GCC ATG ATC GCC GAG TGC AAG ACG CGC ACC       342
   GAG GTG TTC GAG ATC TCC CGG CGC CTC ATA GAC CGC ACC AAC GCC AAC       390
   TTC CTG GTG TGG CCG CCC TGT GTG GAG GTG CAG CGC TGC TCC GGC TGC       438
   TGC AAC AAC CGC AAC GTG CAG TGC CGC CCC ACC CAG GTG CAG CTG CGA       486
   CCT GTC CAG GTG AGA AAG ATC GAG ATT GTG CGG AAG AAG CCA ATC TTT       534
   AAG AAG GCC ACG GTG ACG CTG GAA GAC CAC CTG GCA TGC AAG TGT GAG       582
   ACA GTG GCA GCT GCA CGG CCT GTG ACC CGA AGC CCG GGG GGT TCC CAG       630
   GAG CAG CGA GCC AAA ACG CCC CAA ACT CGG GTG ACC ATT CGG ACG GTG       678
   CGA GTC CGC CGG CCC CCC AAG GGC AAG CAC CGG AAA TTC AAG CAC ACG       726
   CAT GAC AAG ACG GCA CTG AAG GAG ACC CTT GGA GCC TAGGGGCATC            772
   GGCAGGAGAG TGTGTGGGCA GGGTTATTTA ATATGGTATT TGCTGTATTG CCCCCATGGC     832
   CCAATCGATC CCGTCGACCT GCAGGCATGC AAGCTT                               868

### (2) INFORMATION ZU SEQ ID NO: 4:

(i) SEQUENZ CHARAKTERISTIKA:
   (A) LÄNGE: 241 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 4:

### (2) INFORMATION ZU SEQ ID NO: 5:

(i) SEQUENZ CHARAKTERISTIKA:
   (A) LÄNGE: 628 Basenpaare
   (B) ART: Nukleinsäure
   (C) STRANGFORM: Einzel
   (D) TOPOLOGIE: linear
(vi) URSPRÜNGLICHE HERKUNFT:
   (A) ORGANISMUS: Poliovirus Typ 1 (Mahoney strain)
(vii) UNMITTELBARE HERKUNFT:
   (B) CLON: pGEM3-5'Polio (M) (4708 bp), (Sarnow, 1989)
(ix) MERKMALE:
   (A) NAME/SCHLÜSSEL: -
   (B) LAGE: 1..628
   (D) SONSTIGE ANGABEN: /note= "abgebildet sind die ersten 628 nt der 5' nicht-translatierten Region des Poliovirus Typ 1 (Mahoney)"
(ix) MERKMALE:
   (A) NAME/SCHLÜSSEL: -
   (B) LAGE: 610
   (D) SONSTIGE ANGABEN: /note= "nicht-authentische Sequenz auf Grund einer Basenpaarsubstitution von C nach G an der Position 610"
(x) VERÖFFENTLICHUNGSINFORMATION:
   (A) AUTOREN: Sarnow, P.
   (C) ZEITSCHRIFT: J. Virol.
   (D) BAND: 63
   (F) SEITEN: 467-470
   (G) DATUM: 1989
(xi) SEQUENZBESCHREIBUNG: SEQ ID NO: 5:
   TTAAAACAGC TCTGGGGTTG TACCCACCCC AGAGGCCCAC GTGGCGGCTA GTACTCCGGT     60
   ATTGCGGTAC CCTTGTACGC CTGTTTTATA CTCCCTTCCC GTAACTTAGA CGCACAAAAC    120
   CAAGTTCAAT AGAAGGGGGT ACAAACCAGT ACCACCACGA ACAAGCACTT CTGTTTCCCC    180
   GGTGATGTCG TATAGACTGC TTGCGTGGTT GAAAGCGACG GATCCGTTAT CCGCTTATGT    240
   ACTTCGAGAA GCCCAGTACC ACCTCGGAAT CTTCGATGCG TTGCGCTCAG CACTCAACCC    300
   CAGAGTGTAG CTTAGGCTGA TGAGTCTGGA CATCCCTCAC CGGTGACGGT GGTCCAGGCT    360
   GCGTTGGCGG CCTACCTATG GCTAACGCCA TGGGACGCTA GTTGTGAACA AGGTGTGAAG    420
   AGCCTATTGA GCTACATAAG AATCCTCCGG CCCCTGAATG CGGCTAATCC CAACCTCGGA    480
   GCAGGTGGTC ACAAACCAGT GATTGGCCTG TCGTAACGCG CAAGTCCGTG GCGGAACCGA    540
   CTACTTTGGG TGTCCGTGTT TCCTTTTATT TTATTGTGGC TGCTTATGGT GACAATCACA    600
   GATTGTTATG ATAAAGCGAA TTGGATTG                                       628

### (2) INFORMATION ZU SEQ ID NO: 6:

(i) SEQUENZ CHARAKTERISTIKA:
   (A) LÄNGE: 55 Basenpaare
   (B) ART: Nukleinsäure
   (C) STRANGFORM: Einzel
   (D) TOPOLOGIE: linear
(vi) URSPRÜNGLICHE HERKUNFT:
   (A) ORGANISMUS: Xenopus laevis (Falcone & Andrews; Patient et al.)
(ix) MERKMALE:
   (A) NAME/SCHLÜSSEL: -
   (B) LAGE: 12..55
   (D) SONSTIGE ANGABEN: /note= "beta-Globin Homologie; partielle Sequenz, flankiert v. Restriktionsschnittstellen."
(ix) MERKMALE:
   (A) NAME/SCHLÜSSEL: -
   (B) LAGE: 12..55
   (D) SONSTIGE ANGABEN: /note= "Die 5'-3'-Orientierung gilt f. InsertionzwischenPolio-UTR und Cistron 2 der bicistronischen Vektoren"
(x) VERÖFFENTLICHUNGSINFORMATION:
   (A) AUTOREN: Falcone, D. Andrews, D. W.
   (C) ZEITSCHRIFT: Mol. Cell. Biol.
   (D) BAND: 11
   (E) AUSGABE: 5
   (F) SEITEN: 2656-2664
   (G) DATUM: 1991
(x) VERÖFFENTLICHUNGSINFORMATION:
   (A) AUTOREN:
      Patient, R. K.
      Harris, R.
      Walmsley, M. E.
      Williams, J. G.
   (C) ZEITSCHRIFT: J. Biol. Chem.
   (D) BAND: 258
   (F) SEITEN: 8521-8523
   (G) DATUM: 1983
(xi) SEQUENZBESCHREIBUNG: SEQ ID NO: 6:
   GGCCGTCGAC GCTTGTTCTT TTTGCAGAAG CTCAGAATAA ACGCTCAACT TTGGC          55

### (2) INFORMATION ZU SEQ ID NO: 7:

(i) SEQUENZ CHARAKTERISTIKA:
   (A) LÄNGE: 17 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..17
   (D) SONSTIGE ANGABEN: /label= M1317MER
      /note= "synthetische DNA; M13 Sequenzierprimer (New England Biolabs GmbH), eingesetzt fuer PCR"
(xi) SEQUENZBESCHREIBUNG: SEQ ID NO: 7:
   GTAAAACGAC GGCCAGT 17

### (2) INFORMATION ZU SEQ ID NO: 8:

(i) SEQUENZ CHARAKTERISTIKA:
   (A) LÄNGE: 24 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..24
   (D) SONSTIGE ANGABEN: /label= M1324MER
      /note= "synthetische DNA; M13 reverser Sequenzierprimer (New England Biolabs GmbH), eingesetzt fuer PCR"
(xi) SEQUENZBESCHREIBUNG: SEQ ID NO: 8:
   AGCGGATAAC AATTTCACAC AGGA 24

### (2) INFORMATION ZU SEQ ID NO: 9:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..19
   (D) SONSTIGE ANGABEN: /label= NCCLSA1
      /note= "synthetische DNA; synthetischer Linker zur Umklonierung des verkuerzten PDGF-B Vorlaeufers aus pMVW-2 in den Bakteriophagen M13mp19"
(xi) SEQUENZBESCHREIBUNG: SEQ ID NO: 9:
   CATGGCCCAA TCGATCCCG 19

### (2) INFORMATION ZU SEQ ID NO: 10:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..19
   (D) SONSTIGE ANGABEN: /label= NCCLSA2
      /note= "synthetische DNA; synthetischer Linker zur Umklonierung des verkuerzten PDGF-B Vorlaeufers aus pMVW-2 in den Bakteriophagen M13mp19"
(xi) SEQUENZBESCHREIBUNG: SEQ ID NO: 10:
   TCGACGGGAT CGATTGGGC 19

### (2) INFORMATION ZU SEQ ID NO: 11:

(i) SEQUENZ CHARAKTERISTIKA:
   (A) LÄNGE: 37 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..37
   (D) SONSTIGE ANGABEN: /label= PDGBBCL
      /note= "synthetische DNA; Mutageneseprimer zur Einfuehrung einer BclI-Schnittstelle in den 5'-Bereich des PDGF-B Vorlaeufers"
(xi) SEQUENZBESCHREIBUNG: SEQ ID NO: 11:
   GCTTTATGAG ATGCTGAGTG ATCACTCGAT CCGCTCC 37

### (2) INFORMATION ZU SEQ ID NO: 12:

(i) SEQUENZ CHARAKTERISTIKA:
   (A) LÄNGE: 110 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..110
   (D) SONSTIGE ANGABEN: /label= PPDGFB1
      /note= "synthetische DNA; synthetischer Linker zur Rekonstitution der maturen PDGF-B Vorlaeufersequenz"
(xi) SEQUENZBESCHREIBUNG: SEQ ID NO: 12:
   TCGACACCAT GAATCGCTGC TGGGCGCTCT TCCTGTCTCT CTGCTGCTAC CTGCGTCTGG 60
   TCAGCGCCGA GGGGGACCCC ATTCCCGAGG AGCTTTATGA GATGCTGAGT 110

### (2) INFORMATION ZU SEQ ID NO: 13:

(i) SEQUENZ CHARAKTERISTIKA:
   (A) LÄNGE: 110 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..110
   (D) SONSTIGE ANGABEN: /label= PPDGFB2
      /note= "synthetische DNA; synthetischer Linker zur Rekonstitution der maturen PDGF-B Vorlaeufersequenz"
(xi) SEQUENZBESCHREIBUNG: SEQ ID NO: 13:
   GATCACTCAG CATCTCATAA AGCTCCTCGG GAATGGGGTC CCCCTCGGCG CTGACCAGAC 60
   GCAGGTAGCA GCAGAGAGAC AGGAAGAGCG CCCAGCAGCG ATTCATGGTG 110

### (2) INFORMATION ZU SEQ ID NO: 14:

(i) SEQUENZ CHARAKTERISTIKA:
   (A) LÄNGE: 30 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..30
   (D) SONSTIGE ANGABEN: /label= 5'-POLIO1
      /note= "synthetische DNA; synthetischer PCR-Primer"
(xi) SEQUENZBESCHREIBUNG: SEQ ID NO: 14:
   TTTCTGCAGA AGCTTAAAAC AGCTCTGGGG 30

### (2) INFORMATION ZU SEQ ID NO: 15:

(i) SEQUENZ CHARAKTERISTIKA:
   (A) LÄNGE: 28 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..28
   (D) SONSTIGE ANGABEN: /label= 3'-POLIO2
      /note= "synthetische DNA; synthetischer PCR-Primer"
(xi) SEQUENZBESCHREIBUNG: SEQ ID NO: 15:
   TTGCGGCCGC AATCCAATTC GCTTTATC 28

### (2) INFORMATION ZU SEQ ID NO: 16:

(i) SEQUENZ CHARAKTERISTIKA:
   (A) LÄNGE: 13 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..13
   (D) SONSTIGE ANGABEN: /label= E-N-E1
      /note= "synthetische DNA; synthetischer Linker"
(xi) SEQUENZBESCHREIBUNG: SEQ ID NO: 16:
   AATTGCGGCC GCG 13

### (2) INFORMATION ZU SEQ ID NO: 17:

(i) SEQUENZ CHARAKTERISTIKA:
   (A) LANGE: 13 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..13
   (D) SONSTIGE ANGABEN: /label= E-N-E2
      /note= "synthetische DNA; synthetischer Linker"
(xi) SEQUENZBESCHREIBUNG: SEQ ID NO: 17:
   AATTCGCGGC CGC 13

### (2) INFORMATION ZU SEQ ID NO: 18:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..16
   (D) SONSTIGE ANGABEN: /label= PDGFB190-PRIMI
      /note= "synthetische DNA; synthetischer PCR-Primer"
(xi) SEQUENZBESCHREIBUNG: SEQ ID NO: 18:
   GAATTCGAGC TCGCCCGGG 19

### (2) INFORMATION ZU SEQ ID NO: 19:

(i) SEQUENZ CHARAKTERISTIKA:
   (A) LÄNGE: 37 Basenpaare
   (B) ART: Nukleinsäure
   (C) STRANGFORM: Einzel
   (D) TOPOLOGIE: linear
(ix) MERKMALE:
   (A) NAME/SCHLÜSSEL: -
   (B) LAGE: 1..37
   (D) SONSTIGE ANGABEN: /label= PDGFB190-PRIMII
      /note= "synthetische DNA; synthetischer PCR-Primer"
(xi) SEQUENZBESCHREIBUNG: SEQ ID NO: 19:
   CCCGGGAAGC TTCCGGTTAT CAGGTCACAG GCCGTGC 37

### (2) INFORMATION ZU SEQ ID NO: 20:

(i) SEQUENZ CHARAKTERISTIKA:
   (A) LÄNGE: 1956 Basenpaare
   (B) ART: Nukleinsäure
   (C) STRANGFORM: Einzel
   (D) TOPOLOGIE: linear
(ii) ART DES MOLEKÜLS: cDNS
(vi) URSPRÜNGLICHE HERKUNFT:
   (A) ORGANISMUS: Homo sapiens
(vii) UNMITTELBARE HERKUNFT:
   (B) CLON: pSQ2-SEAP (Berger et al., 1988)
(ix) MERKMALE:
   (A) NAME/SCHLÜSSEL: CDS
   (B) LAGE: 43..1560
   (D) SONSTIGE ANGABEN: /note= "humanes SEAP-Gen; flankiert von 5'-EcoRI und 3'-HindIII Restriktionsschnittstellen"
(ix) MERKMALE:
   (A) NAME/SCHLÜSSEL: mat_peptide
   (B) LAGE: 94..1560
   (D) SONSTIGE ANGABEN: /product= "matures Protein"
(x) VERÖFFENTLICHUNGSINFORMATION:
   (A) AUTOREN:
      Berger, J.
      Hauber, J.
      Hauber, R.
      Geiger, R.
      Cullen, B. R.
   (C) ZEITSCHRIFT: Gene
   (D) BAND: 66
   (F) SEITEN: 1-10
   (G) DATUM: 1988
(x) VERÖFFENTLICHUNGSINFORMATION:
   (A) AUTOREN: Millan, J. L.
   (C) ZEITSCHRIFT: J. Biol. Chem.
   (D) BAND: 261
   (F) SEITEN: 3112-3115
   (G) DATUM: 1986
(xi) SEQUENZBESCHREIBUNG: SEQ ID NO: 20:
   GAATTCGAGC TCGCCCGGGG ATCCTCTAGA GTCAGCTTCT GC ATG CTG CTG CTG         54
                                                  Met Leu Leu Leu
                                                  -17     -15
   CTG CTG CTG CTG GGC CTG AGG CTA CAG CTC TCC CTG GGC ATC ATC CCA       102
   Leu Leu Leu Leu Gly Leu Arg Leu Gln Leu Ser Leu Gly Ile Ile Pro
               -10                 -5                      1
   GTT GAG GAG GAG AAC CCG GAC TTC TGG AAC CGC GAG GCA GCC GAG GCC       150
   Val Glu Glu Glu Asn Pro Asp Phe Trp Asn Arg Glu Ala Ala Glu Ala
       5                   10                  15
   CTG GGT GCC GCC AAG AAG CTG CAG CCT GCA CAG ACA GCC GCC AAG AAC       198
   Leu Gly Ala Ala Lys Lys Leu Gln Pro Ala Gln Thr Ala Ala Lys Asn
   20                  25                  30                  35
   CTC ATC ATC TTC CTG GGC GAT GGG ATG GGG GTG TCT ACG GTG ACA GCT       246
   Leu Ile Ile Phe Leu Gly Asp Gly Met Gly Val Ser Thr Val Thr Ala
                   40                  45                  50
   GCC AGG ATC CTA AAA GGG CAG AAG AAG GAC AAA CTG GGG CCT GAG ATA       294
   Ala Arg Ile Leu Lys Gly Gln Lys Lys Asp Lys Leu Gly Pro Glu Ile
               55                  60                  65
   CCC CTG GCC ATG GAC CGC TTC CCA TAT GTG GCT CTG TCC AAG ACA TAC       342
   Pro Leu Ala Met Asp Arg Phe Pro Tyr Val Ala Leu Ser Lys Thr Tyr
           70                  75                  80
   AAT GTA GAC AAA CAT GTG CCA GAC AGT GGA GCC ACA GCC ACG GCC TAC       390
   Asn Val Asp Lys His Val Pro Asp Ser Gly Ala Thr Ala Thr Ala Tyr
       85                  90                  95
   CTG TGC GGG GTC AAG GGC AAC TTC CAG ACC ATT GGC TTG AGT GCA GCC       438
   Leu Cys Gly Val Lys Gly Asn Phe Gln Thr Ile Gly Leu Ser Ala Ala
   100                 105                 110                 115
   GCC CGC TTT AAC CAG TGC AAC ACG ACA CGC GGC AAC GAG GTC ATC TCC       486
   Ala Arg Phe Asn Gln Cys Asn Thr Thr Arg Gly Asn Glu Val Ile Ser
                   120                 125                 130
   GTG ATG AAT CGG GCC AAG AAA GCA GGG AAG TCA GTG GGA GTG GTA ACC       534
   Val Met Asn Arg Ala Lys Lys Ala Gly Lys Ser Val Gly Val Val Thr
               135                 140                 145
   ACC ACA CGA GTG CAG CAC GCC TCG CCA GCC GGC ACC TAC GCC CAC ACG       582
   Thr Thr Arg Val Gln His Ala Ser Pro Ala Gly Thr Tyr Ala His Thr
           150                 155                 160
   GTG AAC CGC AAC TGG TAC TCG GAC GCC GAC GTG CCT GCC TCG GCC CGC       630
   Val Asn Arg Asn Trp Tyr Ser Asp Ala Asp Val Pro Ala Ser Ala Arg
       165                 170                 175
   CAG GAG GGG TGC CAG GAC ATC GCT ACG CAG CTC ATC TCC AAC ATG GAC       678
   Gln Glu Gly Cys Gln Asp Ile Ala Thr Gln Leu Ile Ser Asn Met Asp
   180                 185                 190                 195
   ATT GAC GTG ATC CTA GGT GGA GGC CGA AAG TAC ATG TTT CCC ATG GGA      726
   Ile Asp Val Ile Leu Gly Gly Gly Arg Lys Tyr Met Phe Pro Met Gly
                   200                 205                 210
   ACC CCA GAC CCT GAG TAC CCA GAT GAC TAC AGC CAA GGT GGG ACC AGG      774
   Thr Pro Asp Pro Glu Tyr Pro Asp Asp Tyr Ser Gln Gly Gly Thr Arg
               215                 220                 225
   CTG GAC GGG AAG AAT CTG GTG CAG GAA TGG CTG GCG AAG CGC CAG GGT      822
   Leu Asp Gly Lys Asn Leu Val Gln Glu Trp Leu Ala Lys Arg Gln Gly
           230                 235                 240
   GCC CGG TAT GTG TGG AAC CGC ACT GAG CTC ATG CAG GCT TCC CTG GAC      870
   Ala Arg Tyr Val Trp Asn Arg Thr Glu Leu Met Gln Ala Ser Leu Asp
       245                 250                 255
   CCG TCT GTG ACC CAT CTC ATG GGT CTC TTT GAG CCT GGA GAC ATG AAA      918
   Pro Ser Val Thr His Leu Met Gly Leu Phe Glu Pro Gly Asp Met Lys
   260                 265                 270                 275
   TAC GAG ATC CAC CGA GAC TCC ACA CTG GAC CCC TCC CTG ATG GAG ATG      966
   Tyr Glu Ile His Arg Asp Ser Thr Leu Asp Pro Ser Leu Met Glu Met
                   280                 285                 290
   ACA GAG GCT GCC CTG CGC CTG CTG AGC AGG AAC CCC CGC GGC TTC TTC     1014
   Thr Glu Ala Ala Leu Arg Leu Leu Ser Arg Asn Pro Arg Gly Phe Phe
               295                 300                 305
   CTC TTC GTG GAG GGT GGT CGC ATC GAC CAT GGT CAT CAT GAA AGC AGG     1062
   Leu Phe Val Glu Gly Gly Arg Ile Asp His Gly His His Glu Ser Arg
           310                 315                 320
   GCT TAC CGG GCA CTG ACT GAG ACG ATC ATG TTC GAC GAC GCC ATT GAG     1110
   Ala Tyr Arg Ala Leu Thr Glu Thr Ile Met Phe Asp Asp Ala Ile Glu
       325                 330                 335
   AGG GCG GGC CAG CTC ACC AGC GAG GAG GAC ACG CTG AGC CTC GTC ACT     1158
   Arg Ala Gly Gln Leu Thr Ser Glu Glu Asp Thr Leu Ser Leu Val Thr
   340                 345                 350                 355
   GCC GAC CAC TCC CAC GTC TTC TCC TTC GGA GGC TAC CCC CTG CGA GGG     1206
   Ala Asp His Ser His Val Phe Ser Phe Gly Gly Tyr Pro Leu Arg Gly
                   360                 365                 370
   AGC TCC ATC TTC GGG CTG GCC CCT GGC AAG GCC CGG GAC AGG AAG GCC     1254
   Ser Ser Ile Phe Gly Leu Ala Pro Gly Lys Ala Arg Asp Arg Lys Ala
               375                 380                 385
   TAC ACG GTC CTC CTA TAC GGA AAC GGT CCA GGC TAT GTG CTC AAG GAC     1302
   Tyr Thr Val Leu Leu Tyr Gly Asn Gly Pro Gly Tyr Val Leu Lys Asp
           390                 395                 400
   GGC GCC CGG CCG GAT GTT ACC GAG AGC GAG AGC GGG AGC CCC GAG TAT     1350
   Gly Ala Arg Pro Asp Val Thr Glu Ser Glu Ser Gly Ser Pro Glu Tyr
       405                 410                 415
   CGG CAG CAG TCA GCA GTG CCC CTG GAC GAA GAG ACC CAC GCA GGC GAG     1398
   Arg Gln Gln Ser Ala Val Pro Leu Asp Glu Glu Thr His Ala Gly Glu
   420                 425                 430                 435
   GAC GTG GCG GTG TTC GCG CGC GGC CCG CAG GCG CAC CTG GTT CAC GGC     1446
   Asp Val Ala Val Phe Ala Arg Gly Pro Gln Ala His Leu Val His Gly
                   440                 445                 450
   GTG CAG GAG CAG ACC TTC ATA GCG CAC GTC ATG GCC TTC GCC GCC TGC      1494
   Val Gln Glu Gln Thr Phe Ile Ala His Val Met Ala Phe Ala Ala Cys
               455                 460                 465
   CTG GAG CCC TAC ACC GCC TGC GAC CTG GCG CCC CCC GCC GGC ACC ACC      1542
   Leu Glu Pro Tyr Thr Ala Cys Asp Leu Ala Pro Pro Ala Gly Thr Thr
           470                 475                 480
   GAC GCC GCG CAC CCG GGT TAACCCGTGG TCCCCGCGTT GCTTCCTCTG             1590
   Asp Ala Ala His Pro Gly
       485
   CTGGCCGGGA CCCTGCTGCT GCTGGAGACG GCCACTGCTC CCTGAGTGTC CCGTCCCTGG    1650
   GGCTCCTGCT TCCCCATCCC GGAGTTCTCC TGCTCCCCAC CTCCTGTCGT CCTGCCTGGC    1710
   CTCCAGCCCG AGTCGTCATC CCCGGAGTCC CTATACAGAG GTCCTGCCAT GGAACCTTCC    1770
   CCTCCCCGTG CGCTCTGGGG ACTGAGCCCA TGACACCAAA CCTGCCCCTT GGCTGCTCTC    1830
   GGACTCCCTA CCCCAACCCC AGGGACTGCA GGTTGTGCCC TGTGGCTGCC TGCACCCCAG    1890
   GAAAGGAGGG GGCTCAGGCC ATCCAGCCAC CACCTACAGC CCAGTGGCCT CGAGCTGCAG    1950
   AAGCTT                                                               1956

### (2) INFORMATION ZU SEQ ID NO: 21:

(i) SEQUENZ CHARAKTERISTIKA:
   (A) LÄNGE: 506 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 21:
   Met Leu Leu Leu Leu Leu Leu Leu Gly Leu Arg Leu Gln Leu Ser Leu
   -17     -15                 -10                 -5
   Gly Ile Ile Pro Val Glu Glu Glu Asn Pro Asp Phe Trp Asn Arg Glu
         1               5                  10                  15
   Ala Ala Glu Ala Leu Gly Ala Ala Lys Lys Leu Gln Pro Ala Gln Thr
                    20                  25                  30
   Ala Ala Lys Asn Leu Ile Ile Phe Leu Gly Asp Gly Met Gly Val Ser
                35                  40                  45
   Thr Val Thr Ala Ala Arg Ile Leu Lys Gly Gln Lys Lys Asp Lys Leu
            50                  55                  60
   Gly Pro Glu Ile Pro Leu Ala Met Asp Arg Phe Pro Tyr Val Ala Leu
        65                  70                  75
   Ser Lys Thr Tyr Asn Val Asp Lys His Val Pro Asp Ser Gly Ala Thr
    80                  85                  90                  95
   Ala Thr Ala Tyr Leu Cys Gly Val Lys Gly Asn Phe Gln Thr Ile Gly
                   100                 105                 110
   Leu Ser Ala Ala Ala Arg Phe Asn Gln Cys Asn Thr Thr Arg Gly Asn
               115                 120                 125
   Glu Val Ile Ser Val Met Asn Arg Ala Lys Lys Ala Gly Lys Ser Val
           130                 135                 140
   Gly Val Val Thr Thr Thr Arg Val Gln His Ala Ser Pro Ala Gly Thr
       145                 150                 155
   Tyr Ala His Thr Val Asn Arg Asn Trp Tyr Ser Asp Ala Asp Val Pro
   160                 165                 170                 175
   Ala Ser Ala Arg Gln Glu Gly Cys Gln Asp Ile Ala Thr Gln Leu Ile
                   180                 185                 190
   Ser Asn Met Asp Ile Asp Val Ile Leu Gly Gly Gly Arg Lys Tyr Met
               195                 200                 205
   Phe Pro Met Gly Thr Pro Asp Pro Glu Tyr Pro Asp Asp Tyr Ser Gln
           210                 215                 220
   Gly Gly Thr Arg Leu Asp Gly Lys Asn Leu Val Gln Glu Trp Leu Ala
       225                 230                 235
   Lys Arg Gln Gly Ala Arg Tyr Val Trp Asn Arg Thr Glu Leu Met Gln
   240                 245                 250                 255
   Ala Ser Leu Asp Pro Ser Val Thr His Leu Met Gly Leu Phe Glu Pro
                   260                 265                 270
   Gly Asp Met Lys Tyr Glu Ile His Arg Asp Ser Thr Leu Asp Pro Ser
               275                 280                 285
   Leu Met Glu Met Thr Glu Ala Ala Leu Arg Leu Leu Ser Arg Asn Pro
           290                 295                 300
   Arg Gly Phe Phe Leu Phe Val Glu Gly Gly Arg Ile Asp His Gly His
       305                 310                 315
   His Glu Ser Arg Ala Tyr Arg Ala Leu Thr Glu Thr Ile Met Phe Asp
   320                 325                 330                 335
   Asp Ala Ile Glu Arg Ala Gly Gln Leu Thr Ser Glu Glu Asp Thr Leu
                   340                 345                 350
   Ser Leu Val Thr Ala Asp His Ser His Val Phe Ser Phe Gly Gly Tyr
               355                 360                 365
   Pro Leu Arg Gly Ser Ser Ile Phe Gly Leu Ala Pro Gly Lys Ala Arg
           370                 375                 380
   Asp Arg Lys Ala Tyr Thr Val Leu Leu Tyr Gly Asn Gly Pro Gly Tyr
       385                 390                 395
   Val Leu Lys Asp Gly Ala Arg Pro Asp Val Thr Glu Ser Glu Ser Gly
   400                 405                 410                 415
   Ser Pro Glu Tyr Arg Gln Gln Ser Ala Val Pro Leu Asp Glu Glu Thr
                   420                 425                 430
   His Ala Gly Glu Asp Val Ala Val Phe Ala Arg Gly Pro Gln Ala His
               435                 440                 445
   Leu Val His Gly Val Gln Glu Gln Thr Phe Ile Ala His Val Met Ala
           450                 455                 460
   Phe Ala Ala Cys Leu Glu Pro Tyr Thr Ala Cys Asp Leu Ala Pro Pro
       465                 470                 475
   Ala Gly Thr Thr Asp Ala Ala His Pro Gly
   480                 485

### (2) INFORMATION ZU SEQ ID NO: 22:

(i) SEQUENZ CHARAKTERISTIKA:
   (A) LANGE: 1811 Basenpaare
   (B) ART: Nukleinsäure
   (C) STRANGFORM: Einzel
   (D) TOPOLOGIE: linear
(ii) ART DES MOLEKÜLS: cDNS
(vi) URSPRÜNGLICHE HERKUNFT:
   (A) ORGANISMUS: Feuerfliege (Photinus pyralis)
(vii) UNMITTELBARE HERKUNFT:
   (B) CLON: pRSVLUC (de Wet et al., 1987)
(ix) MERKMALE:
   (A) NAME/SCHLÜSSEL: CDS
   (B) LAGE: 94..1743
   (D) SONSTIGE ANGABEN: /note= "codierende Region des Luciferase-Gens; flankiert von 5'-SmaI und 3'-HindIII Restriktionsschnittstellen"
(x) VERÖFFENTLICHUNGSINFORMATION:
   (A) AUTOREN:
      de Wet, J. R.
      Wood, K. V.
      DeLuca, M.
      Helinski, D. R.
      Subramani, s.
   (C) ZEITSCHRIFT: Mol. Cell. Biol.
   (D) BAND: 7
   (F) SEITEN: 725-737
   (G) DATUM: 1987
(xi)SEQUENZBESCHREIBUNG:SEQ ID NO: 22:
   CCCGGGGATC CTCTAGAGTC AGCTTGAATT CCTTTGTGTT ACATTCTTGA ATGTCGCTCG        60
   CAGTGACATT AGCATTCCGG TACTGTTGGT AAA ATG GAA GAC GCC AAA AAC ATA       114
                                        Met Glu Asp Ala Lys Asn Ile
                                          1               5
   AAG AAA GGC CCG GCG CCA TTC TAT CCT CTA GAG GAT GGA ACC GCT GGA       162
   Lys Lys Gly Pro Ala Pro Phe Tyr Pro Leu Glu Asp Gly Thr Ala Gly
           10                  15                  20
   GAG CAA CTG CAT AAG GCT ATG AAG AGA TAC GCC CTG GTT CCT GGA ACA 210
   Glu Gln Leu His Lys Ala Met Lys Arg Tyr Ala Leu Val Pro Gly Thr
   25 30 35
   ATT GCT TTT ACA GAT GCA CAT ATC GAG GTG AAC ATC ACG TAC GCG GAA 258
   Ile Ala Phe Thr Asp Ala His Ile Glu Val Asn Ile Thr Tyr Ala Glu
   40 45 50 55
   TAC TTC GAA ATG TCC GTT CGG TTG GCA GAA GCT ATG AAA CGA TAT GGG 306
   Tyr Phe Glu Met Ser Val Arg Leu Ala Glu Ala Met Lys Arg Tyr Gly
   60 65 70
   CTG AAT ACA AAT CAC AGA ATC GTC GTA TGC AGT GAA AAC TCT CTT CAA 354
   Leu Asn Thr Asn His Arg Ile Val Val Cys Ser Glu Asn Ser Leu Gln
   75 80 85
   TTC TTT ATG CCG GTG TTG GGC GCG TTA TTT ATC GGA GTT GCA GTT GCG 402
   Phe Phe Met Pro Val Leu Gly Ala Leu Phe Ile Gly Val Ala Val Ala
   90 95 100
   CCC GCG AAC GAC ATT TAT AAT GAA CGT GAA TTG CTC AAC AGT ATG AAC 450
   Pro Ala Asn Asp Ile Tyr Asn Glu Arg Glu Leu Leu Asn Ser Met Asn
   105 110 115
   ATT TCG CAG CCT ACC GTA GTG TTT GTT TCC AAA AAG GGG TTG CAA AAA 498
   Ile Ser Gln Pro Thr Val Val Phe Val Ser Lys Lys Gly Leu Gln Lys
   120 125 130 135
   ATT TTG AAC GTG CAA AAA AAA TTA CCA ATA ATC CAG AAA ATT ATT ATC 546
   Ile Leu Asn Val Gln Lys Lys Leu Pro Ile Ile Gln Lys Ile Ile Ile
   140 145 150
   ATG GAT TCT AAA ACG GAT TAC CAG GGA TTT CAG TCG ATG TAC ACG TTC 594
   Met Asp Ser Lys Thr Asp Tyr Gln Gly Phe Gln Ser Met Tyr Thr Phe
   155 160 165
   GTC ACA TCT CAT CTA CCT CCC GGT TTT AAT GAA TAC GAT TTT GTA CCA 642
   Val Thr Ser His Leu Pro Pro Gly Phe Asn Glu Tyr Asp Phe Val Pro
   170 175 180
   GAG TCC TTT GAT CGT GAC AAA ACA ATT GCA CTG ATA ATG AAT TCC TCT 690
   Glu Ser Phe Asp Arg Asp Lys Thr Ile Ala Leu Ile Met Asn Ser Ser
   185 190 195
   GGA TCT ACT GGG TTA CCT AAG GGT GTG GCC CTT CCG CAT AGA ACT GCC 738
   Gly Ser Thr Gly Leu Pro Lys Gly Val Ala Leu Pro His Arg Thr Ala
   200 205 210 215
   TGC GTC AGA TTC TCG CAT GCC AGA GAT CCT ATT TTT GGC AAT CAA ATC 786
   Cys Val Arg Phe Ser His Ala Arg Asp Pro Ile Phe Gly Asn Gln Ile
   220 225 230
   ATT CCG GAT ACT GCG ATT TTA AGT GTT GTT CCA TTC CAT CAC GGT TTT 834
   Ile Pro Asp Thr Ala Ile Leu Ser Val Val Pro Phe His His Gly Phe
   235 240 245
   GGA ATG TTT ACT ACA CTC GGA TAT TTG ATA TGT GGA TTT CGA GTC GTC 882
   Gly Met Phe Thr Thr Leu Gly Tyr Leu Ile Cys Gly Phe Arg Val Val
   250 255 260
   TTA ATG TAT AGA TTT GAA GAA GAG CTG TTT TTA CGA TCC CTT CAG GAT 930
   Leu Met Tyr Arg Phe Glu Glu Glu Leu Phe Leu Arg Ser Leu Gln Asp
   265 270 275
   TAC AAA ATT CAA AGT GCG TTG CTA GTA CCA ACC CTA TTT TCA TTC TTC      978
   Tyr Lys Ile Gln Ser Ala Leu Leu Val Pro Thr Leu Phe Ser Phe Phe
   280                 285                 290                 295
   GCC AAA AGC ACT CTG ATT GAC AAA TAC GAT TTA TCT AAT TTA CAC GAA     1026
   Ala Lys Ser Thr Leu Ile Asp Lys Tyr Asp Leu Ser Asn Leu His Glu
                   300                 305                 310
   ATT GCT TCT GGG GGC GCA CCT CTT TCG AAA GAA GTC GGG GAA GCG GTT     1074
   Ile Ala Ser Gly Gly Ala Pro Leu Ser Lys Glu Val Gly Glu Ala Val
               315                 320                 325
   GCA AAA CGC TTC CAT CTT CCA GGG ATA CGA CAA GGA TAT GGG CTC ACT     1122
   Ala Lys Arg Phe His Leu Pro Gly Ile Arg Gln Gly Tyr Gly Leu Thr
           330                 335                 340
   GAG ACT ACA TCA GCT ATT CTG ATT ACA CCC GAG GGG GAT GAT AAA CCG     1170
   Glu Thr Thr Ser Ala Ile Leu Ile Thr Pro Glu Gly Asp Asp Lys Pro
       345                 350                 355
   GGC GCG GTC GGT AAA GTT GTT CCA TTT TTT GAA GCG AAG GTT GTG GAT     1218
   Gly Ala Val Gly Lys Val Val Pro Phe Phe Glu Ala Lys Val Val Asp
   360                 365                 370                 375
   CTG GAT ACC GGG AAA ACG CTG GGC GTT AAT CAG AGA GGC GAA TTA TGT     1266
   Leu Asp Thr Gly Lys Thr Leu Gly Val Asn Gln Arg Gly Glu Leu Cys
                   380                 385                 390
   GTC AGA GGA CCT ATG ATT ATG TCC GGT TAT GTA AAC AAT CCG GAA GCG     1314
   Val Arg Gly Pro Met Ile Met Ser Gly Tyr Val Asn Asn Pro Glu Ala
               395                 400                 405
   ACC AAC GCC TTG ATT GAC AAG GAT GGA TGG CTA CAT TCT GGA GAC ATA     1362
   Thr Asn Ala Leu Ile Asp Lys Asp Gly Trp Leu His Ser Gly Asp Ile
           410                 415                 420
   GCT TAC TGG GAC GAA GAC GAA CAC TTC TTC ATA GTT GAC CGC TTG AAG     1410
   Ala Tyr Trp Asp Glu Asp Glu His Phe Phe Ile Val Asp Arg Leu Lys
       425                 430                 435
   TCT TTA ATT AAA TAC AAA GGA TAT CAG GTG GCC CCC GCT GAA TTG GAA     1458
   Ser Leu Ile Lys Tyr Lys Gly Tyr Gln Val Ala Pro Ala Glu Leu Glu
   440                 445                 450                 455
   TCG ATA TTG TTA CAA CAC CCC AAC ATC TTC GAC GCG GGC GTG GCA GGT     1506
   Ser Ile Leu Leu Gln His Pro Asn Ile Phe Asp Ala Gly Val Ala Gly
                   460                 465                 470
   CTT CCC GAC GAT GAC GCC GGT GAA CTT CCC GCC GCC GTT GTT GTT TTG     1554
   Leu Pro Asp Asp Asp Ala Gly Glu Leu Pro Ala Ala Val Val Val Leu
               475                 480                 485
   GAG CAC GGA AAG ACG ATG ACG GAA AAA GAG ATC GTG GAT TAC GTC GCC     1602
   Glu His Gly Lys Thr Met Thr Glu Lys Glu Ile Val Asp Tyr Val Ala
           490                 495                 500
   AGT CAA GTA ACA ACC GCG AAA AAG TTG CGC GGA GGA GTT GTG TTT GTG     1650
   Ser Gln Val Thr Thr Ala Lys Lys Leu Arg Gly Gly Val Val Phe Val
       505                 510                 515
   GAC GAA GTA CCG AAA GGT CTT ACC GGA AAA CTC GAC GCA AGA AAA ATC     1698
   Asp Glu Val Pro Lys Gly Leu Thr Gly Lys Leu Asp Ala Arg Lys Ile
   520                 525                 530                 535
   AGA GAG ATC CTC ATA AAG GCC AAG AAG GGC GGA AAG TCC AAA TTG          1743
   Arg Glu Ile Leu Ile Lys Ala Lys Lys Gly Gly Lys Ser Lys Leu
               540                 545                 550
   TAAAATGTAA CTGTATTCAG CGATGACGAA ATTCTTAGCT ATTGTAATAG CTGCAGGCAT    1803
   GCAAGCTT                                                             1811

### (2) INFORMATION ZU SEQ ID NO: 23:

(i) SEQUENZ CHARAKTERISTIKA:
   (A) LÄNGE: 550 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 23:
   Met Glu Asp Ala Lys Asn Ile Lys Lys Gly Pro Ala Pro Phe Tyr Pro
    1               5                  10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asn Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Asn Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
                   325                 330                 335
   Arg Gln Gly Tyr Gly Leu Thr Glu Thr Thr Ser Ala Ile Leu Ile Thr
               340                 345                 350
   Pro Glu Gly Asp Asp Lys Pro Gly Ala Val Gly Lys Val Val Pro Phe
           355                 360                 365
   Phe Glu Ala Lys Val Val Asp Leu Asp Thr Gly Lys Thr Leu Gly Val
       370                 375                 380
   Asn Gln Arg Gly Glu Leu Cys Val Arg Gly Pro Met Ile Met Ser Gly
   385                 390                 395                 400
   Tyr Val Asn Asn Pro Glu Ala Thr Asn Ala Leu Ile Asp Lys Asp Gly
                   405                 410                 415
   Trp Leu His Ser Gly Asp Ile Ala Tyr Trp Asp Glu Asp Glu His Phe
               420                 425                 430
   Phe Ile Val Asp Arg Leu Lys Ser Leu Ile Lys Tyr Lys Gly Tyr Gln
           435                 440                 445
   Val Ala Pro Ala Glu Leu Glu Ser Ile Leu Leu Gln His Pro Asn Ile
       450                 455                 460
   Phe Asp Ala Gly Val Ala Gly Leu Pro Asp Asp Asp Ala Gly Glu Leu
   465                 470                 475                 480
   Pro Ala Ala Val Val Val Leu Glu His Gly Lys Thr Met Thr Glu Lys
                   485                 490                 495
   Glu Ile Val Asp Tyr Val Ala Ser Gln Val Thr Thr Ala Lys Lys Leu
               500                 505                 510
   Arg Gly Gly Val Val Phe Val Asp Glu Val Pro Lys Gly Leu Thr Gly
           515                 520                 525
   Lys Leu Asp Ala Arg Lys Ile Arg Glu Ile Leu Ile Lys Ala Lys Lys
       530                 535                 540
   Gly Gly Lys Ser Lys Leu
   545                 550

### (2) INFORMATION ZU SEQ ID NO: 24:

(i) SEQUENZ CHARAKTERISTIKA:
   (A) LÄNGE: 625 Basenpaare
   (B) ART: Nukleinsäure
   (C) STRANGFORM: Einzel
   (D) TOPOLOGIE: linear
(ii) ART DES MOLEKÜLS: cDNS
(vi) URSPRÜNGLICHE HERKUNFT:
   (A) ORGANISMUS: Homo sapiens
(vii) UNMITTELBARE HERKUNFT:
   (B) CLON: pSBC-1/-2-PDGF-B
(ix) MERKMALE:
   (A) NAME/SCHLÜSSEL: CDS
   (B) LAGE: 40..609
   (D) SONSTIGE ANGABEN: /product= "PDGF-B Vorlaeufersequenz"
(ix) MERKMALE:
   (A) NAME/SCHLÜSSEL: mat_peptide
   (B) LAGE: 283..609
   (D) SONSTIGE ANGABEN: /product= "mature PDGF-B Kette"
(xi) SEQUENZBESCHREIBUNG: SEQ ID NO: 24:
   GAATTCGAGC TCGCCCGGGG ATCCTCTAGA GTCGACACC ATG AAT CGC TGC TGG          54
                                              Met Asn Arg Cys Trp
                                              -81 -80
   GCG CTC TTC CTG TCT CTC TGC TGC TAC CTG CGT CTG GTC AGC GCC GAG        102
   Ala Leu Phe Leu Ser Leu Cys Cys Tyr Leu Arg Leu Val Ser Ala Glu
       -75                 -70                 -65
   GGG GAC CCC ATT CCC GAG GAG CTT TAT GAG ATG CTG AGT GAT CAC TCG        150
   Gly Asp Pro Ile Pro Glu Glu Leu Tyr Glu Met Leu Ser Asp His Ser
   -60                 -55                 -50                 -45
   ATC CGC TCC TTT GAT GAT CTC CAA CGC CTG CTG CAC GGA GAC CCC GGA        198
   Ile Arg Ser Phe Asp Asp Leu Gln Arg Leu Leu His Gly Asp Pro Gly
                   -40                 -35                 -30
   GAG GAA GAT GGG GCC GAG TTG GAC CTG AAC ATG ACC CGC TCC CAC TCT        246
   Glu Glu Asp Gly Ala Glu Leu Asp Leu Asn Met Thr Arg Ser His Ser
               -25                 -20                 -15
   GGA GGC GAG CTG GAG AGC TTG GCT CGT GGA AGA AGG AGC CTG GGT TCC        294
   Gly Gly Glu Leu Glu Ser Leu Ala Arg Gly Arg Arg Ser Leu Gly Ser
           -10                  -5                   1
   CTG ACC ATT GCT GAG CCG GCC ATG ATC GCC GAG TGC AAG ACG CGC ACC        342
   Leu Thr Ile Ala Glu Pro Ala Met Ile Ala Glu Cys Lys Thr Arg Thr
     5                  10                  15                  20
   GAG GTG TTC GAG ATC TCC CGG CGC CTC ATA GAC CGC ACC AAC GCC AAC        390
   Glu Val Phe Glu Ile Ser Arg Arg Leu Ile Asp Arg Thr Asn Ala Asn
                    25                  30                  35
   TTC CTG GTG TGG CCG CCC TGT GTG GAG GTG CAG CGC TGC TCC GGC TGC        438
   Phe Leu Val Trp Pro Pro Cys Val Glu Val Gln Arg Cys Ser Gly Cys
               40                  45                  50
   TGC AAC AAC CGC AAC GTG CAG TGC CGC CCC ACC CAG GTG CAG CTG CGA        486
   Cys Asn Asn Arg Asn Val Gln Cys Arg Pro Thr Gln Val Gln Leu Arg
           55                  60                  65
   CCT GTC CAG GTG AGA AAG ATC GAG ATT GTG CGG AAG AAG CCA ATC TTT        534
   Pro Val Gln Val Arg Lys Ile Glu Ile Val Arg Lys Lys Pro Ile Phe
       70                  75                  80
   AAG AAG GCC ACG GTG ACG CTG GAA GAC CAC CTG GCA TGC AAG TGT GAG        582
   Lys Lys Ala Thr Val Thr Leu Glu Asp His Leu Ala Cys Lys Cys Glu
   85                  90                  95                 100
   ACA GTG GCA GCT GCA CGG CCT GTG ACC TGATAACCGG AACGTT                  625
   Thr Val Ala Ala Ala Arg Pro Val Thr
                   105

### (2) INFORMATION ZU SEQ ID NO: 25:

(i) SEQUENZ CHARAKTERISTIKA:
(A) LÄNGE: 190 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 25:

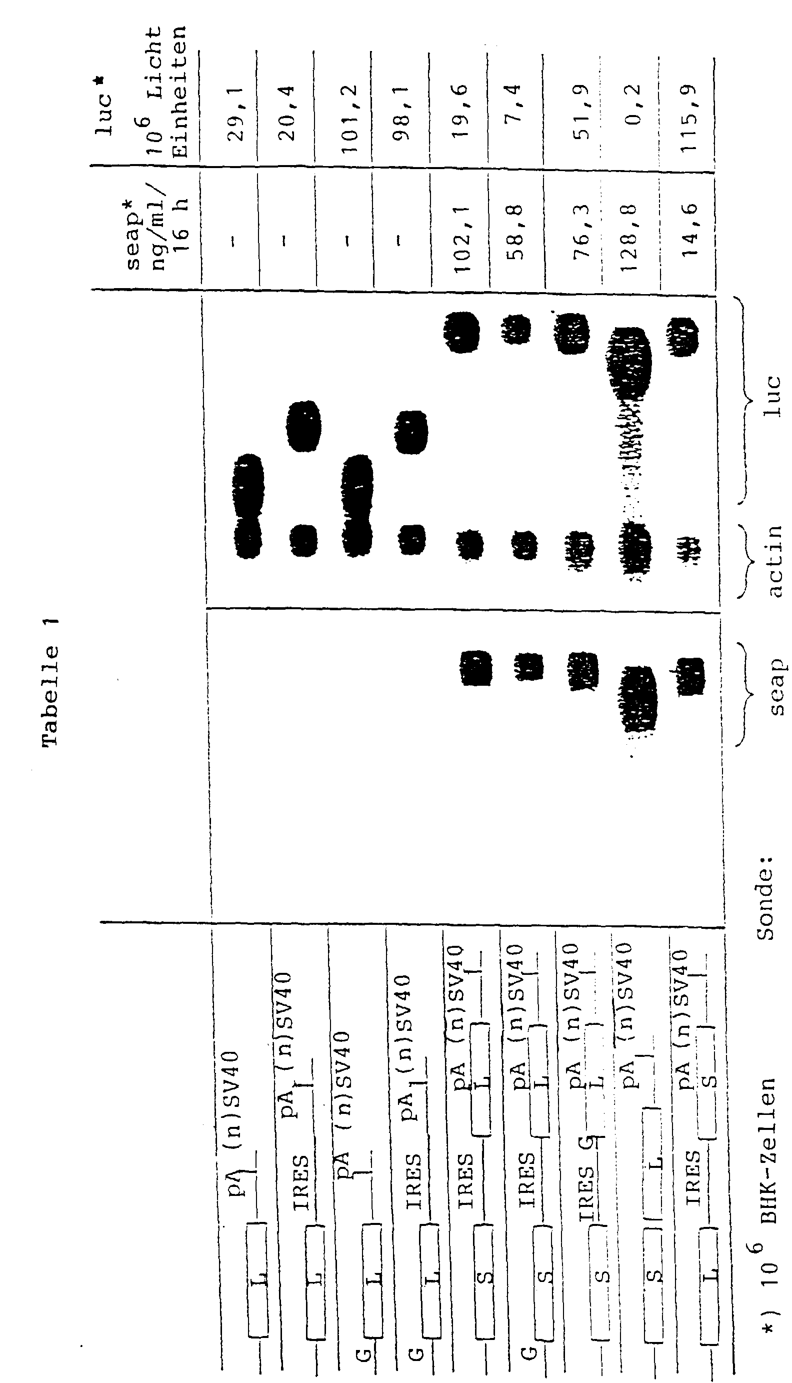

**Tabelle 2)**

| Plasmid | monocis. | bicis. | Elisa* | Mitogentest* |
|---|---|---|---|---|
| pSBC-2-PDGF-A | + | - | 14.3 | 1000 |
| pSBC-2-PDGF-B | + | - | 5 | 250 |
| + pSBC-PDGF-A/B | - | + | 291 | 600 |
| pSBC-PDGF-B/A | - | + | 520 | 550 |
| pSBC-2-PDGF-B₁₉₀ | + | - | 17.5 | 2000 |
| pSBC-2-PDGF-A/G-B₁₉₀ | - | + | 1020 | 1100 |
| pSBC-2-PDGF-B₁₉₀/G-A | - | + | 2550 | 2500 |
| pSBC-2-PDGF-A + B | + | - | 590 | 900 |
| pSBC-2-PDGF-G-A +G-B₁₉₀ | + | - | 380 | 1300 |

| | | | | |
|---|---|---|---|---|
| *PDGF in ng/ml 10⁶ BHK/24h | | | | |

## Patentansprüche

1. Multicistronische Expressionseinheit zur äquimolaren Expression von Polypeptiden oder Untereinheiten derselben in Säugerzellen als Wirtszellen, gekennzeichnet durch die allgemeine Formel
p - 5'UTR - C₁ - (IRES - Y - C₂)ₙ - 3'UTR - polyA,
in der
"p" ein transkriptionaler Promotor ist,
"5'UTR" eine nicht translatierte Nukleotidsequenz ist,
n 1, 2 oder 3 ist,
"C₁" und "C₂" Cistrons sind, welche jeweils ein für ein Polypeptid oder dessen Untereinheit kodierendes Gen enthalten, wobei dann, wenn n 2 oder 3 ist, die Sequenzen C₂ der aufeinanderfolgenden Gruppen (IRES-Y-C₂) untereinander gleich oder verschieden sein können und ferner C₁ und C₂ gleich oder verschieden sein können,
"IRES" eine Nukleotidsequenz viralen, zellulären oder synthetischen Ursprungs ist, die in der Stufe der Translation für die interne Initiation verantwortlich ist,
"Y" eine Nukleotidsequenz ist, welche im Zusammenwirken mit IRES für eine Expression des (der) in C₂ enthaltenen Gens(e) auf solche Weise sorgt, daß die Genprodukte von C₁ und C₂ in äquimolaren Mengen exprimiert werden,
"3'UTR" eine nicht translatierte Nukleotidsequenz ist
und
"polyA" ein Polyadenylierungssignal ist.

2. Expressionseinheit nach Anspruch 1, **dadurch gekennzeichnet**, daß IRES die 5'UTR des Poliovirus Typ 1, 2 oder 3, des *Enzephalomyocarditis Virus* (EMV), des *" Theilers murine encephalomyelitis virus"* (TMEV), des *"foot and mouth disease virus"* (FMDV), des *"bovine enterovirus"* (BEV), des *"coxsackie B virus"* (CBV), des *"human rhinovirus"* (HRV) oder die "human immunoglobulin heavy chain binding protein" (BIP) 5'UTR, die *Drosophila Antennapediae* 5'UTR, die *Drosophila Ultrabithorax* 5'UTR oder genetische Hybride oder Fragmente aus den oben angeführten Sequenzen ist.

3. Expressionseinheit nach Anspruch 1 oder 2, **dadurch gekennzeichnet**, daß IRES die Nukleotidsequenz gemäß SEQ ID NO: 5 ist.

4. Expressionseinheit nach den Ansprüchen 1 bis 3, **dadurch gekennzeichnet**, daß "Y" die β-Globinsequenz aus *Xenopus laevis,* die *Alfalfa mosaic virus* RNA4 5'UTR, Ferritin 5' UTR (animal), *Tobacco mosaic virus* 5' UTR (Omega) oder deren Leadermutanten, *Turnip yellow mosaic virus* (TYMV) 5' UTR, *Brome mosaic virus* (BMV) RNA3 5' UTR, *Rous sarcoma virus* (RSV) 5' UTR, *Adenovirus* tripartite leader (L1-3) und Varianten derselben, *Xenopus borealis* 5' UTR β-Globin oder *Xenopus tropicalis* 5' UTR β-Globin Sequenz ist.

5. Expressionseinheit nach den Ansprüchen 1 bis 4, **dadurch gekennzeichnet**, daß "Y" die β-Globinsequenz aus *Xenopus laevis* gemäß SEQ ID NO: 6, ein Fragment oder eine Variante derselben ist.

6. Expressionseinheit nach den Ansprüchen 1 bis 4, **dadurch gekennzeichnet**, daß IRES die *Poliovirus* Typ 1 UTR gemäß SEQ ID NO: 5 und "Y" die β-Globinsequenz aus *Xenopus laevis* gemäß SEQ ID NO: 6 sind.

7. Expressionseinheit nach den Ansprüchen 1 bis 6, dadurch gekennzeichnet, daß C₁ und C₂ jeweils Gene enthalten, die für Polypeptid-Untereinheiten singulärer oder heteromerer Proteine kodieren.

8. Expressionseinheit nach den Ansprüchen 1 bis 7, dadurch gekennzeichnet, daß C₁ und C₂ jeweils Gene enthalten, welche für die verschiedenen Untereinheiten von Faktor VIII, Kreatin-Kinase, Hämoglobin, Immunglobulinen, Histokompatibilitäts-Antigenen, Scatter-Faktor (HGF-SF), Mitgliedern der Familie des "Transforming Growth Factor Typ β, des Bone Morphogenic Proteins (BMP) Mitgliedern der Integrin-Familie, PDGF oder deren natürliche oder synthetische Varianten und Derivate kodieren.

9. Expressionseinheit nach Anspruch 6, dadurch gekennzeichnet, daß "n" 1 ist und C₁ und C₂ alternativ ein für die A- oder die B-Kette von PDGF, ein biologisch aktives Analogon oder ein Fragment derselben kodierendes Gen enthalten, wobei beide Gene gleichzeitig in der Expressionseinheit vertreten sind.

10. Expressionseinheit nach Anspruch 9, dadurch gekennzeichnet, daß C₁ oder C₂ die PDGF-A_{K}- (SEQ ID Nr. 1) oder die PDGF-A_{L} Vorläufer-Sequenz enthält.

11. Expressionseinheit nach den Ansprüchen 9 oder 10, dadurch gekennzeichnet, daß C₁ oder C₂ die vollständige PDGF-B Vorläufersequenz (SEQ ID Nr. 3), das *v-sis*-Gen aus Simian Sarcoma Virus oder Varianten dieser Sequenzen enthalten.

12. Expressionseinheit nach den Ansprüchen 9 bis 11, dadurch gekennzeichnet, daß C₁ oder C₂ ein Genfragment enthalten, das für ein PDGF-B-Vorläufermolekül kodiert, welches durch Ersetzen des für Arginin kodierenden Kodons in der Aminosäureposition 191 durch ein Translations-Stop-Kodon verkürzt ist (SEQ ID Nr. 24).

13. Expressionseinheit nach Anspruch 9, **dadurch gekennzeichnet**, daß C₁ und C₂ alternativ die PDGF-A_{K}-Sequenz (SEQ ID Nr. 1) oder die verkürzte PDGF-B190 Vorläufersequenz (SEQ ID Nr. 24) enthalten und beide Gene gleichzeitig in der Expressionseinheit vertreten sind.

14. Expressionseinheit nach den Ansprüchen 1 bis 6, **dadurch gekennzeichnet**, daß "n" 1 ist und C₁ und C₂ voneinander verschiedene Reportergene enthalten.

15. Expressionseinheit nach Anspruch 14, **dadurch gekennzeichnet**, daß die Reportergene für Luciferase bzw. für sekretorische alkalische Phosphatase kodieren.

16. Rekombinanter DNA-Vektor, welcher eine Expressionseinheit nach den Ansprüchen 1 bis 15, operativ insertiert enthält.

17. Wirtszelle, welche eine Säugerzelle transformiert mit einem Vektor gemäß Anspruch 16 ist.

18. Wirtszelle nach Anspruch 17, **dadurch gekennzeichnet**, daß sie eine CHO- oder BHK-Zelle ist.

19. Wirtszelle **dadurch gekennzeichnet**, daß sie eine Säugerzelle ist, die mit einem Vektor transformiert ist, welcher die Expressionseinheit nach den Ansprüchen 9 bis 13 operativ insertiert enthält.

20. Wirtszelle nach Anspruch 19, **dadurch gekennzeichnet**, daß sie eine CHO- oder BHK-Zelle ist.

21. Wirtszelle nach Anspruch 20, **dadurch gekennzeichnet**, daß sie eine PDGF-AB produzierende BHK-Zelle ist, die von einem der Klone 92-22-6 entsprechend DSM ACC 2048 oder 92-22-7 entsprechend DSM ACC 2049 abstammt.

22. Verfahren zur Herstellung von Proteinen bestehend aus äquimolaren Anteilen von Polypeptiduntereinheiten, **dadurch gekennzeichnet**, daß man Wirtszellen nach den Ansprüchen 17 bis 21 in einem geeigneten Medium kultiviert und das so erzeugte Protein von den Zellen und dem Medium abtrennt.

23. Verfahren nach Anspruch 22 zur Herstellung von heteromeren Proteinen.

24. Verfahren nach Anspruch 22 oder 23, **dadurch gekennzeichnet**, daß das Protein Faktor VIII, Kreatin-Kinase, Hämoglobin, ein Immunglobulin, ein Histokompatibilitäts-Antigen, Scatter-Faktor (HGF-SF), ein Mitglied der Transforming Growth Factor Typ β-Familie, Bone-Morphogenic Protein (BMP), ein Mitglied der Integrin-Familie, PDGF oder eine natürliche oder synthetische Variante oder ein Derivat derselben ist.

25. Verfahren zur Herstellung von heteromerem rPDGF-AB, **dadurch gekennzeichnet**, daß man Wirtszellen nach den Ansprüchen 19 bis 21 in einem geeigneten Medium kultiviert und das so erzeugte rPDGF-AB von den Zellen und dem Medium abtrennt.

26. Verfahren zur Herstellung eines rPDGF-AB enthaltenden pharmazeutischen und/oder kosmetischen Präparates, **dadurch gekennzeichnet**, daß man das Verfahren nach den Ansprüchen 22 bis 25 durchführt und das abgetrennte rPDGF-AB in einem weiteren Schritt zusammen mit pharmazeutisch und/oder kosmetisch verträglichen Hilfs- und Trägerstoffen formuliert.

27. Verfahren nach Anspruch 26, dadurch gekennzeichnet, daß das pharmazeutische und/oder kosmetische Präparat eine Salbe, ein Spray, Gel, Wundverband, ein Pflaster oder eine Wundauflage ist.

28. Wirtszelle **dadurch gekennzeichnet**, daß sie eine Säugerzelle ist, die mit einem Vektor transformiert ist, welcher die Expressionseinheit nach den Ansprüchen 14 oder 15 operativ insertiert enthält.

29. Wirtszelle nach Anspruch 28, **dadurch gekennzeichnet**, daß sie von dem Klon 91-46-9 entsprechend DSM ACC 2046 abstammt.

30. Verfahren zum Auffinden von translations-beeinflussenden Sequenzen "Y", welche im Zusammenwirken mit IRES in Expressionseinheiten nach den Ansprüchen 1 bis 15 die äquimolare Expression der Genprodukte von C₁ und C₂ bewirken, **dadurch gekennzeichnet**, daß man
(a) die zu untersuchenden Sequenzen als Y in Expressionseinheiten nach den Ansprüchen 1, 14 oder 15 einbringt,
(b) Vektoren konstruiert, welche die jeweilige Expressionseinheit operativ insertiert enthalten,
(c) Säugerzellen als Wirtszellen mit den Vektoren aus Stufe (b) transformiert und in einem geeigneten Medium kultiviert, und
(d) die Expressionsprodukte von C₁ und C₂ in dem Medium oder nach Abtrennen von den Zellen und/oder dem Medium quantifiziert.

31. Verfahren nach Anspruch 30, **dadurch gekennzeichnet**, daß in Stufe (c) CHO- oder BHK-Zellen als Wirtszellen verwendet werden.

32. Verfahren nach Anspruch 30, **dadurch gekennzeichnet**, daß in Stufe (c) Wirtszellen gemäß Anspruch 28 verwendet werden.

33. Verfahren nach den Ansprüchen 30 bis 32, **dadurch gekennzeichnet**, daß IRES eine Sequenz gemäß SEQ ID Nr. 5 ist.

34. Verfahren zum Auffinden von translations-initiierenden Sequenzen IRES, welche im Zusammenwirken mit "Y" in Expressionseinheiten nach den Ansprüchen 1 bis 15 die äquimolare Expression der Genprodukte von C₁ und C₂ bewirken, **dadurch gekennzeichnet**, daß man
(a) die zu untersuchenden Sequenzen als IRES in Expressionseinheiten nach den Ansprüchen 1, 14 oder 15 einbringt,
(b) Vektoren konstruiert, welche die jeweilige Expressionseinheit operativ insertiert enthalten,
(c) Säugerzellen als Wirtszellen mit den Vektoren aus Stufe (b) transformiert und in einem geeigneten Medium kultiviert, und
(d) die Expressionsprodukte von C₁ und C₂ in dem Medium oder nach Abtrennen von den Zellen und/oder dem Medium quantifiziert.

35. Verfahren nach Anspruch 34, **dadurch gekennzeichnet**, daß in Stufe (c) CHO- oder BHK-Zellen als Wirtszellen verwendet werden.

36. Verfahren nach Anspruch 34, **dadurch gekennzeichnet**, daß in Stufe (c) Wirtszellen gemäß Anspruch 28 verwendet werden.

37. Verfahren nach den Ansprüchen 34 bis 36, **dadurch gekennzeichnet**, daß "Y" eine Sequenz gemäß SEQ ID Nr. 6 ist.

## Claims

1. Multicistronic expression unit for the equimolar expression of polypeptides or subunits thereof in mammalian cells as host cells, characterized by the general formula
p - 5'UTR - C₁ - (IRES - Y - C₂)ₙ - 3'UTR - polyA,
in which
"p" is a transcriptional promoter,
"5'UTR" is an untranslated nucleotide sequence,
n is 1, 2 or 3,
"C₁" and "C₂" are cistrons which in each case contain a gene encoding a polypeptide or its subunit, in which case, if n is 2 or 3, the sequences C₂ of the successive groups (IRES-Y-C₂) may be identical to or different from each other and, furthermore, C₁ and C₂ may be identical or different,
"IRES" is a nucleotide sequence of viral, cellular or synthetic origin, which at the stage of translation is responsible for internal initiation,
"Y" is a nucleotide sequence which, in synergy with IRES, ensures expression of the gene(s) contained in C₂ in such a manner that the gene products of C₁ and C₂ are expressed in equimolar quantities,
"3'UTR" is an untranslated nucleotide sequence,
and
"polyA" is a polyadenylation signal.

2. Expression unit according to Claim 1, characterized in that the IRES is the 5'UTR of polio virus type 1, 2 or 3, of encephalomyocarditis virus (EMV), of "Theiler's murine encephalomyelitis virus" (TMEV), of "foot-and- mouth disease virus" (FMDV), of "bovine enterovirus" (BEV), of "coxsackie B virus" (CBV), or of "human rhinovirus" (HRV), or the "human immunoglobulin heavy chain binding protein" (BIP) S'UTR, the Drosophila antennapediae 5'UTR or the Drosophila ultrabithorax 5'UTR, or genetic hybrids or fragments from the above-listed sequences.

3. Expression unit according to Claim 1 or 2, characterized in that the IRES is the nucleotide sequence according to SEQ ID NO: 5.

4. Expression unit according to Claims 1 to 3, characterized in that "Y" is the β-globin sequence from Xenopus laevis, the alfalfa mosaic virus RNA4 5'UTR, ferritin 5'UTR (animal), tobacco mosaic virus S'UTR (omega), or their leader mutants, turnip yellow mosaic virus (TYMV) S'UTR, brome mosaic virus (BMV) RNA3 5'UTR, Rous sarcoma virus (RSV) 5'UTR, adenovirus tripartite leader (L1-3) and variants thereof, Xenopus borealis 5'UTR β-globin or Xenopus tropicalis 5'UTR β-globin sequence.

5. Expression unit according to Claims 1 to 4, characterized in that "Y" is the β-globin sequence from Xenopus laevis according to SEQ ID NO: 6, or a fragment or a variant thereof.

6. Expression unit according to Claims 1 to 4, characterized in that the IRES is the polio virus type 1 UTR according to SEQ ID NO: 5 and "Y" is the β-globin sequence from Xenopus laevis according to SEQ ID NO: 4.

7. Expression unit according to Claims 1 to 6, characterized in that C₁ and C₂ in each case contain genes which encode polypeptide subunits of single or heteromeric proteins.

8. Expression unit according to Claims 1 to 7, characterized in that C₁ and C₂ in each case contain genes which encode the different subunits of factor VIII, creatine kinase, haemoglobin, immunoglobulins, histocompatibility antigens, scatter factor (HGF-SF), members of the transforming growth factor type β family, of bone morphogenic protein (BMP), members of the integrin family, or PDGF, or their natural or synthetic variants and derivatives.

9. Expression unit according to Claim 6, characterized in that "n" is 1 and C₁ and C₂ alternatively contain a gene encoding the A or B chain of PDGF, or a biologically active analogue or a fragment thereof, both genes being represented simultaneously in the expression unit.

10. Expression unit according to Claim 9, characterized in that C₁ or C₂ contains the PDGF-A_{K} (SEQ ID NO: 1) or the PDGF-A_{L} precursor sequence.

11. Expression unit according to Claim 9 or 10, characterized in that C₁ or C₂ contains the complete PDGF-B precursor sequence (SEQ ID NO: 3), the ***v-sis*** gene from simian sarcoma virus, or variants of these sequences.

12. Expression unit according to Claims 9 to 11, characterized in that C₁ or C₂ contains a gene fragment which encodes a PDGF-B precursor molecule which is truncated by replacing the arginine-encoding codon in amino acid position 191 by a translation stop codon (SEQ ID NO: 24).

13. Expression unit according to Claim 9, characterized in that C₁ and C₂ alternatively contain the PDGF-A_{K} sequence (SEQ ID NO: 1) or the truncated PDGF-B190 precursor sequence (SEQ ID NO: 24), and both genes are represented simultaneously in the expression unit.

14. Expression unit according to Claims 1 to 6, characterized in that "n" is 1 and C₁ and C₂ contain reporter genes which are different from each other.

15. Expression unit according to Claim 14, characterized in that the reporter genes encode luciferase and secretory alkaline phosphatase.

16. Recombinant DNA vector which contains an expression unit according to Claims 1 to 15 inserted in an operative manner.

17. Host cell which is a mammalian cell transformed with a vector according to Claim 16.

18. Host cell according to Claim 17, characterized in that it is a CHO or BHK cell.

19. Host cell, characterized in that it is a mammalian cell which is transformed with a vector which contains the expression unit according to Claims 9 to 13 inserted in an operative manner.

20. Host cell according to Claim 19, characterized in that it is a CHO or BHK cell.

21. Host cell according to Claim 20, characterized in that it is a PDGF-AB-producing BHK cell which is derived from one of the clones 92-22-6, corresponding to DSM ACC 2048, or 92-22-7, corresponding to DSM ACC 2049.

22. Process for preparing proteins consisting of equimolar proportions of polypeptide subunits, characterized in that host cells according to Claims 17 to 21 are cultivated in a suitable medium and the resulting protein is separated off from the cells and the medium.

23. Process according to Claim 22 for preparing heteromeric proteins.

24. Process according to Claim 22 or 23, characterized in that the protein is factor VIII, creatine kinase, haemoglobin, an immunoglobulin, a histocompatibility antigen, scatter factor (HGF-SF), a member of the transforming growth factor type β family, bone morphogenic protein (BMP), a member of the integrin family, or PDGF, or a natural or synthetic variant or a derivative thereof.

25. Process for preparing heteromeric rPDGF-AB, characterized in that host cells according to Claims 19 to 21 are cultivated in a suitable medium and the resulting rPDGF-AB is separated off from the cells and the medium.

26. Process for preparing an rPDGF-AB-containing pharmaceutical and/or cosmetic preparation, characterized in that the process according to Claims 22 to 25 is carried out and the rPDGF-AB which is separated off is formulated in a further step together with pharmaceutically and/or cosmetically tolerated auxiliaries and excipients.

27. Process according to Claim 26, characterized in that the pharmaceutical and/or cosmetic preparation is an ointment, a spray, gel, wound bandage, a plaster or a wound dressing.

28. Host cell, characterized in that it is a mammalian cell which is transformed with a vector which contains the expression unit according to Claims 14 or 15 inserted in an operative manner.

29. Host cell according to Claim 28, characterized in that it is derived from the clone 91-46-9, corresponding to DSM ACC 2046.

30. Process for detecting translation-influencing "Y" sequences which, in synergy with the IRES, bring about the equimolar expression of the gene products of C₁ and C₂ in expression units according to Claims 1 to 15, characterized in that
(a) the sequences to be investigated as Y are introduced into expression units according to Claim 1, 14 or 15,
(b) vectors are constructed which contain the respective expression unit inserted in an operative manner,
(c) mammalian cells, as host cells, are transformed with the vectors from step (b) and cultivated in a suitable medium, and
(d) the expression products of C₁ and C₂ are quantified in the medium or following separation from the cells and/or the medium.

31. Process according to Claim 30, characterized in that CHO or BHK cells are used as host cells in step (c).

32. Process according to Claim 30, characterized in that host cells according to Claim 28 are used in step (c).

33. Process according to Claims 30 to 32, characterized in that the IRES is a sequence according to SEQ ID NO: 5.

34. Process for detecting translation-initiating IRES sequences which, in synergy with "Y", bring about the equimolar expression of the gene products of C₁ and C₂ in expression units according to Claim 1 to 15, characterized in that
(a) the sequences to be investigated as the IRES are introduced into expression units according to Claim 1, 14 or 15,
(b) vectors are constructed which contain the respective expression unit inserted in an operative manner,
(c) mammalian cells, as host cells, are transformed with the vectors from step (b) and cultivated in a suitable medium, and
(d) the expression products of C₁ and C₂ are quantified in the medium or following separation from the cells and/or the medium.

35. Process according to Claim 34, characterized in that CHO or BHK cells are used as host cells in step (c).

36. Process according to Claim 34, characterized in that host cells according to Claim 28 are used in step (c).

37. Process according to Claims 34 to 36, characterized in that "Y" is a sequence according to SEQ ID NO: 6.

## Revendications

1. Unité d'expression multicistronique pour l'expression équimolaire de polypeptides ou de sous-unités de ceux-ci dans des cellules de mammifère en tant que cellules-hôtes, caractérisée par la formule générale
p - 5'UTR - C₁ - (IRES - Y - C₂)ₙ - 3'UTR -polyA,
où
"p" est un promoteur transcriptionnel,
"5'UTR" est une séquence de nucléotides non traduite, n est 1, 2 ou 3,
"C₁" et "C₂" sont des cistrons qui contiennent chacun un gène codant pour un polypeptide ou sa sous-unité, pour lesquels alors, quand n est 2 ou 3, les séquences C₂ des groupes successifs (IRES-Y-C₂) peuvent être mutuellement identiques ou différentes, et en outre, C₁ et C₂ peuvent être identiques ou différents,
"IRES" est une séquence de nucléotides d'origine virale, cellulaire ou synthétique qui est responsable de l'initiation interne à l'étape de traduction,
"Y" est une séquence de nucléotides qui assure en coopération avec IRES l'expression du/des gène(s) contenu(s) dans C₂ de telle sorte que les produits des gènes de C₁ et C₂ sont exprimés en quantités équimolaires,
"3'UTR" est une séquence de nucléotides non traduite et
"polyA" est un signal de polyadénylation.

2. Unité d'expression selon la revendication 1, caractérisée en ce qu'IRES est la 5'UTR du virus de la polio de type 1, 2 ou 3, du *"Enzephalomyocarditis virus"* (EMV), du *"Theilers murine encephalomyelitis virus"* (TMEV), du *"Foot and mouth disease virus"* (FMDV), du *"Bovine enterovirus"* (BEV), du *"Coxsackie B virus"* (CBV), du *"Human rhinovirus"* (HRV) ou la 5'UTR de la *"Human immunoglobulin heavy chain binding protein"* (BIP), la 5'UTR de *Drosophila antennapediae,* la 5'UTR de *Drosophila ultrabithorax* ou des hybrides ou fragments génétiques des séquences énumérées ci-dessus.

3. Unité d'expression selon la revendication 1 ou 2, caractérisée en ce que l'IRES est la séquence de nucléotides selon SEQ ID N°5.

4. Unité d'expression selon les revendications 1 à 3, caractérisée en ce que "Y" est la séquence de la β-globine de *xenopus laevis,* la 5'UTR RNA4 de *alfalfa mosaic virus,* la 5'UTR de la ferritine (animale), la 5'UTR de *Tobacco mosaic virus* (omega) ou leurs principaux mutants, la 5'UTR de *Turnip yellow mosaic virus* (TYMV), la 5'UTR RNA3 de *Brome mosaic virus* (BMV), la 5'UTR de *Rous sarcoma virus* (RSV), le leader tripartite (L1.3) *d'Adenovirus* et des variants de ceux-ci, la séquence 5'UTR de la β-globine de *Xenopus borealis* ou la séquence 5'UTR de β-lobine de *Xenopus tropicalis.*

5. Unité d'expression selon les revendications 1 à 4, caractérisée en ce que "Y" est la séquence de la β-globine de *Xenopus laevis* selon SEQ ID N°6, un fragment ou un variant de celle-ci.

6. Unité d'expression selon les revendications 1 à 4, caractérisée en ce qu'IRES est l'UTR du virus de la polio de type 1 selon SEQ ID N°5 et "Y" est la séquence de la β-globine de *Xenopus laevis* selon SEQ ID N°6.

7. Unité d'expression selon les revendications 1 à 6, caractérisée en ce que C₁ et C₂ contiennent chacun des gènes codants pour des sous-unités de polypeptides de protéines singulières ou hétéromères.

8. Unité d'expression selon les revendications 1 à 7, caractérisée en ce que C₁ et C₂ contiennent chacun des gènes codants pour les différentes sous-unités du Facteur VIII, de la créatine kinase, de l'hémoglobine, des immunoglobulines, des antigènes d'histocompatibilité, du facteur de dispersion (HGF-SF), de membres de la famille du *transforming growth factor* de type β, des *bone morphogenic proteins* (BMP), de membres de la famille de l'intégrine, de PDGF ou de leurs variants et dérivés naturels ou synthétiques.

9. Unité d'expression selon la revendication 6, caractérisée en ce que "n" est 1 et C₁ et C₂ contiennent alternativement un gène codant pour la chaîne A ou B de PDGF, un analogue biologiquement actif ou un fragment de ceux-ci, les deux gènes étant représentés simultanément dans l'unité d'expression.

10. Unité d'expression selon la revendication 9, caractérisée en ce que C₁ ou C₂ contient la séquence précurseur PDGF-A_{K}-(SEQ ID N°1) ou PDGF-A_{L}.

11. Unité d'expression selon les revendications 9 ou 10, caractérisée en ce que C₁ ou C₂ contient la séquence précurseur complète PDGF-B (SEQ ID N°3), le gène *v-sis* du *Simian sarcoma virus* ou des variants de ces séquences.

12. Unité d'expression selon les revendications 9 à 11, caractérisée en ce que C₁ ou C₂ contient un fragment de gène codant pour un précurseur PDFG-B qui est raccourci par remplacement du codon codant pour l'arginine à la position d'acide aminé 191 par un codon d'arrêt de traduction (SEQ ID N°24).

13. Unité d'expression selon la revendication 9, caractérisée en ce que C₁ et C₂ contiennent alternativement la séquence PDGF-A_{K} (SEQ ID N°1) ou la séquence précurseur courte PDGF-B190 (SEQ ID N°24) et les deux gènes sont représentés simultanément dans l'unité d'expression.

14. Unité d'expression selon les revendications 1 à 6, caractérisée en ce que "n" est 1 et C₁ et C₂ contiennent des gènes rapporteurs différents les uns des autres.

15. Unité d'expression selon la revendication 14, caractérisée en ce que les gènes rapporteurs codent pour la luciférase ou la phosphatase alcaline sécrétoire.

16. Vecteur à ADN recombinant, dans lequel une unité d'expression selon les revendications 1 à 15 est insérée de façon opérationnelle.

17. Cellule-hôte qui est une cellule de mammifère transformée par un vecteur selon la revendication 16.

18. Cellule-hôte selon la revendication 17, caractérisée en ce qu'elle est une cellule CHO ou BHK.

19. Cellule-hôte, caractérisée en ce qu'elle est une cellule de mammifère transformée par un vecteur dans lequel l'unité d'expression selon les revendications 9 à 13 est insérée de façon opérationnelle.

20. Cellule-hôte selon la revendication 19, caractérisée en ce qu'elle est une cellule CHO ou BHK.

21. Cellule-hôte selon la revendication 20, caractérisée en ce qu'elle est une cellule BHK produisant du PDGF-AB, qui provient d'un des clones 92-22-6 selon DSM ACC 2048 ou 92-22-7 selon DSM ACC 2049.

22. Procédé de préparation de protéines composées de parties équimolaires de sous-unités de polypeptides, caractérisé en ce que des cellules-hôtes selon les revendications 17 à 21 sont cultivées dans un milieu adéquat et la protéine ainsi produite est séparée des cellules et du milieu.

23. Procédé selon la revendication 22 pour fabriquer des protéines hétéromères.

24. Procédé selon la revendication 22 ou 23, caractérisé en ce que la protéine est le Facteur VIII, la créatine kinase, l'hémoglobine, une immunoglobuline, un antigène d'histocompatibilité, un facteur de dispersion (HGF-SF), un membre de la famille du *transforming growth factor* de type β, une *bone morphogenic protein* (BMP), un membre de la famille de l'intégrine, PDGF ou un variant ou un dérivé naturel ou synthétique de ceux-ci.

25. Procédé de préparation de rPDGF-AB hétéromère, caractérisé en ce que des cellules-hôtes selon les revendications 19 à 21 sont cultivées dans un milieu adéquat et le rPDGF-AB ainsi produit est séparé des cellules et du milieu.

26. Procédé de préparation une préparation pharmaceutique et/ou cosmétique contenant du rPDGF-AB, caractérisé en ce que le procédé selon les revendications 22 à 25 est mis en oeuvre et le rPDGV-AB séparé est formulé dans une étape supplémentaire avec des additifs et des véhicules compatibles sur le plan pharmaceutique et/ou cosmétique.

27. Procédé selon la revendication 26, caractérisé en ce que la préparation pharmaceutique et/ou cosmétique est une pommade, un spray, un gel, un bandage, un pansement ou une compresse.

28. Cellule-hôte, caractérisée en ce qu'elle une cellule de mammifère qui est transformée par un vecteur dans lequel l'unité d'expression selon les revendications 14 ou 15 est insérée de façon opérationnelle.

29. Cellule-hôte selon la revendication 28, caractérisée en ce qu'elle provient du clone 91-46-9 selon DSM ACC 2046.

30. Procédé de détection des séquences "Y" ayant une influence sur la traduction, qui provoquent en coopération avec l'IRES dans des unités d'expression selon les revendications 1 à 15 l'expression équimolaire des produits des gènes de C₁ et C₂, caractérisé en ce que
(a) les séquences à analyser sont introduites en tant que Y dans des unités d'expression selon les revendications 1, 14 ou 15,
(b) des vecteurs sont construits dans lesquels l'unité d'expression respective est insérée de façon opérationnelle,
(c) des cellules de mammifère sont transformées en cellules-hôtes par les vecteurs de l'étape (b) et cultivées dans un milieu adéquat, et
(d) les produits d'expression de C₁ et C₂ sont quantifiés dans le milieu ou après la séparation des cellules et/ou du milieu.

31. Procédé selon la revendication 30, caractérisé en ce qu'à l'étape (c), des cellules CHO ou BHK sont utilisées comme cellules-hôtes.

32. Procédé selon la revendication 30, caractérisé en ce qu'à l'étape (c), des cellules-hôtes selon la revendication 28 sont utilisées.

33. Procédé selon les revendications 30 à 32, caractérisé en ce que l'IRES est une séquence selon SEQ ID N°5.

34. Procédé de détection de séquences IRES initiant la traduction, qui provoquent en coopération avec "Y" dans des unités d'expression selon les revendications 1 à 15 l'expression équimolaire des produits des gènes de C₁ et C₂, caractérisé en ce que
(a) les séquences à analyser sont introduites en tant que IRES dans des unités d'expression selon les revendications 1, 14 ou 15,
(b) des vecteurs sont construits dans lesquels l'unité d'expression respective est insérée de façon opérationnelle,
(c) des cellules de mammifère sont transformées en cellules-hôtes par les vecteurs de l'étape (b) et cultivées dans un milieu adéquat, et
(d) les produits d'expression de C₁ et C₂ sont quantifiés dans le milieu ou après la séparation des cellules et/ou du milieu.

35. Procédé selon la revendication 34, caractérisé en ce qu'à l'étape (c), des cellules CHO ou BHK sont utilisées comme cellules-hôtes.

36. Procédé selon la revendication 34, caractérisé en ce qu'à l'étape (c), des cellules-hôtes selon la revendication 28 sont utilisées.

37. Procédé selon les revendications 34 à 36, caractérisé en ce que "Y" est une séquence selon SEQ ID N°6.
